(19) Europäisches Patentamt
European Patent Office
Office européen des brevets

(11) EP 1 209 228 B1

(12) EUROPEAN PATENT SPECIFICATION

(45) Date of publication and mention of the grant of the patent:
**01.10.2008 Bulletin 2008/40**

(51) Int Cl.:
*C12N 15/00 (2006.01)* *C12N 15/65 (2006.01)*
*C12N 15/82 (2006.01)* *A01H 5/00 (2006.01)*

(21) Application number: **01127716.7**

(22) Date of filing: **21.11.2001**

# (54) Environmental stress responsive promoter

Promotoren, die auf Umweltstress reagieren

Promoteur sensible au stress environnemental

(84) Designated Contracting States:
**DE FR GB**

(30) Priority: **22.11.2000 JP 2000356652**
**05.10.2001 JP 2001309984**

(43) Date of publication of application:
**29.05.2002 Bulletin 2002/22**

(73) Proprietors:
- **RIKEN**
**Wako-shi,**
**Saitama 351-0198 (JP)**
- **Toyota Jidosha Kabushiki Kaisha**
**Toyota-shi, Aichi-ken, 471-8571 (JP)**

(72) Inventors:
- **Shinozaki, Kazuo**
**Tsukuba-shi,**
**Ibaraki 305-0074 (JP)**
- **Seki, Motoaki**
**Tsukuba-shi,**
**Ibaraki 305-0074 (JP)**
- **Nanjo, Tokihiko**
**Tsukuba-shi,**
**Ibaraki 305-0051 (JP)**

(74) Representative: **TBK-Patent**
**Bavariaring 4-6**
**80336 München (DE)**

(56) References cited:
- **KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156**
- **LIU QIANG ET AL: "Two transcription factors, DREB1 and DREB2, with an EREBP/AP2 DNA binding domain separate two cellular signal transduction pathways in drought-and low-temperature-responsive gene expression, respectively, in Arabidopsis" PLANT CELL, AMERICAN SOCIETY OF PLANT PHYSIOLOGISTS, ROCKVILLE, MD, US, vol. 10, no. 8, August 1998 (1998-08), pages 1391-1406, XP002145075 ISSN: 1040-4651**
- **SHINOZAKI K ET AL: "GENE EXPRESSION AND SIGNAL TRANSDUCTION IN WATER-STRESS RESPONSE" PLANT PHYSIOLOGY, ROCKWILLE, MD, US, vol. 115, no. 2, 1997, pages 327-334, XP001018199 ISSN: 0032-0889**
- **BOHNERT H J ET AL: "ADAPTATIONS TO ENVIRONMENTAL STRESSES" PLANT CELL, AMERICAN SOCIETY OF PLANT PHYSIOLOGISTS, ROCKVILLE, MD, US, vol. 7, July 1995 (1995-07), pages 1099-1111, XP001018275 ISSN: 1040-4651**
- **LIN X ET AL: "SEQUENCE AND ANALYSIS OF CHROMOSOME 2 OF THE PLANT ARABIDOPSIS THALIANA" NATURE, MACMILLAN JOURNALS LTD. LONDON, GB, vol. 402, 16 December 1999 (1999-12-16), pages 761-768, XP000877287 ISSN: 0028-0836**
- **DATABASE EM_PL [Online] Database accession no. AB010069 XP002200522**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE EM_EST [Online] Database accession no. BI272680 XP002200523
- DATABASE EM_PL [Online] Database accession no. AY039589 XP002200524

SEQUENCE LISTING

<110> RIKEN
TOYOTA MOTOR CORPORATION

<120> A STRESS-RESPONSIVE PROMOTER

<130> xxxxx

<150> JP 2001-309984
<151> 2001-10-05

<150> JP 2000-356652
<151> 2000-11-22

<160> 20

<170> PatentIn Ver. 2.0

<210> 1
<211> 2072
<212> DNA
<213> Arabidopsis thaliana

<400> 1

tgggctttg atccacatga cgatcatata gttctttcct tcaacacaaa acacttgaag 60
tgtatcttat caactaaact ctattccagc aaaattattt caaaattaga tatatgggag 120
aatttttaca ggccattcta gattttcatt aacgctgtga tttatccgtt aataagtacg 180
attaagagta aaacgacgtc gtttgcaatt gaaacaaaaa tccccaattc agaaataaga 240

aaccctagat cctcaaatct cttcttcttt tcttcttcga attcactcct gtattttgaa 300
atcaaagtga attgcaaggc acaaagttca tgctttaaag cactgcaaat ggcgaagtca 360
tgatttatat ggcatttttc ctgtttttgt ttcaattgca aacgacgtcg ttttgctctt 420
aaacggtctc attaacggat aaatcacatc ttctaattgc tctgtttatt cagttgatgt 480
tgtgactaat ttggctctgt caacgaaagt tagtgatttt atctgaagtc aacacgtaat 540
tgatgatttt aatcacaaag tacaaagttt atgtttaaag caccacaaat tgcgaagtcg 600
tgatttaaat gacatttttc ccgttctggt taaactaagt ttctattatt taatatggat 660
attgtggagc gtagtgtcag cactcagcag cttatttaac gtgatactct ttaaccgagt 720
aacaactagt gaggtcatat tcaggaccga tccaacaata ttgagggttt tactccaagt 780
aaaatttag tttatttt aattatcata aacgacataa atataatatg gaaagatcac 840
aaatactgat taaaaactaa aatcatcaaa acgaaaaaga aaaaagaaaa aattgggttc 900
aactctcatg agttattaaa cattttaggt tttaggctta aatctttaaa aaaaatcaga 960
actgaaaaac gaaaaattct aattttattt tggactctga ttcatagctt atgtcgctta 1020
tgtagttatg ctagggatga atctgtattt cgttaccgta atgagagttc gatactctct 1080
tacttgttac gattctggag catgttacat tttttctttt ccgtcaacaa caactttaat 1140
atggtaaaac aaaatttatt tttatttggc tggtcctact caagacaaat cttctgccga 1200
catcacataa tcatattaaa aaccataact tctgccactc tgtttttttt tttttttgta 1260
accattaact gattggattt tgatccatct catctgattt tttagctcaa caatttactt 1320
gcacattttc tatttggttt tatttatact tagttacata tatgattatc gaactagtat 1380
ctctttataa ttaagtattt ttctattttt ttttaattta gatttttgtg aattcattta 1440
cagtagaaaa ctgtaaaacc atatggtcta attatagaat gaaaacttca acgaatccat 1500
acaacttatt ggctaaatat aataaatctg cttgaagcat attgttatta tttagttgga 1560
tttgacgatc tctgacttta atgtataccg acatacccta tgatttagat gttgattttt 1620
cccattctta atatatccat gttaagagat tccaccataa catatctaat tatttgcatt 1680
gtaataaata ttatcattaa aaaaaaatac aactggacag ctggctcgtc ccattgtttc 1740
ttacgtccac caattacatt tgttaaagca aacttattag aacgttcatg tgtgagaagt 1800
tggtgtcgac atgtgtctaa ggtctatgtc agaaatcgga ttagcttatt aagtaaacta 1860
tactatatca ttgttaatat agataaaata tctagttcgt ccaaattaaa ctattttcat 1920
aactgccacg tggcgtaaac gtatccatcg agtcacttgt aatatcttta taaccaaagt 1980

cttccaacac attcatcacc atctatctac tctttactct cttctcttct cacatcaatt 2040
attcatagtt ctctcttctc cggcaagaaa ag 2072

<210> 2
<211> 2000
<212> DNA
<213> Arabidopsis thaliana

<400> 2

ctcctgtttc atgatcatat ataaaaggaa agagattgat tcagctaatc aaattcataa 60
acatggataa gatatcacaa actctaaacc tagctcaaac tatgaacaat agaggataac 120
cacttgaaac ctaattttaa tcagaagatt ttagtacttg tgctgcttat tcaaaattcc 180
tttgcccaaa aaaaaaaagt ttgacttacc ttttgaaaga cgaacgtatc cactatggaa 240
caatctttcg taatctcact ttctagtttg gtatttaaaa aacccaaaat ttgattgtca 300
aattaaatac aataagagat tcctaaaccc agaattcaat tgcgaaatca aacagagacg 360
atacagagag agcgagagag cgagagagag agagtgattt acgtgagaac gaatcggata 420
aacggaaatt tccagaagcc ggatgagttt cccgctattg tgatgagtaa caaaacaaat 480
tcgggagttg tctatttaat acttatcaat agcccatgaa cgcagagacg tatggacgcg 540
agaatcgtta gtacgtgaca cgtgtaaacc agtgacgtca ccgctctcgt cacaaacccg 600
ctagtattgt cctcttctat tttatctcac acaatcatgt taggttatca tagtaccact 660
aaacatttat gtgagaatgg atggatatta gacaaccgac gacaaataaa acaaacaaaa 720
gtagatagac gaccataaat taaactataa acgaagattg agggaaaaaa tcattggttt 780
agattttcga acaaatatcg tgaaaagttt ttcatcttgt agtttttgta attaatggtg 840
tatgaaaaat cattggtttg tttagttgaa ttaaatttca gtaaacggtt tagagtatga 900
acccactcaa taaagatttg actgccaagc gttactggtg tctggtggat tgaatagtta 960
agagtataag gtttgggcca ttatgtggtt tttattaaag ttgaaggtct catttattaa 1020
ttccttagag ctgatggcta atgtacaacg aatttgattc aggagtaaga taaacggtac 1080
acccaaacac gcgacgcgag cgaagaagac gatgacgaac aaggagaaag ctcgagagag 1140
aagggagaaa agaatgcagg agatctctct ccttcgaact attccttact ctgaccacca 1200

taggtatgaa gagacctaat ttgtccaatt gtttcggctt ctttgtatcc taagatctat 1260
catcatttac tctgcgagat tctcaatttc acagcattcg atgtgttaat ctctaatttc 1320
acttgcttaa atccatttct tagtgtcgct tacagtgttt gatgagtttc tatttttctc 1380
tatttgggag ttggagactg aattggtatt gttgttggta cttacagatt tgtgtttcac 1440
taaaccaaaa gaatttagag aaattgaaaa tgatgaagta tgtgtctctc aagccctctt 1500
tttgaatttg acttgtcatt aggcgtcaaa tgatttttta ggtggtggtc ttgtgaaaat 1560
gtagcagtag tgactggttc aaaccgcggg attggattcg agattgcaag acagcttgcg 1620
gttcacggat tgacggttgt tcttacagct agaaacgtga atgctggtct tgaagcagtt 1680
aaatctttga ggcaccaaga agaaggtctc aaggtttatt ttcatcaact tgatgtcaca 1740
gactcttcgt cgattagaga gtttggttgc tggcttaagc aaacatttgg aggtttagat 1800
attctcgtaa gatccttatc gattcgtaat cttggaggat agttctaatg ttttcttaga 1860
tttgaattac atgcatttgt gttgctatgt ttttcaggtg aataatgcag gtgttaacta 1920
caatctcggc tcagataata cggttgaatt tgctgaaaca gttatatcta ctaactacca 1980
aggaaccaaa aacatgacaa 2000

<210> 3
<211> 2000
<212> DNA
<213> Arabidopsis thaliana

<400> 3

catataccaa ccgcctaaca ttacagcctg tccccatccc ctatctttat ttttcttttt 60
ggctcattta gcttcaactt tttttaatag gtgtgattag aacatactaa aaaaaacata 120
tgttttaact tatattatat gaataaagtg ttgtttttaa tagtttatat ttctattaca 180
agtacaatat ataggatatt ttggttaaca attttaaatt taaaagatat tgtgagtaca 240
aatcattaag cttaacacaa atgttcattg cttcttaatt cttacaatcg gaaaatttgg 300
acaaaaaatc tagactaatc tccaaaacat tgtgaaaaat tggggttttt ttttatcatt 360
taaattgtct atagtggtca atcattatta aaaaaaattg tttttttgttg aaattttaaa 420
ttataaaaaa aaaacacgtt tatacataaa atgcaaaaac ttttcttaaa ccaaattaca 480

gagaaaggct atcaccatcc ttgtcttgat gccaaccaca cttgtaatcc caaaatacat 540
acttaaacta tactatacta acatgaattg gttatacgat tatctatcta gttagctaca 600
taattcatat aattagtttt ttaaaatata cttatgtaat gatagtaact aaaaaatata 660
ctatctcgag aaatccaacc aaaatgctat gatagtatat aaaataaaat gaaagaaaga 720
ggaaccaagt tgtgaaacca tttaccaaga agaagaagaa aaaaacaaga tggtcgagtc 780
tcgtgatagt atgaagctgg ggccgaaacc acgaatcaaa gaaccatttg acgatgcaag 840
tggtcatcga taaccaatca atagaaactc caccgcctca aaatatcata gtgctattta 900
atttttcata ctatcattta ttgtaggcta taacatattt ttgtattgta gatggacggc 960
tagtacacgc aagttagtca ataaactttt aacaaatggc atcttgacat gcttaaacac 1020
attttataat tcgaacctca ctttgcctac tttacacacg tctacttata gtagatgagc 1080
atcaatcatt gaaccgttcg tataatacaa tttttttttt gtaatacgtt ataattagaa 1140
agtaatggaa aataagttga aacacaaatt ttcaacggtt ttactaagat ttaattttac 1200
aatttcaaaa atataaccga attatttaaa tcgtattaaa tttataatta attttctaaa 1260
aaaaaacaca gattaaaaaa atatattaaa actaatcata aatatattaa tttttgtaat 1320
ttatattttt ttataacttt aaactaataa tattcaatta tttatcttaa agtttacaaa 1380
ttatcaataa taactaataa ataatgcata aaaacttttt aaaaattaac tttttgaaac 1440
aaattttttt ctaaaaaatt aaataataaa gaacagaaaa aatattattc aactaaaaat 1500
attggataat aactctgaaa gtttgcattt ttcaaataaa tcattgattt tttttgtttg 1560
acataaaaca ttaatttccc cttgtaaatc acacatttaa aatctaacta tatttaaaca 1620
aaaaaaaaaa gtccaatttt gactttaatt atataaaaaa agcaaagcta attatgggtc 1680
aaaagactca aaggccagaa ttgacgcagc cgtttatgag aagtgagaag acaatttcgg 1740
agtcacttcc tttatttcct ctcttccttc actctctcct atataaacct tcctctcctc 1800
ttcctctctt ctcatctctt caaaccattt tcgaaagcct tgagagagag aacacagacg 1860
ataccaactt tcttcaatct cgttgccgca gtataattat ctcattcctc ggatatatct 1920
ctccttctgc ggcggcgaca agaagctaca agaataaaaa gtctgttttc tctcttttca 1980
agaaaccact tacttcgaaa 2000

<210> 4
<211> 2000
<212> DNA
<213> Arabidopsis thaliana

<400> 4

agcaagtagg ttacgtggaa ggaagatatc aacgcacaaa aagagaaaca ctcaacgtta 60
agggaccaac cacaaatctt ggatttttgc tgatgcagtg tgtgaaaaca ggtccacacg 120
ctttgtactt ttttcatcat cggtctacaa taaagctcaa gtctttccat ggacagagat 180
aagcaagaaa aagtattaac attgttcacc tcaaaataat ttaggaaaat gacaatgaga 240
tttttacctc aaaagccaaa atcaacatct acccattctt tcactcgtca caatcatgag 300
tttcttacaa tgcttacctt agagtttaga gacatattct cacaaaaaaa gcttgtgtct 360
atttcaaatt cttgaccgta gatgtcacaa catgcatata tcattgaaaa cagagcaaca 420
caggaaacca agcatatgta tctagatata cttagcaaga cataactata gtctttgaat 480
caacataggg attaatgata gagaatgagg aagctcaaga ttttatactc agtttcttac 540
aaaacaaatt tctctctaac tgcaaaaaca ccaattagga tttgaagagc gtacctgttt 600
gagtcaatgt ccaatgtcgt ccccccgcct tctacatttc ttagcctgct gaataaaagc 660
acaagccaaa atgagaaggt gccaaaggcg ataaggatca atttctacca ttcaaaaaac 720
taatggtgag aattagaaac gagagaaaac tacttgttga ggaaatagcc aaaagcgcaa 780
tcttcgtcac ctgaataaag accaaaccgt cactttcaat gagtcagcaa gaaaaagaga 840
gagagagaga gagagattct ctataacatt tgagtcgaca tggattctaa tgcatcaaaa 900
gtcatctcca ataaacaaac acttgaaact cacatggcta atagaacaag atcaaagcct 960
taagtattaa gcattacaga cactactggc taacttttga cacatgttct taagtaacat 1020
agtatcaata tccgtgaatc acatcgaaca cacacaacaa gggcttaatg catcaaagtc 1080
ctgttatttc catataacaa catatttcat ttacaaacag aatgcagcat tcaggcagtc 1140
caaatggaaa ggttgacaaa aaaatataat cttgtaactc tacatatatg gcagaatgta 1200
ataaccaggc aagaaaaaaa cagaataaac agatcaatga gtatgatata aaaaaaagtc 1260
acaaagaatg tgccacagtg aacaagaggg ccatgagaag aaatttcaa agaaaatatt 1320
agcattgtta gaatttttg ggtcaatgga tctgtcagct gcttagttgg aaaacacaaa 1380
tcttacagga aggaaagtcc aagaaaaaga aaataagcaa agttaatagc caccacaaga 1440
aatttcatac agaaataatt aaatcgttgc acttatcttc ttattcaaac taaaatcaag 1500

agaacttaat aattttcagc cacacgaacc atgtgttcaa agccaaaggt gagaagccaa 1560
aattatcagc ttatctccat taacaaggga aaagcaagac tagatttaag agttctctgt 1620
aactaaaaac tgcaggagtg agtaagtaaa taattcacca acaggaaaac aaaactcaat 1680
tatctatagc tgaatacaca tgtaaatgag aatttattaa ctaaaacatc ttcctttgta 1740
actgatgtga catttacaat ttttcatttt gaggtgtaag aaccgtgtga caagtgaaaa 1800
ggttaaaata agcaaccttt gtgatatttt ctctccactt tttgaaattg ggtctccaaa 1860
ccacagccaa tcaatattct ttataaatac aaacacacaa acagcatctt tctctcaaac 1920
acaaacatat cttctatcaa acaccaacag ctctattctc tacctcattt ctcatcataa 1980
caaagagaga gaaaaaaact 2000

<210> 5
<211> 2000
<212> DNA
<213> Arabidopsis thaliana

<400> 5

tttctggatc acgttcacat gccgataatt attgttaagt tgtttgcata ttttattatg 60
tgccttgttt gtgggatttt cgctaatttc ttttctcgtt tttcgttct cctcttccct 120
caattatttt ctcaacttcc tattcgacca aagcattaat tctacacacg acagtaaagg 180
acccactcaa acaataatta gttttcaact ctttcagaat atattttagt gacactaaaa 240
tagcatatca taaacacaac agaggccaac gactctgaat gatccgtacc gcagtggaaa 300
taacagtccg gttatgtcgg taggctatat aagccactac tttcatttca atttctcaac 360
tttgttgtac ttagttcgta tcctatcagt ttatcatcac tttaatttgt taccatatag 420
tatctgtaaa aactaattaa tatttatgaa tcatgctcat attttcaaat cttagagcta 480
ctcaacagaa cacacacaca tacagtcaaa attttatact acataatttt tgttttaagt 540
ttacagttat aaaccaaaca aatcacctca caaaagtata tagttatacc aaaccaagca 600
cctcataaat gaaaacaaaa ttaaaaataa taatatatat aataagaaat attcatcaat 660
actaacgttc aattttagta aattggcaaa aataaaatat tagtatcact tccagagatt 720
acattattat ttggtgaatt tggcaaaaat aaaatatttc gttggtggat gctttgctgg 780

gacgactttg tttattaccg tcatattgta gtaacctaat tttattacct aacattcatt 840
tgaacattaa aaacattaat gtcatttgat tggaagagtt tattaatgac cattatttcc 900
ttatttccaa acctaataaa cccattattt ccttatatta aattatggct tgattggtgc 960
aatttctttc tagctctttc tgtttccgtc ttgattcgaa taaaaacgac aaatgttatc 1020
tctacccgta tccgattatg tttaatatat tatgcattga tatttcacgc tacgatctgg 1080
accgacgttt agtgtggacc ctgtcacgag ttttatctat gacggaccaa aagaaaaact 1140
aattaaagca gaatatcaaa gttagtcggg taagttccaa ctaataaccg agccctgttt 1200
ggctttgagc attagtagta atcaaagtaa acatatgata gaaaagttat tataatctat 1260
tatatacaga ttaatatgta cgattccaga ttctatttat gtttctcgta tgtaaaacat 1320
ataatgtgaa cctacttaaa ctatactaag actattagtt tggtttcaaa ttagattaat 1380
tagtttaacc aaactgaaca tatccctaga ttgtaaaagt ctaatatatc cactaattat 1440
ttttgcttgc cgacctctaa tccacaactt tttgtttttc catttccact gattttcaac 1500
attccaccgg gtgttaccaa cagtaacata catacagtga atttttcaa cactccaccg 1560
catgttacca atagtagcaa aaccctcgac aaaaatttgt accgactata aatattcaac 1620
tccaactctt tcctctccac acactcaaat atctctccaa cactaaaata attgtaccga 1680
ctctctctct ctctctctcc gtaacaaaaa aatcaccaat ggcaaaacaa tatctctttg 1740
tactcctctc aatctcctat ctcttatcac tggagctcac ggcggccacc gcagcctcac 1800
agaccggagc ttccaaaaaa gccataaact tcatccaatc ttcttgcaaa accaccacat 1860
accctgcctt atgtgtccac tcactctccg tctacgcaaa cgacatccaa acaagcccta 1920
aacgtttagc tgagaccgct atagccgtga cactaagccg agcccaatcc acgaagctct 1980
tcgtctcgcg tctaacacgt 2000

<210> 6
<211> 2000
<212> DNA
<213> Arabidopsis thaliana

<400> 6

attatatcta taataacatg attctatttc aatcgagtat gtaaaaaaag taaaaatttg 60

atcgattaat catgtagtaa tctcgtgttt ttttccaaca aataattgat taaaagttta 120
aaacatatca taaatcaaaa ccgaatgagt aatccggatt gagtctagaa ttacaagtta 180
agttatctgt catacggtgg taatctcata ttttttgtag aatccttaag agttgagaac 240
tttcaagaac agttaagttt aataatattt taatctttgt ataagagatt tcttcattct 300
tctaaaacca caacaccttg tgatcgacaa ttcatacgta catatttgag atttaatttg 360
ttttccttat ataagaaatc aataatctta ttgtatatga ctaatttatt atataaaaac 420
ccttactagg ggttatataa gacacgtaga aatgtctcca acatacagta ctataccaca 480
aaattcagaa atttgaacaa ccaaagcaat cagaaaacaa tattcatttt tgttttattg 540
gaaaagattg agccaatatt tcttaaataa ctccttttta gataatcttg gaaccgggct 600
tgttcgagcg ggtcgtgcca gccgggctgc tagggctgtt ggtagccgga ctcatcccga 660
gagtgttctt cacagcatcg gctgctcctt gtgccatgtt cttcacttgt ccacccgtct 720
acactccaaa atcataatta atgcatataa ttgtctataa ttaataataa tgaacaaaat 780
ttttgagaag aatcgttaac ctgttggatg acactagagg cttgagaaat tagggaagga 840
ttgttctgat catgctctga gtgtaactgt gactgtgagt gggtgtgatg atcagcattc 900
tggttcattg cgtgtgatac attgttcaag tactcttcct tcttcagcta taaataaaca 960
tatatttgat ggttcattca tcacaaaaat cagatttcga aaattgtcaa atttcagatc 1020
atataagcaa aacaaatgtc gctattttgt gtgttgtatg tacctgaact tgaccggtga 1080
cttctccggc gctgtggctc aactgctggc tcgacgacat cttcttggtt gttctgtgaa 1140
atgtcttaat taattaagat tgtaagcttg tagaatactt gaagggattt gatatgcatt 1200
ataatggtat ttatataatt agaaagtgta tattttagta aaatcctaaa tctaagcatt 1260
acactaacac gtggaaaata acataccatt gacgattgac atggctaatt ttttgtggag 1320
gtgaatagtt tgaggattta ttaccctaac gttgcttggt caagaagtga agtaggatga 1380
caggcaatag gaagatctta aacctttttt tccggtgaca attatttatg actttttatt 1440
gttgtcaaaa aatatattat cagtaatata tcaataacga atacaataaa aactcatccg 1500
atcgatttc aagaatttat agctatatta aaattacttc gaatccatgt aagaattgtg 1560
tattggttct ttttagaaaa aagtaaatat ctatgcagta atggcggttg cataatatat 1620
gccttgagta gatgaatatc caatatcaag ataacgtgag tcaccacgtg tctaacatct 1680
tccgtagctc cgtttttacc atgacgtgtc acatagatat aggtcatcat gaaaacgaga 1740
aacctaactt taacactcgc acataactcc aagtttcgaa acttcgtcac atcaacctaa 1800

tcggggcacg tacctacaca cctgtcgcga aactgcaaca cctatcttgt tctctcgccg 1860
accaagactt gctataaata actctgacta acgagtcgga gacaactcac agttccaaac 1920
acacaaaaaa cacaagatct aaaaaaaaaa gcttttatca tttagaaaaa tttggtttcg 1980
aatttcttcg aagagtgaaa 2000

<210> 7
<211> 2000
<212> DNA
<213> Arabidopsis thaliana

<400> 7

tgaacaaatt taatgcagaa tttagtggag gatcagttgt gattttcctt cggtaaaatc 60
attttcagtt attgacattt gaattgttca atttcaaatt tcaaatatta taattcaatt 120
caaagactag ctaagttttt aaaagtaatt agtgagagat ctcacagtga tataaaccac 180
attcatttta attcatagca aagtaaatgg taagaaatga aaatggaaga aagagaggag 240
agtagttcac acgaacatct gaaggagaag ttgagagagc ttgaggagga atggacagcc 300
atgaaaacag gtaaaaactc atctgcggtt tcatggatta ccgtggaaga tgctcttgag 360
tatgtcgaaa actctcccag aaacctaatg ctctctctcc aacacaaacc aaaggcagag 420
atgatacaag aaatatcgcc attaaggaga aaactctttc acgactctga tgatgatgat 480
caaactaaga agacgacatt gctttctcat tcatcttgtt ggtcttcgaa tgtgacgagt 540
tcgagtgata ccacgaaagc aaagaagaag acaacgatta gaagatttgt ttctgttaca 600
atggtgctgc ttttgtcatg ggttttagtt gtgttgatga accactttga tcatctctcc 660
atgaatacac aaatcattac tttggttcct acttgatatt tttatttatt ttggtttgag 720
aatgttcatc aatctcaata aataaaaaaa gtttagtttt gtattattac ctctcgcctt 780
ctcttttaat tcattgaacc tcttcaactt tggctgtaac tatgttggtt aaaacagtat 840
aagtaatgta tgtatttatg gttttaggaa aatcttacaa aataatactc tatcattgga 900
tgaagaagca aaaactacac ccctctacat cttttctatt cataaacttt tttggggaaa 960
gaactacatt ggtggtgatt aggtgcatcg gtgacactaa tgttgaaaat atgttttgtg 1020
taaaattagt atggttaaag ctaatcattt agttgagcaa aaaaagatgc taatcattta 1080

aatagaaaat ggactcaaac aaaagaaggc cacacattca attgttacat ctttattggg 1140
ttttcatttt tggaagctat ttttatgaat ttgcagtgtt ttgttgccca aaagaaaaaa 1200
agcataattc caagtatatc tttcattttg aatccatcag tcatatgttt tacttgaatc 1260
aactatttgt tgggttttac gaagttactt gttaagaaga ttcaaaatat atataattag 1320
actatcaaag gaacaaaaga atgtaattgg gtcataactt tttccgaagc ttacgtgact 1380
tgggagaaaa tcaaacccat tagtttcacc cttaatcaaa ctaatcacaa attttgaact 1440
ttgcaaagaa ggtaaaataa taatcagcaa aaagattaat aaaatgacaa aagtatctct 1500
ttaaaagatt aataaagaaa taaagaaatg acataagctc tcaccgaccg acgtcttgtc 1560
tttttattc ttaaggtcgg tgccatgtcg acgccttcaa gcttagaatc tcgccacgtg 1620
tgggataaac taaccgaacc caatcgaacc agacagacca aagacagacc agaccggact 1680
agttgtatgt cggcatcagc gttctagtac gacgttaatt aaaaaaataa aaataaaata 1740
ttatttatga tattccacca ataagagcca atgaaaagtg tgtgaaccaa ccaacttctg 1800
tagcaaaccg taccatttct ctcttagata cctttacctt ccttctataa attaggttct 1860
tcctttcaca tttctttgtc ttcttcttct acataaaatt gattcgcctc gaaatttaca 1920
cagacttgtc gctctctctt tctctcttat ttcacagagc ccctaaacat ttccaaaaat 1980
cttgcaaagg aagaaaaaca 2000

<210> 8
<211> 2000
<212> DNA
<213> Arabidopsis thaliana

<400> 8

aaatatcatc atttatatca aatttatgat atataaaata tcattatcat tttaaaataa 60
cactaaaata atcagtgcaa ttccgtataa tcttcatatg aaactgcaag aatccttggt 120
gttccaaaga ttatgatgat tattgtgagc tttgatttat ggtttccatc ttgtcaaaaa 180
atggctttta taaatggtag agctttctta gttttcctta agatatacac aacaggttac 240
aaatataatt tatttttgct aaaagaaata gttatagtaa acttgtaaaa actactacaa 300
ctattttatg gaaaatgtct taaagctgtt tcaactttta tacacattac tgcatttact 360

attctgaaat caatttttaa ttaaggtcct tgtactataa ggaatccttt aaataaatat 420
aaatattaac aataaaaaaa tccaaaattt cagaatgcaa tggtcttaca attacaataa 480
taatgtattt gattgtacta aaaatatacc ttaaatactc accaattatc ttttataata 540
taatccacaa atttctggaa gaggaaaaac aagtaaatgc aagtcacatt tcacatttca 600
aattaccaac taccaactac aaactacttc cgcgtctcaa attgaaccac taattaatca 660
ctactttata tgctcatcat tctttttctt tcattacaat tctagaataa tgacttaacc 720
aaaattcata caaataaaac aatattttgg cttccttcac caggctaaga atttggtat 780
tgaaaatcta agtacttcat aagaaactgt tggaatcatt taaaatttgt gatcatatat 840
aaaaaaattt attcatcttt atatttaaga gtttaaaaac tgcaactttt gtttttcttt 900
cactaagtct tatggccaca gttaattaaa agcagatgaa aggtggtcca atggaaaagg 960
agaatgtgat tgggctagtt gggagagttc tgatgtctag tgttgggtac acgtgtccgt 1020
cagttacaca tagcattaaa tcagacggca tgtcattatt caaatctagt tcacatagta 1080
cgactaatag ctgataaatt aatgattata cagcatatga attatgaatt caaaaaaaaa 1140
aaaaaattga aaatgttaag gagatgctat attttacaaa attcatcgca atgctttcta 1200
ctaatttgct aagtggtctt ctccagttag tcttgtcgat tccaagcgat attattaaat 1260
cttgaagcat cgctcaaagc attatagctt aagataacca aattgttatt aaaaacacct 1320
agtgaaattt ttaaattaaa acaattttga tatctttgta atatctaata ctactctttc 1380
tgtgtctaaa aggattaatt ttcaaaaatt tcacacatat taaaaaaaaa aaaaaattac 1440
tagctaaaca attttcaata atcataaaac aatagtaact taataatttt tttttatttt 1500
caaaatagtc cttcaagttt acaattcatt ttagtattat aatcaacaaa atttgtatta 1560
aaaagttgga aaattaatct ttgtggaaca aaaaaatcta gaaatcattt tttagaatta 1620
gagagaggtt tgataaaaaa aaataaaaaa aaatagagag aggtagtaca tactaaacga 1680
tgtgatacta ctattgacaa aatcttaatt ctcagtttag tagaataaac tagaaggaat 1740
gaatgaagta aatgcgaatc caactactaa caaaccctac ttagtcatca tattttccca 1800
tatgaaatcc ctatataaac ccatcatcat ctcccacttt tttcatatcc aacaacaaaa 1860
acataagcta agaaaacgaa actcaactaa ttttgttatc acccaaaaag aagttcaaac 1920
acaatggctt tcgctttgag gttcttcaca tgccttgttt taacggtgtg catagttgca 1980
tcagtcgatg ctgcaatctc 2000

<210> 9

<211> 2000
<212> DNA
<213> Arabidopsis thaliana

<400> 9

tcaatttgag acgcggaagt agtttgtagt tggtagttgg taatttgaaa tgtgaaatgt 60
gacttgcatt tacttgtttt tcctcttcca gaaatttgtg gattatatta taaaagataa 120
ttggtgagta tttaaggtat atttttagta caatcaaata cattattatt gtaattgtaa 180
gaccattgca ttctgaaatt ttggattttt ttattgttaa tatttatatt tatttaaagg 240
attccttata gtacaaggac cttaattaaa aattgatttc agaatagtaa atgcagtaat 300
gtgtataaaa gttgaaacag ctttaagaca ttttccataa aatagttgta gtagttttta 360
caagtttact ataactattt cttttagcaa aaataaatta tatttgtaac ctgttgtgta 420
tatcttaagg aaaactaaga aagctctacc atttataaaa gccatttttt gacaagatgg 480
aaaccataaa tcaaagctca caataatcat cataatcttt ggaacaccaa ggattcttgc 540
agtttcatat gaagattata cggaattgca ctgattattt tagtgttatt ttaaaatgat 600
aatgatattt tatatatcat aaatttgata taaatgatga tattttacag aatgaaatgt 660
tgttatacat actatacctt aagagcatat aacatgatat aattgctaca ttagtgtcgg 720
tagaggtttg tgtagtcaaa ttgtgactat taagtactac acatgtgaga catttatcaa 780
tcttgtacga tgttgcttga cggtttttgg ctgatagtat ccaacaccat acatcaacct 840
ttcacgtccc aagctctcta agacatcgga gaagcatcat cacatgcgta cgtagaaagg 900
aaacacaaaa gttaaagaca ttttgtaata tgtgtaacta tgagacatat atgagactgc 960
atgtgactat gagttatata tggcttatag agcgatagtg ttatggagat tatattgcat 1020
tcatgctatg tactgcataa gacgaattcg gtttcgtgta tttattcccg cttgagtcta 1080
gaattggttt caagtatagt tagaaaaaga ctgttttttt cgtcctatta tggttcgtat 1140
acaaaattat gcattgaatt gatgtggcat tcacgtaaaa atcttcctgc agggctataa 1200
aacgtatttt tggcgtacga ttataataaa ttttgttatt attcagatta tttattaaat 1260
ctttttgtt gttgacaaac gcatttatgt taatgattat acgaaatatc gagaaaaact 1320
ccatatagca cctctatcat tcaattatgt agtaagtcat aaaatgtgaa ctttgctaat 1380

```
tataatggcc ataattagta aacgtgatgg gagaattgtg atgtttagtg ttggatacac 1440
gtgtctatca gctgcacatc gcatttaatt ggacggtatt acatatatag tacacataat 1500
agctgacatt tcatctacaa cgaagataca aatttttaaa cgatttctc ttgtctacta 1560
cagtataaca tcgctctaaa catgctaatt aaaaaaacaa gtttaattaa ctgcagataa 1620
atgaatatat atttttaac agtatagctg tatatatatt gacgtattgg tctttgtaca 1680
ttctgaaaac tgatactatt gtttacataa ttacatatga ctggagaaac taattatttt 1740
ggttagtaga agaaacataa ctacatatga ctggagaaac taattatttt cgttagtaga 1800
agaaactaga aagaatgcat gaagtaaatg caaaaccaac tactaacaaa ccctacttag 1860
tcatcatttt tcccccatac gaaatcccta tataaaccca tcatcatctc ccacttttct 1920
cacatccaac aacaaaacac aagcgtagaa aacaaaactc aactaattgt gttatcaccc 1980
aaaagagaag agcaaacaca                                             2000
```

<210> 10
<211> 2000
<212> DNA
<213> Arabidopsis thaliana

<400> 10

```
ttttcattga ttacatttac aaatttttgg ttcatgttat tagtaaaata gatgtttatg 60
aacaaaattg agctaacata tgacctctca taatatattg ttgatatcag ttttaatatc 120
aatacattaa taataccggg aaaaaaaatc attcccaagt tttgtaaaga agatgttcat 180
ataatttaag tttaaaaggg tataaccaac aaaatgatta caaatgcgtc gatgggataa 240
ataatagata tgagagaacc atacaaatat gatagtgaac aaaataaaat ctagtataat 300
tatgggtcaa aaaaacataa aaagagggga aaagaaaaga taaagggaaa atcaaaaggg 360
atgaggcaaa atagagagtt tcaataaaga taaatttggg tgatggtgac ggaataattc 420
ttcgatgttt tcgttttgac catcttttcc tttatttctc actttaattt tcctttattt 480
ctctcgctgt ttattcccat aaaattttta aaattaacta gaacaaagca caaaaatggg 540
caatcttcga aatatactaa aattttctat tataattaac aaaataaaaa agagagggaa 600
```

actgctaata tctggagtcc attctcttgg agaagagtaa agtgggcaaa caaaaaaaaa 660
ggaactgagc tgattcgatt ctctttggga ctcccatcca tgcatcacct ctttcatagt 720
ttcattttac ttttatgttt attattttac aagattaagt aattagcttt tttaactgca 780
agcataacaa ataacaaaac ctttattacg gtctcaaacg ctcaaaaatg tttttaaaca 840
tagtaaaagg tttcgttcaa tacaatttga aaaaggaaaa aacaaaaaca attttcttac 900
aaatagatgg ccaccaacga ttaattatta ccgtcaaact tataattggg atgactcggt 960
ggttttgcga tggcgtttgt tccaaaagag tgccatattg ttcatttgtt cttatatatg 1020
ttttaatcca taacacgttt tggatcaatg ttttcaagta atcaaagtga aagtagagaa 1080
tatatcaatt ggtgaaaatt ttgaaatatc aatgtaattc tttttaagtg gatccattga 1140
tcaaggaggc cactaaaatt tgctcgaggc ttggtagtgg gttcatttgt gtttgataaa 1200
tacacttttt agtactcatc cccttttttt gtctgacatc gtatatgtat attggttaca 1260
tctaaatttg tcacacttgt gtagcagtga tagttgtctc agtgtgtcta tagactatag 1320
ttaaatgtta attagtagta tttatacaat actaattaca tcatctaaat atttgcatat 1380
gattcacaca aggcaaattt ccttggctaa ttgtcacact atttatgtga aggtgagtag 1440
ttaaagctaa taacctcgaa gttgcttggt ttatatgaga tcaagtttgg ctcttgtaag 1500
tgaagataat aatttaaaga gtattttaat cgtgataatc taaatgttag ttgatagtta 1560
atacgtcacg gaacatgtac cgaccgacgg ccgatttcca tgatacaata aaaaaatgaa 1620
aattagcaat ctcataacat gcgacgacga tacttatggt gttgccacgt agcaagcatc 1680
ttcctcttaa ccatgaggtg tcgcaacctc agaggacaac acggacaaga ccgagaaacc 1740
gcatactaac gcttgcaacg taaagcacaa cacccctaaa ctcctatttt tttaatttc 1800
ttttaacctc aacacaccac gcagctatac acgtttcaat gtgctaatac gtgtcttctt 1860
ctccgccgac caagacacac tacaaatgtc ccgatggttt ggggagacaa atcacagttt 1920
ctactacaac aacaaatact tttacgaaaa aagcttttaa aacttatcta gtttcgtttg 1980
atacacattt gaagaaagaa 2000

<210> 11
<211> 2000
<212> DNA
<213> Arabidopsis thaliana

<400> 11

attaccataa tcataatatc atggtgccga ctatatggct cgagtcttta tcgtttattt 60
ttatttctta aatcattgac ttataaaccg cccatgagca ttaaataatt tgtctcaaaa 120
actcgaaaga aaatcgttat tgaaaatggt ttaataacca aatctaatcc tttccgactc 180
atattggttt cattataaca cttaacaagt aaaagaattt tactcgactg cttcagccac 240
aatgcggtag aagtcgccaa aggaccattc ttgatcgtca tttttttctt ctaagcgttt 300
gcggtagcaa ttttcagcca tagcttccaa gtctttcgcg tctacctctc ctttaccttt 360
tgcttcgttc cataattaag tataccaaac aaaaaaatga taattttatt aatgaagtga 420
acatcaatga aagacgtgat tttgggggag aaagagaaag gacgaaccgg tattgagagc 480
ttgacccata ttgtgataac ttccttcttc ttgttttttt gcatatcgta acaaatttga 540
aggataatat tgagaaataa attttggaga ccgtgacttg tgacttgtga ggtgggatca 600
gtatcttggc atttcctgat cgataagcca cgtaccggaa gcgtgacagt ttaacttaag 660
gatctagaga aaaagtgtga gtctattcat ttcatatttt acatatagca tataaatact 720
attttatttt cctttgttta cagtttaata gttttagact cacaatatga cttttgaaag 780
ccaaaatgta gcagaacaat taagtttttt taatattcct tttgaaatgc aaatatgttt 840
ttaacctata taactggcaa cattaaaagg aagagattct catgatttgc aaaatatcaa 900
ggtggaaaag gcatgtaaat ctttatttaa tatatatctt tgtttaaaac cacattggag 960
ataattgacc aaaaaaaata caattttgtc aaaaagctta aattattgga ttgttaatga 1020
gcatatggaa gtttgacact tggagaaaat tcacatgtca ccacatagag ataatgtatt 1080
tctatgttca ctaacacgat tacatttaca ccccattccc tcgtcgacct tgagtcactc 1140
ccatctcctt ctttggactc ttgcgagaca cttagattta gaacaacaaa tgagtttaag 1200
taagtactaa gcctatgaaa aaaaaaaaag gtagaataag cttaagaatc cagcaacatt 1260
catggtatca ttcgccattt atatcttaaa cgtgaaagaa ataaagagag aaaggttatg 1320
aattcctttt gtcaatagat atcattggaa ctaagcgtta tgaattcttt ttgtcaatag 1380
gctaaaaacc gttttatata ttttacaaga aacatgattt ttccataata ttgaaacttt 1440
atatatttct taatattcta ggcatttata aattattcta tgttttgaat atttatttta 1500
aatttttgga gaatactaga acatataact ttccaagtcc tagtgatccc ggctacggca 1560

acacatccct acgagggatc aatatatcga accatctcaa cgcaacactt gtcgtcgatc 1620
aaaagtctaa aaacattgct tctcaacttt gatctctttc ctctagtctc tatcatcacc 1680
gccacgtcac tattttcccg ccttataact aggggcaatc aactctcgcg aacagagaga 1740
gacggagaac aagagcaatc aaatcacaca aatcctcgaa aaagtaaaat tcttgattaa 1800
aaaaaaacgg acattttgtg ctcagacact cagctttcct tattagtctt ttgccacctc 1860
tctgcaaagt tctcgaaccc actctgtaac caaaatcaat cataaagatt tgatcttttt 1920
ttttcctttt tcggtgagat ttaatcctct aaggttcttg atactctcta gatctattct 1980
tacttgggtt tgttcgaaat 2000

<210> 12
<211> 2000
<212> DNA
<213> Arabidopsis thaliana

<400> 12

aaattaaaaa aaaaaaaaca ttgatacaat tcttaccttc agtttttaaa atttcctgtg 60
cactatttcc ttccttgggt gtcttgcgac tgctttttga ctttgttttt ggagattttt 120
cctttcgaac tgtcttaact gctggttctt cctctgtagc ttcaagaaca aaatccgcat 180
ccttttcagt tgacacagtc acttcctgct ttataggaga ggatggctca gaatcagatg 240
agacaagcca tacagaatca gctcctccct gctaatgata catttaaata atgttaacat 300
atcttctcaa cactatacca aattaagagt tagtagtttt acacccctac gatattatat 360
tccataaatt aaactgatcc atgcagacac aaaaccttga aataaaaaac taagacagat 420
aagagtgctg gcgatagatc accaactaat ttcatcatat ctcatagaag tccaaagctg 480
tcctagagtt agtttataac tagtatacca ctatcttgac aatacaatcc atttatcagg 540
cagatacatt ctgacaagaa acacttgaat cacaatatcg acccactaaa gcaagtagag 600
gcaacagaaa cactaaggag tacgatggag acagtgtcca aaaggttctt acttgagcat 660
ccttatgctt gctagagagt ttttcacagt caacgttgtt tccattctcc ttccctgcac 720
acgaatctgc ccgagtcaaa tacatgcttt taagagctca acagttgaac aatactcccc 780

taaattcata aattgatatt ctctccctcg ccctccaaga aggaaatgac ccataaatgt 840
ttgttcctct aatctcaaag caaaacaaca cagaaaatct tccatacctt caaggttgag 900
actagcatct gctttcttct ttctagaaaa cacctgctcg atactcactt gcttcgtcac 960
aaccttcgtt tcggaattct tcctagtcag taactccaca gattctgtct caagaaccac 1020
aatgtcgtca ccgtcatcat caggcaaagg aagagacgaa atgacttcag attccctata 1080
aggactatca tcatctgaag atgatagcga caacaatgaa gtagtcatgg gtgcctgcgt 1140
aacaaaacca tccactttca attgttaatt tctatatatt tcaaacaaat atataaaata 1200
gaaacagaaa tcagattgaa tctggaacct cgtaagagcg aagccaatct ggagatccct 1260
ctctagagct gctgctcata atttcacttc ttcagagctc aaaacaacaa tctaagcttc 1320
ttcccagctt caatatccac tcaattacaa caaaatcagg tcgtacattc aggatttact 1380
tcgcaatatc aaaaccctaa aaagagatct taaaagctat aaattcgatt gaaaatcaca 1440
tgaaatcgaa gctcacatga agaagaagaa gacaaaattg agtgattgac gaatctaatg 1500
aggttaaaac ccggcgagga gacgtggaaa aggcatctga attgactgag gcctgaggga 1560
agaagcaatg atttcgccgg cggagagatt gaagtgtgtg aaagacgcgg gaagaagatt 1620
ggggcataaa ctgcttgttc gagtttccaa gtgtttggtc acaagagcaa tactatcccc 1680
aacgtcgacg taaaatttac taaaagccca atatgtttta atagaaaaat taacataaag 1740
cccaaattcg atgtaatggg ctttcatctt tcaggaaaaa ttaacattag cccagatatg 1800
ccaatagagt agagataatg acgagtgacg acataaaaaa aggcacgcaa agaatataat 1860
attaatttct tggtggacaa gcatgtttga ccattctcat tttcttgtgc ccaaacttat 1920
atttggtatg ctcctcctac atatagacaa gacaatggta attcgacaat tcatcaagtc 1980
tcaaatctaa ataagtaaac 2000

<210> 13
<211> 2000
<212> DNA
<213> Arabidopsis thaliana

<400> 13

agaaacggca gtcccacgag tatctgtata accattcatt gaccagctga atggtacttc 60
ttgcataatc aaaactataa ttcgctggat aatgcaactc acctatttac attgaagaaa 120
ctacagttaa caagtttcca aattgtcttg gagaaacaat gctacgcata caaattggtg 180
aataagaagg cttataagag atatagtgaa acctgaacaa gagctgcacc agccaaagca 240
gacaccactc caatgatatt catggcctgt gaatagtaaa acttcacata tgaagaaaaa 300
caatgctaga agaataataa agcaaatcca taaactaatt ctcttccacg aacatatata 360
ccttaagtag cagaagtaac aaagttggag catacaaaag tacattcatc tttacagaaa 420
cagctccact gtataaaaag aagggtaagc agactagtca atctaaagaa tcgagaagca 480
aaagcagcaa tctaacctga aaacaagcat ccctagatgc catttacgat agagaaagag 540
agccattgat gcatgtagga gagtcatggc aaaacaatca ttaaagagac ggagaacaaa 600
gattgaatgt atcctcttgg ataaacatag tagacttaat gcccaccatg gcacctgaaa 660
tacgtaagga tacaagaaga gatatcatca ctgaattcac taaaatcact cccatcataa 720
acagacaaat caaaaccaaa atattgttca ttaacatcgt aaatggacaa cgcttgacaa 780
cgtctacaaa tacttgatga aaagagataa gaaataagtc ttcatttata ccacatcagt 840
cttcacgtag atgatcaaga cgatccccag attgacaatg tagagaacac caaaaagaat 900
ctgcaaaatg taagagagta gaattgagac aaatgaagcg atggatggat caaattccgc 960
agctaatcca aagataggag attcaaagag agggaattgc aagcacctga gcagggtaga 1020
cttcccctcc agttaagttc tgcactgcag agtaaacata gaggaaacct gctggataaa 1080
ccaatggccc tgtatcgcct ttcaagttcc catagtctct ctctcccccg agaaacccac 1140
taacctgccc caattagata aatttcactt aaagactact agtttacttt gaagaaacca 1200
cagttgacca ttgacactta cctgtgacat gtaagcatcc caatcgatct tcgtgtctgt 1260
taaaggcaaa tgacgaaatt tggtttaaaa cctatgcaaa tgcacatttc gagggatacg 1320
caagcaaaaa aaaaaaggag gtaaatagct ctcacatgga acataggcga taataagtgc 1380
gactaggatt gcatcagcaa gaattagcgc gaatgcgaat ggaactgcag gtttttttgaa 1440
tagatcggat cgattcgtct ccttccccag ccgacggcta cgagaagctc tcaaactcgc 1500
cggtgatgag gcgcccgcca tgaaaacaga gcaaatcgca tcagcgtcta gccaacgccg 1560
cgtaacagac aactacttcc atattactac tcttctaatt agcccaaatt aaatgagcct 1620
attgggcttc ttgtcttagt cggtgtagag cccaattgtt gttttatttt ttaataatgc 1680
aaaagtatta agcgataaat aaataagcat cgcaatcgtc ccaaaactgt gtgtatgcat 1740

cagacatgag catatagagt aagcacgtgt ccacactttt tcacaaagtt atctaaaaac 1800
aaaaaacaat taattagcat tcgacgtgta catatcactc gccacgtgta caagagcctt 1860
ggccttttg cttcttcttc ttgtctatta atatcatctc ctgattatac tctcttttga 1920
ccaagctgct tcttctccat ctatccaaca tctcaaatct cagtaatctt gttccttcga 1980
ttctgttttg gacgtttgta 2000

<210> 14
<211> 2000
<212> DNA
<213> Arabidopsis thaliana

<400> 14

ttcggagaaa atgcagctaa cgatatcagg aaaagtgatt ggtattgtgg gagtgacagt 60
agaagatgaa gaggaagcaa tactggggaa gccaagatcc catcaccgtt gagctctctc 120
tctctctctc tcgtctccgt cgtgtggatc tcgatctcga tctctctttg agccgcaaca 180
actgatgagt tgtctcagtc tctctctctt tctctctcct cttccttttc ctctctctct 240
ttctctctct aaaagggcta agttttttaa aaaaatgtct accaaactga tacaaagagg 300
aagaagaaga aacactctgt acttttttttt ttaacaagag aagaaatgaa aacaaagatg 360
tgaatttaag aaaaaaaata atgtgtcgac attacaaaac tgtatttaat catcatttga 420
aaacatttaa tcatttgata aactatgtaa cattaccccc ttaatcttct ttccacctaa 480
cctttgtatc atttgttggt ctttctattt tataacattc cttaaattat tgaattaaaa 540
ctatctaaat tctgttaatt actcacttgt aatccatttt gttcactgga aatgtaaatt 600
ttactgtctt attttgcaaa acactcttga attttttttt atttttttc tggtcaataa 660
gacacaaaca tgttggatta taaactattg tgatcataac tctgttcggt aatgtttca 720
atatcaaatt tgatagttgg tgattggatt tgtcaacatc tcttttttttt tttgtttaat 780
cctttgctaa gttaagtcgg ccacatatat attgggtacg attttcatag gtctttcctc 840
acgccagaag tgttgtttta ttttgttgat tgagttatta attattggaa gcttttcttt 900
caagcaaagt aaaatgcgta ataatgatta gtcacatcca atggttagtc agtctattac 960

accgttaatc aagctctggt catataattt ttttattttt ggaactaaca cttattagtt 1020
taggtttcca tcacctattt aattcgtaat tcttatacat gcatataata gagatacata 1080
tatacaaatt tatgatcatt tttgcacaac atgtgatctc attcattagt atgcattatg 1140
cgaaaacctc gacgcgcaaa agacacgtaa tagctaataa tgttactcat ttataatgat 1200
tgaagcaaga cgaaaacaac aacatatata tcaaattgta aactagatat ttcttaaaag 1260
tgaaaaaaaa caaagaaata taaaggacaa ttttgagtca gtctcttaat attaaaacat 1320
atatacataa ataagcacaa acgtggttac ctgtcttcat gcaatgtgga ctttagttta 1380
tctaatcaaa atcaaaataa aaggtgtaat agttctcgtc attttcaaa ttttaaaaat 1440
cagaaccaag tgatttttgt ttgagtattg atccattgtt taaacaattt aacacagtat 1500
atacgtctct tgagatgttg acatgatgat aaaatacgag atcgtctctt ggttttcgaa 1560
ttttgaactt taatagtttt ttttttagg gaaactttaa tagttgttta tcataagatt 1620
agtcacctaa tggttacgtt gcagtaccga accaattttt taccctttttt tctaaatgtg 1680
gtcgtggcat aatttccaaa agagatccaa aacccggttt gctcaactga taagccggtc 1740
ggttctggtt tgaaaaacaa gaaataatct gaaagtgtga aacagcaacg tgtctcggtg 1800
tttcatgagc cacctgccac ctcattcacg tcggtcattt tgtcgtttca cggttcacgc 1860
tctagacacg tgctctgtcc ccaccatgac tttcgctgcc gactcgcttc gctttgcaaa 1920
ctcaaacatg tgtgtatatg taagtttcat cctaataagc atctcttacc acattaattt 1980
aaaacaaaga aaacatcaaa 2000

<210> 15
<211> 2000
<212> DNA
<213> Arabidopsis thaliana

<400> 15

atgcccgtta cttgatccca cagggttata attcactgaa ggatttttc ccaaccatga 60
aaatgagacc aaatccacaa tgctcgaatg tagcttgtct agagcggcag gtaacctgat 120
taaatgcaac cacaaaacta tctcagcttg tctttgtttt gcgccttttg tttccatcca 180

tgttctggtg aaaaagttga aatcccaaaa tgcagaaaga atatatgctt gcaaaaccag 240
aaagggatgc tgctgctaaa gctaagatgg aagctgatgc atccacaact atcgatgaag 300
gtccactcca tgatgataat gagtggaaca taaggtaata actcagtgaa ttagttaaaa 360
atcaaaattt gtatttttgt tatcatattt tctcctttcc agccatattt gcaacaagaa 420
atctgatgtg tatctcatgt cttttatca gtgttgtcga tgatgaaaat gagaaggata 480
ctacaaaagc tgcctcaagt tcaggttact taaacttggg actctatgtg ctatccatgt 540
gaaaatgtat caaatataag tcctgttctt ctgactgtta tctaaattga tcctgtgtta 600
tgtgtagata cattacccga agggcttact cgggagcttc cagttgcaga tgagtatgag 660
aaagcaatag ccatagcctc agggtcagga gaaacagagg aggaagatga ccttgaagat 720
ctgaagaaac agctcgaagc tctcaacgct gcttgagtct ttatgaggga gaaaaaatag 780
ttgttttagc atatttatac atgaaagagg ctacaagtgc aattgtgtat gcaaaaagag 840
tgtgttagga ttggtaaatt ggatctcatg gacatataaa cgtttttgat gatgacttct 900
cccgtttttt tagttgttac caaagacaaa aagatacaat tttgaagatg tttcatggat 960
ttattgtcat aatcatccga actagaactt agaaattcaa attagaaaaa gatttgtcc 1020
aatgatgatg atccaagttt ggtttgtaat gtgtaaacca gctttttgg aggtaagata 1080
ttaattgcgc ggggaaatca actagatgac gtgtgaatcg cgaattacaa ttacgttttt 1140
ggatggagaa ctgtcttttt tttccttttt atatccctaa aatactttt tgtacattca 1200
tttatatcag gaaaaacaga gttgtaacca aattattaca gagatattca aagatatttc 1260
aacctatcaa ctaaagatga cgtgtatatt gagaatggca attgcgttta tgagtgggaa 1320
accaaattat ttcattaaca tgatctttag gaataccgct aataagagat tactaatcac 1380
tatttgaggt tttaatttaa atctttgtag tcctgatttt gaatatttcg ttttagctaa 1440
atcagctcca atttttccca gttatcgttt gtcgaattca tattactgtt aatttagtta 1500
acttccgaag actattagta catgattctt caacaaagca aataaagaaa agaatcttga 1560
tgtcgttctc tcaacaattc aacaaaactt ggtctcgacg actcgaccgt gtgagatgtt 1620
tgagaagtca gaacaccaca cgtgtcgaca taaagcccgc accacgcacg cttttcgtct 1680
ggatatatcc aaattacttt acagaaatca tttgtattgt ttgttaataa aaactttgaa 1740
ccactttgat tactatatta tttttttgga atttattctg gtgacattaa tgttctatcc 1800
tccacgcggt tctcataaac cgacttggct tctttatttt tcctttatct ttctctatat 1860
atttacacac acacgcattt gtgcagagat attcaaagat tagaaacatt cttgatagat 1920

acaaaaaaca tttttcagac acaaaatcat aaaatctttg cctttagtag atcaaagttc 1980
tttacattaa tcgttagaag 2000

<210> 16
<211> 2000
<212> DNA
<213> Arabidopsis thaliana

<400> 16

ttagctgggg caggtcggct tgatttcatt gaggatctgc ttgagcatca gaagacactt 60
ccacaaggta ggcgtgaggg tttcattgtt aggattataa tgttgtatgg caaggctgga 120
atgacgaagc aagcacttga tactttcttc aatatggatt tatacggttg caaaagatct 180
gttaagtcct tcaatgccgc tcttcaagtc ttatctttca atcctgatct ccacaccatc 240
tgggagttcc ttcacgacgc cccatcgaag tatggcattg atattgatgc ggtttctttt 300
aacattgcca ttaaatcttt ctgtgagctg ggtattcttg acggggccta tatggcaatg 360
agggagatgg agaaatcagg gttaacacca gatgtagtta catatactac acttatatcg 420
gcgttataca agcacgagag gtgtgtgatt ggaaatggat tgtggaacct tatggtcctc 480
aagggctgta agccgaacct caccactttt aatgttagga ttcagttttt agtgaatagg 540
agacgagctt gggatgcaaa tgatctgttg ctgttgatgc ctaaactcca ggtggaaccg 600
gacagcataa catataacat ggttatcaaa gggtttttcc ttgcgaggtt ccctgacatg 660
gcggaaagag tttatacagc gatgcatggt aaggggtaca aacccaattt gaagatatac 720
cagacgatga ttcactattt gtgcaaggca ggaaattttg atttggctta tacaatgtgc 780
aaggattgta tgagaaagaa gtggtaccca aacttggaca cagttgagat gttgctcaaa 840
gggcttgtga aaaaggggca gcttgatcag gctaagtcga tcatggagtt agttcacaga 900
agagtacctc ctttcaggtc aaaacagttg ctctccctta agtccatttt gtaatgttta 960
catgaaatgg aacaaatttg ctacatgtac acgacaatgc ggccaaaaga attctttctt 1020
tctttctcct gagtttattt tgtatagctg tggtggaact ggaaatggga tgtagcctca 1080
tattaggagg cacacttatc ttccaatcag attcaactat ttgttgttca atgtgaataa 1140

agcttaggat gatttggaaa cttcaatgga gggataccca gtaagtcatg gatgcctcta 1200
caatcttaag ttgtgatagc acagaactag tttacctttt gtaatgctac ttacaaaaca 1260
tgttgttgat ttcagctgga gagcaaaatg gcatacacaa agcagggacc atgtgctgaa 1320
gaaaccaaaa ggatcttcgt ctgacgatgc aaaagctctt gagcacctgg aaaaatgagt 1380
tatgttgtaa gcttcatcta gccaaattaa aagcactaaa catataacaa aggatatcaa 1440
cgttgatagc tttttcttga tatagcacga atgtaaacaa cagagaatag aacagagaca 1500
acattaccag caatgaatat ttttattatt atgtcgaata aatacatttt cccacaagtg 1560
acagagcaaa caaaattcac atatttttgt ctattacact ttggtgtcag actgacttgt 1620
tacgtagatt tatggcggtg gattataaca ttgtaacttt aaatatcttt taacgtaaga 1680
aacaaaactc caaaattggt gtttattttt aaaaataaaa atttgagggg gcatgtatgt 1740
ttgtttagtt gattcacgtg ttctttgtgt ctgaaaattc tgaatttgat gattttgaaa 1800
aaggaaaaaa agatacgagg tatcctcaat tccaaaagga tgaatgatac aactcatata 1860
tacaagatta tagtaaaaag aagggatgca tataaagaaa catagacatc atcgccatcc 1920
atcctcagat cagttccgta gtttactgga tcacgacaca cacacatata cacgtgtaca 1980
tataagccat tcttatcaaa 2000

<210> 17
<211> 2000
<212> DNA
<213> Arabidopsis thaliana

<400> 17

cacatttgac ggcgaatcaa gcgattctag aagcgacgga gaagtcgaac aagattcaca 60
tcgtcgattt tggaatcgtt caaggtatac aatggcctgc tcttcttcaa gctctagcta 120
ctcgtacttc tggtaaaccc actcaaatcc gggtctcggg tatacccgct ccatctctcg 180
gtgaatctcc ggaaccgtcg ttaatcgcca ccggaaaccg cctccgtgat ttcgccaagg 240
ttctggatct gaatttcgat ttcatcccaa ttctcactcc catacattta cttaacgggt 300
caagtttccg ggtcgacccg gatgaagtac tggccgtgaa tttcatgctc cagctctaca 360

aattactcga cgagacgccg acgatagttg acaccgcact acggctcgcc aaatcgttga 420
acccgagggt cgtcactctc ggagaatacg aagtgagctt aaaccgggtc ggtttcgcta 480
accgggtaaa gaacgcgctt caattctatt ccgcggtttt cgaatccctt gaaccgaact 540
tggggcgtga ttcggaggag agagtgagag ttgagcgaga gttgttcggc cggagaatct 600
cgggtttgat tggaccggag aaaaccggaa ttcatagaga aagaatggaa gagaaagagc 660
aatggcgggt attaatggag aatgccggtt ttgaatcggt taagctgagt aattacgcag 720
tgagccaagc gaagattcta ttgtggaatt acaattacag caatttgtat tcaattgttg 780
aatctaagcc tggcttcatc tctttggcct ggaacgattt acctctcctc actctttctt 840
cctggcgata accaaaccaa accgatccgg tattcttagt tttgttttgt tttcaatgtt 900
atttttggtt agacaaatat tcaattgtta atatactccg tggtcagagt gttttgtttt 960
tcttttagtt cgaacgttga attaattcag gggtaggttt tgaattctct gaaccttatg 1020
tgtttttttgg taacatcatt tggatttgtg aactaggttt aaaaactggt cttagtcttg 1080
ttgttttctc attagataat ttaaactggt ttgcttcttt atttttggtt ggataaagtg 1140
accggttctg gtcatctgtt tgagatgtaa ttactatttc ataaaattag gaagttgaaa 1200
gccaaatata tttgtaacta ctcttttatt tgtaattttg ctcaaaaaag tgatgaaatg 1260
tagttttgat atatgaatat ctaccattat acataagtat atctgaacat ggtacaactt 1320
atgaaagcta aatgtcaata cttgcaaaga tataacaaat acaagttaca tgaataagag 1380
atgtgtgttg aatttataag tgtcattttc ttttcacttt aaaacaaact tcatcttctt 1440
ttgtttctta tgtgtcaaag ttgccacagt tgctctattt gagtctttca gtgtcagtct 1500
cagtcactgt actgatttta cttttttttg ttgagtgtgc caatgatgac atcactccca 1560
cgtcctccat ccgtcttctt ttaacggtca cgtggctccc accctctttt ctcgatgtct 1620
ttaccgactt gttctagccc aacttacttg ggccatttag atttttggt ggcccaagtt 1680
gctaaaagag gatttatcat agaaatctga acccgttgca gcgctcaaca catgtcacag 1740
tcctgacaaa cacgtattca aatccttgtt aagtcccgcc acctgtcacc agagcaccac 1800
gaggcaaact ctgatcagga caccgtcgta ctattatgtc ggaagacagg aaagcttaat 1860
taagcttaaa cctgacgtat ttaacttcgt taactctacc ttactaaagg gttttaattt 1920
aaaacttatc atctcctcgt aagaataaaa actacttact ctataaattt aagcttcaag 1980
aaacctccaa aagcagagaa 2000

<210> 18
<211> 2000

<212> DNA
<213> Arabidopsis thaliana

<400> 18

```
ggggttcca caatgcattg attgagtttg tgctgatgca gttacaactt gcttccgtct 60
tttcacgtt tcagctcggg acaaaaacac attactacgg aaggacattg tttcatggag 120
gcgcagagta tagaggaact ggtcgagggt ttgtggtctt ccacgcgaaa tttgctgaga 180
actataggtt ctattcccgt agtcactttg tgaagggcat tgaacttatg attcttctac 240
tggtatatca gatctttggt caatcatata ggggtgtggt tacttatatt ctgatcactg 300
tttcgatatg gttcatggtg gtgacatggc tctttgctcc tttccttttc aatccgtctg 360
gttttgagtg gcaaaagatt gttgatgact ggacagactg gaacaaatgg atttataacc 420
gaggaggaat tggtgtccct cctgagaaga gttgggaatc ttggtgggag aaagagttag 480
aacatcttag acattcaggg gttcgtggta tcacccttga gatcttttg gccttgcgct 540
tcttcatctt tcaatatgga cttgtgtatc atctcagtac cttcaaagga aaaaatcaga 600
gcttttgggt aagctaacct tataactact tccctgtgaa tggtgaaact cttgattatt 660
gaacgataat gactctgttt tcaggtttat ggagcgtcat ggtttgtgat cttattcatt 720
ctactgattg tgaaggtatg aatctttaag cacattcaca tttcaatttc ttgccaaatc 780
ttaacattgt gttgctttct tctatgtaac gatcattgca gggtttgggc gttggaaggc 840
gaagattcag cacgaatttc cagcttctat tcagaatcat aaagggtctt gtgttcctca 900
catttgtggc aattctcata actttcctcg cacttcctct gataacaatc aaagacttat 960
tcatctgcat gcttgccttc atgccaactg gatggggaat gcttctcgta agtctttctc 1020
ctccatcaaa ctcctctatt tgtatctaag ataccagttt tgtctggttt ttgtgacagt 1080
tgctttgttt tgtcctccac gtcagattgc acaagcgtgt aagcctctga tccaacagct 1140
agggatatgg tcatcagtta ggacgctagc tcgaggctac gagatcgtga tgggcttgct 1200
tctgttcaca ccagttgcgt tttggcctg gtttcctttt gtgtctgagt tccagacaag 1260
aatgctcttt aaccaagcct ttagtagagg tctacaaatc tctcgtattc ttggtggtca 1320
```

aagaaaagac cggtcttcaa aaaacaagga gtgacatgta cacatgtaaa tggcgtatgt 1380
gtagatttaa tggttatctg ttattgtttc cttcccaagt aatctatagt tagaaagttc 1440
ctgtaacttt catagatata tgttaattat tttccatttc cataagttcg cgtccattat 1500
aatagaaagt gcatgagacg tatctttttc atatggaaga cgaaaacatt ggttctccat 1560
tctaaaagtc aaaaagaaaa tgtatatcac ttgaactttt ccacataggt catgataaag 1620
acaggactaa tcaatcattc attgttgata atggtctcaa ttacctgttg ctagttattt 1680
ctacatttgt atagtgcaat actgtttttt tttcctcttc caaataaaaa ctacagtact 1740
aaaagaaagt tggtaatcat aaaagttata aaactcaaat aaaagcaatt actctgtttc 1800
tttttcactc ttattttata tgactgaaat gaaattgtct atatgttgtt tttgtttgta 1860
tgttcattga tatgtaaaca tcaaatctgc aaaaaacaac cgcaagtttg gatttatacg 1920
atgaagcgtt tatctagtga gcgtcatcgc agacgtcaca agttatgtgg tacaattttg 1980
cgtatgtttg atgaatacgc 2000

<210> 19
<211> 23
<212> DNA
<213> Artificial Sequence

<220>

<223> Description of Artificial Sequence: synthetic DNA

<400> 19
cgccagggtt ttcccagtca cga 23

<210> 20
<211> 24
<212> DNA
<213> Artificial Sequence

<220>
<223> Description of Artificial Sequence: synthetic DNA

<400> 20
agcggataac aatttcacac agga 24

## Description

The present invention relates to an environmental stress responsive promoter.

By means of gene sequencing projects, large quantities of genomic and cDNA sequences have been determined for a number of organisms, and in a plant model, *Arabidopsis thaliana*, the complete genomic sequences of two chromosomes have been determined (Lin, X. et al., (1999) Nature 402, 761-768.; Mayer, K. et al., (1999) Nature 402, 769-777.)

The EST (expressed sequence tag) project has also contributed greatly to the discovery of expression genes (Hofte, H. et al., (1993) Plant J. 4, 1051-1061.; Newman, T. et al., (1994) Plant Physiol. 106, 1241-1255.; Cooke, R. et al., (1996) Plant J. 9, 101-124. Asamizu, E. et al., (2000) DNA Res. 7, 175-180.) For example, dbEST (the EST database of the National Center for Biotechnology Information (NCBI)) comprises partial cDNA sequences, in which more than a half (about 28,000 genes) of the total gene complement is represented (as estimated from the gene content of the completely sequenced *Arabidopsis thaliana* chromosome 2 (Lin, X. et al., (1999) Nature 402, 761-768.))

In recent years, microarray (DNA chip) technology has become a useful tool for analysis of genome-scale gene expression (Schena, M. et al., (1995) Science 270, 467-470.; Eisen, M. B. and Brown, P. O. (1999) Methods Enzymol. 303, 179-205.) In this DNA chip-based technology, a cDNA sequence is arrayed on a glass slide at a density of more than 1,000 genes/$cm^2$. The thus arrayed cDNA sequence is hybridized simultaneously to a two-color fluorescently labeled cDNA probe pair of different cell or tissue type RNA samples, so as to allow direct and large-scale comparative analysis of gene expression. This technology was first demonstrated by analyzing 48 Arabidopsis genes in respect of differential expression in roots and shoots (Schena, M. et al., (1995) Science 270, 467-470.) Furthermore, microarrays were used to study 1,000 clones randomly selected from a human cDNA library for identification of novel genes responding to heat shock and protein kinase C activation (Schena M. et al., (1996) Proc. Natl. Acad. Sci. USA, 93, 10614-10619.)

In another study, expression profiles of inflammatory disease-related genes were analyzed under various induction conditions by this DNA chip-based method (Heller, R. A. et al., (1997) Proc. Natl. Acad. Sci. USA, 94. 2150-2155.) Moreover, the yeast genome of more than 6,000 coding sequences has also been analyzed in respect of dynamic expression by the use of microarrays (DeRisi, J. L. et al., (1997) Science 278, 680-686.; Wodicka, L. et al., (1997) Nature Biotechnol. 15, 1359-1367.)

In plant science, however, only a few reports regarding microarray analysis have been published (Schena, M. et al., (1995) Science 270, 467-470.; Ruan, Y. et al., (1998) Plant J. 15, 821-833.; Aharoni. A. et al., (2000) Plant Cell 12, 647-661.; Reymond, P. et al., (2000) Plant Cell 12, 707-719.)

Plant growth is greatly affected by environmental stresses such as drought, high salinity and low temperature. Among these stresses, drought or water deficiency is the most severe limiting factor for plant growth and crop production. Drought stress induces various biochemical and physiological responses in plants.

Plants acquire responsivity and adaptability to these stresses to survive under stress conditions. Recently, a number of genes responding to drought at a transcriptional level have been described (Bohnert, H.J. et al., (1995) Plant Cell 7, 1099-1111.; Ingram, J., and Bartels, D. (1996) Plant Mol. Biol. 47, 377-403.; Bray, E. A. (1997) Trends Plant Sci. 2, 48-54.; Shinozaki. K., and Yamaguchi-Shinozaki, K. (1997) Plant Physiol. 115, 327-334.; Shinozaki, K., and Yamaguchi-Shinozaki, K. (1999). Molecular responses to drought stress. Molecular responses to cold, drought, heat and salt stress in higher plants. Edited by Shinozaki, K. and Yamaguchi-Shinozaki, K. R. G. Landes Company.; Shinozaki, K., and Yamaguchi-Shinozaki, K. (2000) Curr. Opin. Plant Biol. 3, 217-223.)

Stress-inducible genes have been used to improve stress tolerance of plants by gene transfer (Holmberg, N., and Bulow, L. (1998) Trends Plant Sci. 3, 61-66.; Bajaj. S. et al., (1999) Mol. Breed. 5, 493-503.) It is important to analyze the functions of stress-inducible genes not only to understand the molecular mechanisms of stress tolerance and responses of higher plants, but also to improve the stress tolerance of crops by gene manipulation.

DRE/CRT (dehydration-responsive element/C-repeat sequence) has been identified as an important *cis*-acting element in drought-, high salt-, and cold stress-responsive gene expression in an ABA-independent manner (ABA refers to abscisic acid which is a kind of plant hormone and which acts as a signal transmission factor of seed dormancy and environmental stress) (Yamaguchi-Shinozaki, K., and Shinozaki, K. (1994) Plant Cell 6, 251-264.; Thomashow, M.F. et al., (1999) Plant Mol. Biol. 50, 571-599.; Shinozaki, K., and Yamaguchi-Shinozaki, K. (2000) Curr. Opin. Plant Biol. 3, 217-223.) Transcription factors (DREB/CBF) involved in DRE/CRT-responsive gene expression have been cloned (Stockinger. E.J. et al., (1997) Proc. Natl. Acad. Sci. USA 94, 1035-1040.; Liu, Q. et al., (1998) Plant Cell 10, 1391-1406.; Shinwari, Z.K. et al., (1998) Biochem. Biophys. Res. Commun. 250, 161-170.; Gilmour, S.J.et al., (1998) Plant J. 16, 433-443.) DREB1/CBFs are considered to function in cold-responsive gene expression, whereas DREB2s are involved in drought-responsive gene expression. Strong tolerance to freezing stress was observed in transgenic Arabidopsis plants that overexpress *CBF1* (*DREB1B*) cDNA under the control of a cauliflower mosaic virus (CaMV) 35S promoter (Jaglo-Ottosen, K.R. et al., (1998) Science 280, 104-106.)

The present inventors have reported that overexpression of the *DREB1A* (*CBF3*) cDNA molecules in transgenic plants under the control of a CaMV 35S promoter or a stress-inducible rd29A promoter gave rise to strong constitutive expression of the stress-inducible DREB1A target genes and increased tolerance to freezing, drought and salt stresses (Liu, Q. et al., (1998) Plant Cell 10, 1391-1406.; Kasuga, M. et al., (1999) Nature Biotechnol. 17, 287-291.) Furthermore, the present inventors have already identified six DREB1A target genes such as *rd29A*/*lti78*/*cor78, kin1, kin2*/*cor6.6, cor15a, rd17*/*cor47* and *erd10* (Kasuga, M. et al., (1999) Nature Biotechnol. 17, 287-291.) However, it is not well clarified how overexpression of the *DREB1A* cDNA molecules in transgenic plants increases stress tolerance to freezing, drought and salt. To study the molecular mechanisms of drought and freezing tolerance, it is important to identify and analyze as many genes controlled by DREB1A as possible.

The present invention is directed to providing an environmental stress responsive promoter.

Through intensive studies directed toward the above object, the present inventors have succeeded in identifying a novel DREB1A target gene and isolating a promoter region thereof by applying cDNA microarray analysis, thereby completing the present invention.

That is to say, the present invention is an environmental stress responsive promoter comprising DNA of the following (a) or (b) :

(a) DNA consisting of a nucleotide sequence identified as SEQ ID NO: 1 ;
(b) DNA consisting of a nucleotide sequence comprising a deletion, substitution or addition of one to ten nucleotides relative to the nucleotide sequence identified as SEQ ID NO: 1, and functioning as an environmental stress responsive promoter.

The environmental stress is at least one selected from the group consisting of cold stress, drought stress, and salt stress

Moreover, the present invention is an expression vector comprising the above promoter, or the expression vector further comprising a desired gene.

Furthermore, the present invention is a transformant comprising the above expression vector.

Still further, the present invention is a transgenic plant (e.g. a plant body, plant organ, plant tissue or plant culture cell) comprising the above expression vector.

Figure 1 is a photograph showing results of cDNA microarray analysis of gene expression under cold stress.

Figure 2 is a figure showing strategy for the identification of drought- or cold-inducible genes and DREB1A target genes.

Figure 3 is a photograph showing a comparison of cDNA microarray and Northern Blot analysis for new DREB1A target genes and a DREB1A gene.

Figure 4 is a figure showing results of classification of the identified drought-or cold-inducible genes into four groups on the basis of RNA gel blot and microarray analyses.

Figure 5 shows the relation between cold treatment period and expression rate regarding FL3-5A3.

Figure 6 shows the relation between dehydration treatment period and expression rate regarding FL3-5A3.

Figure 7 shows the relation between high salt treatment period and expression rate regarding FL3-5A3.

Figure 8 shows the relation between cold treatment period and expression rate regarding FL5-2H15.

Figure 9 shows the relation between dehydration treatment period and expression rate regarding FL5-2H15.

Figure 10 shows the relation between high salt treatment period and expression rate regarding FL5-2H15.

Figure 11 shows the relation between dehydration treatment period and expression rate regarding FL5-3M24.

Figure 12 shows the relation between high salt treatment period and expression rate regarding FL5-3M24.

Figure 13 shows the relation between cold treatment period and expression rate regarding FL5-90.

Figure 14 shows the relation between cold treatment period and expression rate regarding FL5-2I22.

Figure 15 shows the relation between dehydration treatment period and expression rate regarding FL5-2I22.

Figure 16 shows the relation between high salt treatment period and expression rate regarding FL5-2I22.

Figure 17 shows the relation between dehydration treatment period and expression rate regarding FL6-55.

Figure 18 shows the relation between high salt treatment period and expression rate regarding FL6-55.

Figure 19 shows the relation between dehydration treatment period and expression rate regarding FL1-159.

Figure 20 shows the relation between dehydration treatment period and expression rate regarding FL5-2D23.

Figure 21 shows the relation between high salt treatment period and expression rate regarding FL5-2D23.

Figure 22 shows the relation between dehydration treatment period and expression rate regarding FL05-08-P24.

Figure 23 shows the relation between dehydration treatment period and expression rate regarding FL05-09-G08.

Figure 24 shows the relation between dehydration treatment period and expression rate regarding FL05-09-P10.

Figure 25 shows the relation between ABA treatment period and expression rate regarding FL05-09-P 10.

Figure 26 shows the relation between high salt treatment period and expression rate regarding FL05-10-N02.

Figure 27 shows the relation between dehydration treatment period and expression rate regarding FL05-18-I12.

Figure 28 shows the relation between high salt treatment period and expression rate regarding FL05-18-I12.

Figure 29 shows the relation between ABA treatment period and expression rate regarding FL05-18-I12.

Figure 30 shows the relation between dehydration treatment period and expression rate regarding FL05-21-F13.

Figure 31 shows the relation between cold treatment period and expression rate regarding FL05-21-F13.

Figure 32 shows the relation between dehydration treatment period and expression rate regarding FL06-10-C16.

Figure 33 shows the relation between high salt treatment period and expression rate regarding FL06-10-C16.

Figure 34 shows the relation between ABA treatment period and expression rate regarding FL06-10-C16.

Figure 35 shows the relation between dehydration treatment period and expression rate regarding FL06-15-P15.

Figure 36 shows the relation between high salt treatment period and expression rate regarding FL06-15-P15.

Figure 37 shows the relation between ABA treatment period and expression rate regarding FL06-15-P15.

Figure 38 shows the relation between dehydration treatment period and expression rate regarding FL08-10-E21.

Figure 39 shows the relation between high salt treatment period and expression rate regarding FL09-11-P10.

Hereinafter, the present invention is described in detail.

Applying the biotinylated CAP trapper method (Carninci. P. et al., (1996) Genomics, 37, 327-336.), the present inventors constructed full-length cDNA libraries from Arabidopsis plants under different conditions, such as drought-treated and cold-treated plants, etc. (Seki. M. et al., (1998) Plant J. 15, 707-720.) Using about 1,300 full-length cDNA molecules and about 7,000 full-length cDNA molecules which both contained stress-inducible genes, the present inventors prepared an Arabidopsis full-length cDNA microarray for each case. In addition to these drought- and cold-inducible full-length cDNA molecules, the present inventors also prepared another cDNA microarray, using a DREB1A target gene, a transcriptional regulator for controlling expression of a stress-responsive gene. Thereafter, expression patterns of genes under drought- and cold-stresses were monitored to exhaustively analyze stress-responsive genes. As a result, novel environmental stress responsive genes, that is, 44 drought-inducible genes and 19 cold-inducible genes were isolated from a full-length cDNA microarray containing about 1,300 full-length cDNA molecules. 30 out of the 44 drought-inducible genes and 10 out of the 19 cold-inducible genes, were novel stress-inducible genes. Moreover, it was found that 12 stress-inducible genes were DREB1A target genes, and 6 of these 12 were novel genes. Furthermore, as a result of this analysis, 301 drought-inducible genes, 54 cold-inducible genes and 211 high salt-inducible genes were isolated from a cDNA microarray containing about 7,000 full-length cDNA molecules.

Thereafter, promoter regions were successfully isolated from these environmental stress responsive genes.

As stated above, a full-length cDNA microarray is a useful tool for analysis of the expression manner of *Arabidopsis thaliana* drought- and cold-stress inducible genes, and analysis of the target gene of a stress-related transcriptional regulator.

1. Isolation of promoter

The promoter of the present invention is a cis-element existing upstream of a gene encoding a stress-responsive protein expressed by environmental stresses such as cold-, drought- and high salt-stresses, and the *cis*-element has a function of binding to a transcriptional factor to activate transcription of a gene existing downstream. Examples of such cis-elements include a drought stress responsive element (DRE; dehydration-responsive element), an abscisic acid responsive element (ABRE), and a cold stress responsive element, etc. Examples of genes encoding proteins binding to these elements include a DRE binding protein 1A gene (referred to also as a DREB1A gene), a DRE binding protein 1C gene (referred to also as a DREB1C gene), a DRE binding protein 2A gene (referred to also as a DREB2A gene) and a DRE binding protein 2B gene (referred to also as a DREB2B gene), etc.

For isolation of the promoter of the present invention, first, stress responsive genes are isolated using a microarray. For preparation of a microarray, there can be used about 1,300 cDNA molecules in total, being genes isolated from Arabidopsis full-length cDNA libraries, RD (responsive to dehydration) genes, ERD (early responsive to dehydration) genes, *kin1* genes, *kin2* genes, *cor15a* genes, α-tubulin genes as an internal standard, and as negative controls, epsilon subunit (nAChRE) genes of a mouse acetylcholine nicotinate receptor and homologous genes of a mouse glucocorticoid receptor.

As a microarray used to isolate the promoter of the present invention, there can be used about 7,000 cDNA molecules in total, being genes isolated from Arabidopsis full-length cDNA libraries, RD (responsive to dehydration) genes, ERD (early responsive to dehydration) genes, and PCR amplification fragments as an internal standard obtained from λ control template DNA fragments (TX803, Takara Shuzo), and as negative controls, epsilon subunit (nAChRE) genes of a mouse acetylcholine nicotinate receptor and homologous genes of a mouse glucocorticoid receptor.

A plasmid DNA extracted with a plasmid preparation device (Kurabo) is sequenced by sequence analysis, using a DNA sequencer (ABI PRISM 3700, PE Applied Biosystems, CA, USA). Based on the GenBank/EMBL database, homology detection of the obtained sequence is carried out with the BLAST program.

After poly A selection, reverse transcription is carried out to synthesize double-stranded DNA molecules, and a cDNA molecule is inserted into a vector.

The cDNA molecule inserted into a vector for preparation of cDNA libraries is amplified by PCR, using primers complementary to sequences of vectors on both sides of the cDNA molecule. Examples of such vectors include λ ZAPII and λ PS, etc.

A microarray can be prepared according to ordinary methods and so the method is not particularly limited. For example, using a gene tip microarray stamp machine, GTMASS SYSTEM (Nippon Laser & Electronics Lab.), the above obtained PCR product is loaded from a microtiter plate and spotted on a micro slide glass at regular intervals. Then, to prevent expression of non-specific signals, the slide is immersed into a blocking solution.

Examples of plant materials include plant strains obtained by destroying specific genes as well as wild type plants, and there can be used a transgenic plant, into which cDNA of DREB1A is introduced. Examples of plant varieties include *Arabidopsis thaliana,* tobacco and rice, etc., and *Arabidopsis thaliana* is preferable.

Drought- and cold-stress treatments can be carried out according to a known method (Yamaguchi-Shinozaki, K., and Shinozaki, K. (1994) Plant Cell 6, 251-264.)

After performing the stress treatments, plant bodies (wild type plants and DREB1A overexpression transformants) are sampled, and are subjected to cryopreservation with liquid nitrogen. The wild type plants and the DREB1A overexpression transformants are used for an experiment to identify DREB1A target genes. According to a known method or using a kit, mRNA is isolated from plant bodies and purified.

In the presence of Cy3 dUTP or Cy5 dUTP for labeling (Amersham Pharmacia), each of the mRNA samples is subjected to reverse transcription and then used for hybridization.

After hybridization, the microarray is scanned with a scanning laser microscope. As a program for analyzing data of a microarray, Imagene Ver 2.0 (BioDiscovery) and QuantArray (GSI Lumonics), etc. can be used.

After scanning, genes of interest are isolated by preparation of a plasmid comprising the gene.

Determination of a promoter region is carried out by analysis of the nucleotide sequences of the above isolated genes, followed by the use of a gene analysis program based on the genomic information of database (GenBank/EMBL, ABRC). The isolated genes can be classified into ones having both drought- and cold-stress inductivity, ones specific for drought stress inductivity, and ones specific for cold stress inductivity (Figure 4). According to a gene analysis program, 18 types of genes (FL3-5A3, FL5-2H15, FL5-3M24, FL5-90, FL5-2I22, FL6-55, FL1-159, FL5-2D23, FL05-08P-24, FL05-09-G08, FL05-09-P10, FL05-10-N02, FL05-18-I12, FL05-21-F13, FL06-10-C16, FL06-15-P15, FL08-10-E21 and FL09-11-P10) are identified from the above genes. The promoter regions of these genes are shown in SEQ ID NOS: 1 to 18, respectively.

As long as the promoter of the present invention acts as an environmental stress responsive promoter, it may be a promoter having a nucleotide sequence comprising a deletion, substitution or addition of one or more nucleotides, preferably one or several nucleotides (i.e. 1 to 10 nucleotides, preferably 1 to 5 nucleotides) relative to a nucleotide sequence identified as SEQ ID NO: 1. Furthermore, the promoter also includes DNA hybridizing under stringent conditions to the DNA comprising any nucleotide sequence selected from SEQ ID NOS: 1 to 18 and further acting as an environmental stress responsive promoter.

Once the nucleotide sequence of the promoter of the present invention is determined, then the promoter itself can be obtained by chemical synthesis, PCR using a cloned probe as a template, or hybridization, using as a probe, DNA fragments having the nucleotide sequence. Furthermore, a mutant of the present promoter, which has functions equivalent to those of a non-mutated promoter, can also be synthesized by a site-directed mutagenesis, etc.

To introduce a mutation into a promoter sequence, the known methods such as the Kunkel method and Gapped duplex method, or an equivalent method, can be applied. Introduction of a mutation can be carried out, for example, using a kit for introducing mutant (e.g. Mutant-K (Takara) and Mutant-G (Takara)) by a site-directed mutagenesis or using the LA PCR in vitro Mutagenesis series kit (Takara).

The term "function as an environmental stress responsive promoter" is used herein to mean a function of binding RNA polymerase to a promoter to allow initiation of transcription, when the promoter is exposed to a specific environmental stress condition.

The term "environmental stress" is used generally to mean an abiotic stress such as drought stress, cold stress, high salt stress, etc. The term "drought" is used herein to mean a water deficient state, while the term "cold" is used herein to mean a state of being exposed to a lower temperature than the optimum living temperature of each organism variety (e.g., in the case of *Arabidopsis thaliana*, it is continuously exposed at -20 to +21°C for 1 hour to several weeks). The term "high salt" is used herein to mean a state after continuous treatment with NaCl having a concentration of 50mM to 600mM for 0.5 hours to several weeks. The term "high photo stress" is used herein to mean a state wherein a strong light greater than its photosynthetic ability is applied to a plant, and an example is application of a strong light of more than 5,000 to 10,000 lx. With regard to these environmental stresses, one kind of stress may be loaded, or several kinds of stresses may be loaded.

The plant promoter of the present invention includes a promoter comprising an addition of a nucleotide sequence which increases translation efficiency at the 3'-terminus of a nucleotide sequence of SEQ ID NO: 1 , or a promoter retaining a promoter activity thereof while deleting a 5'-terminus thereof.

Furthermore, the promoter includes DNA which hybridizes under stringent conditions to DNA consisting of any nucleotide sequence selected from SEQ ID NOS: 1 to 18, and functions as an environmental stress responsive promoter. The term "stringent conditions" used herein means sodium concentration of 25 to 500mM, preferably 25 to 300mM, and temperature of 42°C to 68°C, preferably 42°C to 65°C. More specifically, such conditions are 5 X SSC (83mM NaCl, 83mM sodium citrate) and temperature of 42°C.

2. Construction of expression vector

The expression vector of the present invention can be obtained by ligation (insertion) of the promoter of the present invention to an appropriate vector. As long as a vector into which the promoter of the present invention is inserted is capable of replicating in a host, it is not particularly limited, and examples of vectors include a plasmid, a shattle vector and a helper plasmid, etc.

Examples of plasmid DNA include a plasmid derived from *Escherichia coli* (e.g. pBR322, pBR325, pUC118, pUC119, pUC18, pUC19 and pBluescript, etc.), a plasmid derived from *Bacillus subtilis* (e.g. pUB110 and pTP5, etc.), and a plasmid derived from yeast (e.g. YEp13 and YCp50, etc.), and examples of phage DNA include λ phage (e.g. Charon4A, Charon21A, EMBL3, EMBL4, λ gt10, λ gt11 and λ ZAP, etc.) Further, animal virus vectors such as a retrovirus and a vaccinia virus, and insect virus vectors such as a baculovirus can also be used.

To insert the promoter of the present invention into a vector, there is applied a method in which, first, the purified DNA is cleaved with suitable restriction enzymes, and, next, the obtained DNA fragment is inserted into the restriction enzyme site of a suitable vector DNA or a multi-cloning site so as to ligate to the vector.

In the present invention, in order to express a desired gene, the desired gene can be further inserted into the above expression vector. The technique involving insertion of a desired gene is the same as the method involving insertion of a promoter into a vector. A desired gene is not particularly limited, and examples of the gene include genes shown in Table 2 and the known genes other than those, etc.

In a case where the promoter of the present invention is used with a reporter gene, e.g. a GUS gene widely used in plant science, ligated to a 3'-terminus thereof, the strength of the promoter can easily be determined by examining a GUS activity. As a reporter gene, not only a GUS gene but also luciferase and a green fluorescent protein can be used.

Thus, various types of vectors can be used in the present invention. Further, there can be prepared a product by connecting a desired gene of interest to the promoter of the present invention in a sense or antisense direction, and thereafter such product can be inserted into a vector called a binary vector, such as pBI101 (Clonetech).

3. Preparation of transformant

The transformant of the present invention can be obtained by introduction of the expression vector of the present invention into a host. A host herein is not particularly limited, as long as it can express a promoter or gene of interest, a plant being preferable. Where a host is a plant, a transformant plant (a transgenic plant) can be obtained as follows.

A plant to be transformed in the present invention means any of an entire plant, a plant organ (e.g. a leaf, a petal, a stem, a root, a seed, etc.), a plant tissue (e.g., an epidermis, a phloem, a parenchyma, a xylem, a vascular bundle, etc.) and a plant culture cell. Examples of plants used for transformation include plants belonging to Brassicaceae, Gramineae, Solanaceae and Leguminosae, etc. (see below), but are not limited thereto.

Brassicaceae: *Arabidopsis thaliana*
Gramineae: *Nicotiana tabacum*
Solanaceae: *Zea mays, Oryza sativa*
Leguminosae: *Glycine max*

The above recombinant vector can be introduced into a plant by ordinary transformation methods such as electroporation, Agrobacterium method, particle gun method, PEG, etc.

For example, where electroporation is applied, using an electroporation device equipped with a pulse controller, a vector is processed under conditions of a voltage of 500 to 1,600V, 25 to 1,000μF and 20 to 30msec, and a gene is introduced into a host.

Where a particle gun method is applied, a plant body, plant organ or plant tissue may be used as is, after preparation of a section, or a protoplast may be prepared. The thus prepared sample can be processed with a gene-introduction device (e.g. PDS-1000/He, Bio-Rad, etc.) Conditions for processing depend on a plant or sample, but generally, a pressure of about 1,000 to 1,800psi and a distance of 5 to 6cm are applied as processing conditions.

A gene of interest can be introduced into a plant by using a plant virus as a vector. Examples of available plant viruses include a cauliflower mosaic virus. That is, first, a virus genome is inserted into a vector derived from *Escherichia coli* to prepare a recombinant, and then the gene of interest is inserted into the virus genome. The thus modified virus genome is cut from the recombinant with restriction enzymes, and inoculated into a plant host, so that a gene of interest can be introduced therein.

When bacteria belonging to *Agrobacterium* are transfected to a plant, the bacteria introduce a portion of plasmid DNA thereof into a plant genome. In a method involving the use of Ti plasmid of *Agrobacterium*, using such a character, a gene of interest is introduced into a plant host. Among bacteria belonging to *Agrobacterium, Agrobacterium tumefaciens* transfects to a plant and forms therein a tumor called a crown gall, whereas *Agrobacterium rhizogenes* transfects to a plant to generate hairy roots. These phenomena originate from a cause whereby a region called a T-DNA region (a transferred DNA region) located on a plasmid called a Ti plasmid or Ri plasmid existing in each bacterium is transferred into a plant and incorporated into a plant genome at a time of transfection.

By inserting DNA to be incorporated into a plant genome, into the T-DNA region of a Ti or Ri plasmid, DNA of interest can be incorporated into a plant genome, when *Agrobacterium* bacteria are transfected to a plant host.

Tumoral tissues, shoots and hairy roots obtained as a result of transformation can directly be used for cell culture, tissue culture or organ culture, and according to the previously known plant tissue culture method, a plant body can be regenerated by administration of a plant hormone (e.g. auxin, cytokinin, gibberellin, abscisic acid, ethylene, brassinoride, etc.) in a suitable concentration.

The vector of the present invention cannot only be introduced into the above-stated plant hosts, but can also be introduced into bacteria belonging to *Escherichia* such as *Escherichia coli, Bacillus* such as *Bacillus subtilis* and *Pseudomonas* such as *Pseudomonas putida*; yeast such as *Saccharomyces cerevisiae* and *Schizosaccharomyces pombe*; animal cells such as COS cell and CHO cell; and insect cells such as Sf9 cell, so that a transformant can be obtained. Where a bacterium such as *Escherichia coli* or yeast is used as a host, it is preferable that the recombinant vector of the present invention is capable of self-replicating in the bacterium and, at the same time, is also comprised of the promoter of the present invention, a ribosome binding sequence, a gene of interest and a transcription termination sequence. Furthermore, it may also comprise a gene for controlling a promoter.

A method for introduction of a recombinant vector into bacteria is not particularly limited, as long as it is a method for introduction of DNA into bacteria. For example, a method involving the use of calcium ions and an electroporation method can be applied.

Where yeast is used as a host, *Saccharomyces cerevisiae* and *Schizosaccharomyces pombe*, etc. can be used. A method for introduction of a recombinant vector into yeast is not particularly limited, as long as it is a method for introduction of DNA into yeast, and examples of such methods include electroporation, spheroplast method, lithium phosphate method, etc.

Where an animal cell is used as a host, a monkey COS-7 cell, Vero, a Chinese hamster ovary cell (a CHO cell), a mouse L cell, etc. are used. Examples of methods for introduction of a recombinant vector into an animal cell include electroporation, calcium phosphate method, lipofection method, etc.

Where an insect cell is a host, a Sf9 cell and the like can be used. Examples of methods for introduction of a recombinant vector into an insect cell include calcium phosphate method, lipofection method, electroporation, etc.

Confirmation regarding whether a gene is incorporated into a host or not can be carried out by methods such as PCR, Southern hybridization, Northern hybridization, etc. For example, DNA is prepared from a transformant, and DNA specific primers are designed for use with PCR. PCR is carried out under the same conditions as used for preparation of the above plasmid. Thereafter, the obtained amplified product is subjected to agarose gel electrophoresis, polyacrylamide gel electrophoresis or capillary electrophoresis, so that the product is stained with ethidium bromide or a SYBR Green solution, etc. Then, it is confirmed that the amplified product was transformed, by detecting the product as a single band. Or, the amplified product can also be detected by PCR, using primers stained with fluorescent dye or the like beforehand. Furthermore, there may also be adopted a method in which the amplified product is bound to a solid phase such as a microplate and confirmed by fluorescent or enzymic reaction, etc.

4. Production of plant

In the present invention, a transformed plant body can be regenerated from the above transformed plant cell and the like. An adaptable regeneration method is one in which transformed cells are transferred to and cultured in media with different types of hormones and concentrations to promote nucellular embryony, thereby obtaining an entire plant body. Examples of applicable media include an LS medium and an MS medium, etc.

The "method for producing a plant body" of the present invention comprises processes of, introducing a plant expression vector, into which the above plant promoter is inserted, into a host cell to obtain a transformed plant cell; regenerating a transformed plant body from the transformed plant cell; obtaining plant seeds from the resulting transformed plant body; and producing a plant body from the plant seed.

To obtain a plant seed from a transformed plant body, for example, a transformed plant body is collected from a rooting medium and transferred to a pot with water-containing soil. Then, the transformed plant body is grown at constant temperature to form flowers, thereby finally obtaining seeds. To produce a plant body from a seed, for example, after a seed formed in a transformed plant body has matured, the seed is isolated and implanted in water-containing soil, followed by growing at constant temperature under illumination. The thus bred plant becomes an environmental stress-resistant plant corresponding to the stress responsivity of a promoter introduced.

EXAMPLES

The present invention is further described in the following examples. The examples are not intended to limit the scope of the invention.

<u>Example 1. Isolation of promoter</u>

1. Materials and methods

(1) Arabidopsis cDNA clone

For preparation of a microarray, there were used about 1,300 cDNA molecules in total, which are genes isolated from Arabidopsis full-length cDNA libraries, RD (responsive to dehydration) genes, ERD (early responsive to dehydration) genes, *kin1* genes, *kin2* genes, *cor15a* genes, and $\alpha$-tubulin genes as an internal standard, and as negative controls, epsilon subunit (nAChRE) genes of a mouse acetylcholine nicotinate receptor and homologous genes of a mouse glucocorticoid receptor.
Positive control: drought-inducible genes (responsive-to-dehydration genes: rd, and early responsive-to-dehydration genes: erd)
Internal standard: $\alpha$-tubulin genes
Negative control: for analysis of non-specific hybridization, acetylcholine nicotinate receptor $\varepsilon$ subunit (nAChRE) genes

and mouse glucocorticoid receptor homolog genes, which do not substantially have homology with any given sequence in Arabidopsis database.

Furthermore, for preparation of a microarray, there were used about 7,000 cDNA molecules in total, which are genes isolated from Arabidopsis full-length cDNA libraries, RD (responsive to dehydration) genes, ERD (early responsive to dehydration) genes, and PCR amplification fragments (hereinafter referred to as PCR fragments) as an internal standard obtained from λ control template DNA fragments (TX803, Takara Shuzo), and as negative controls, epsilon subunit (nAChRE) genes of a mouse acetylcholine nicotinate receptor and homologous genes of a mouse glucocorticoid receptor.

Positive control: drought-inducible genes (responsive-to-dehydration genes: rd, and early responsive-to-dehydration genes: erd)

Internal standard: PCR fragments

Negative control: for analysis of non-specific hybridization, acetylcholine nicotinate receptor ε subunit (nAChRE) genes and mouse glucocorticoid receptor homolog genes, which do not substantially have homology with any given sequence in the Arabidopsis database.

(2) Arabidopsis full-length cDNA microarray

According to the biotinylated CAP trapper method, the present inventor has constructed a full-length cDNA library from Arabidopsis plant bodies under different conditions (e.g. dehydration treatment, cold treatment and untreatment in various growth stages from budding to mature seeds). The present inventor has independently isolated each of about 1,300 and about 7,000 Arabidopsis full-length cDNA molecules from a full-length cDNA library. According to the known method (Eisen and Brown, 1999), cDNA fragments amplified by PCR were arranged on a slide glass. The present inventor has prepared both a full-length cDNA microarray containing about 1,300 Arabidopsis full-length cDNA molecules and another full-length cDNA microarray containing about 7,000 Arabidopsis full-length cDNA molecules, which comprise the genes stated below.

(3) Isolation of drought-, cold- and high salt-inducible genes using cDNA microarray

In this example, using a full-length cDNA microarray containing about 1,300 Arabidopsis full-length cDNA molecules, drought- and cold-inducible genes were isolated. Further, using a full-length cDNA microarray containing about 7,000 Arabidopsis full-length cDNA molecules, drought-, cold- and high salt-inducible genes were isolated.

Both Cy3 and Cy5 fluorescent labeled probes of drought treated, cold treated and untreated plants were mixed, and the obtained mixture was hybridized to a full-length cDNA microarray containing about 1,300 Arabidopsis full-length cDNA molecules. Figure 1 shows an image of the cDNA microarray. By the double labeling of a pair of cDNA probes, wherein one mRNA sample is labeled with Cy3-dUTP and the other mRNA sample is labeled with Cy5-dUTP, simultaneous hybridization to DNA elements on a microarray becomes possible and direct assay of gene expression level under two different conditions (that is, stressed and unstressed) is facilitated. The Cy3 and Cy5 emissions of each DNA element on the hybridized microarray was scanned using two different laser channels. Thereafter, the intensity rate of the two fluorescent signals of each DNA element was determined as a relative value, and then the change of differential expression of genes was determined, which was shown as a cDNA spot on a microarray. In this example, there was used, as an internal control gene, an α-tubulin gene, the expression level of which remains almost constant under two different experimental conditions.

In the case of a full-length cDNA microarray containing about 7,000 Arabidopsis full-length cDNA molecules, Cy3 and Cy5 fluorescent labeled probes of each of a dehydration treated plant, a cold treated plant, a high salt -inducible gene and an unstressed plant were mixed and the obtained mixture hybridized. A PCR fragment was used as an internal control gene in this cDNA microarray.

Figure 2 shows the identification process of drought- or cold-inducible genes in a full-length cDNA microarray containing about 1,300 Arabidopsis full-length cDNA molecules. Furthermore, in the case of a full-length cDNA microarray containing about 7,000 Arabidopsis full-length cDNA molecules also, the identification of drought-, cold- or high salt-inducible genes was performed according to the same process as shown in Figure 2.

1) Both mRNA molecules derived from a dehydration- or cold-treated plant and mRNA molecules derived from an unstressed wild type plant were used to prepare both Cy3-and Cy5-labeled cDNA probes. These cDNA probes were mixed, and then hybridized to a cDNA microarray. In this example, there was used, as an internal control gene, an α-tubulin gene, the expression level of which remains almost constant under two different experimental conditions. A gene having more than double the expression level (drought/unstressed or cold/unstressed) of an α-tubulin gene was defined as a drought- or cold-inducible gene (Figure 2).

2) Both mRNA molecules derived from a 35S:DREB1A transgenic plant and mRNA molecules derived from an unstressed wild type plant were used to prepare both Cy3-and Cy5-labeled cDNA probes. These cDNA probes were mixed, and then hybridized to a cDNA microarray. In this example, there was used, as an internal control gene, an α-tubulin gene, the expression level of which remains almost constant under two different experimental conditions. A gene having an expression level in a 35S:DREB1A transgenic plant of more than double its expression level in an unstressed wild type plant was defined as a DREB1A target gene (Figure 2).

Both mRNA molecules derived from a dehydration- or cold-treated plant and mRNA molecules derived from an unstressed wild type plant were used to prepare both Cy3- and Cy5-labeled cDNA probes. These cDNA probes were mixed, and then hybridized to a cDNA microarray. To evaluate reproducibility of microarray analysis, the same experiment was repeated five times. When the same mRNA sample was hybridized to various types of microarrays, a good correlation was observed. A gene having more than double the expression level (drought/unstressed or cold/unstressed) of an α-tubulin gene was defined as a drought- or cold-inducible gene (Figure 2).

(4) Analysis of sequence

To perform homology detection of gene sequences, a plasmid DNA extracted with a plasmid preparation device (NA 100, Kurabo) was used for sequence analysis. A DNA sequence was determined by dye terminator cycle sequencing method, using a DNA sequencer (ABI PRISM 3700, PE Applied Biosystems, CA, USA). Based on the GenBank/EMBL database, homology detection of sequences was carried out using the BLAST program.

(5) Amplification of cDNA

As a vector for preparation of cDNA libraries, λZAPII (Carninci et al., 1996) was used. The cDNA inserted into a vector for libraries was amplified by PCR, using primers complementary to vector sequences located on both sides of the cDNA.

The sequences of primers are as follows:

FL forward 1224: 5'-CGCCAGGGTTTTCCCAGTCACGA (SEQ ID NO: 19)
FL reverse 1233: 5'-AGCGGATAACAATTTCACACAGGA (SEQ ID NO: 20)

As a template, a plasmid (1 to 2ng) was added to 100μl of PCR mixture (0.25mM dNTP, 0.2μM PCR primers, 1 X Ex Taq buffer, 1.25 U Ex Taq polymerase (Takara Shuzo)). PCR was performed under the following conditions: initial reaction at 94°C for 3 minutes, 35 cycles of 95°C for 1 minute, 60°C for 30 seconds and 72°C for 3 minutes, and the final reaction at 72°C for 3 minutes. After precipitation of a PCR product with ethanol, the precipitate was dissolved into 25μl of 3 X SSC. 0.7% agarose gel electrophoresis was performed to confirm the quality of the obtained DNA and amplification efficiency of PCR.

(6) Production of cDNA microarray

Using a gene tip microarray stamp machine, GTMASS SYSTEM (Nippon Laser & Electronics Lab.), 0.5 μl of PCR product (100 to 500ng/ml) was loaded from a 384-well microtiter plate, and 5nl each of the product was spotted on 6 micro slide glasses (S7444, Matsunami) coated with poly-L-lysine at a space of 280 μm. To spot an equivalent amount of DNA, after printing, slides were wetted in a beaker containing hot distilled water and then dried at 100°C for 3 seconds. Thereafter, the slides were placed on a slide rack, and the rack was placed into a glass chamber. Then, a blocking solution (containing 15ml of 1M sodium borate salt (pH8.0), 5.5g of succinic anhydrous compound (Wako), and 335ml of 1-methyl-2-pyrrolidone (Wako)) was poured into the glass chamber. After shaking the glass chamber containing the slides rack up and down 5 times, it was further shaken gently for 15 minutes. Thereafter, the slide rack was transferred to a glass chamber containing boiling water and shaken 5 times, followed by being left at rest for 2 minutes. Then, the slide rack was transferred to a glass chamber containing 95% ethanol and shaken 5 times, followed by centrifugation (800rpm) for 30 minutes.

(7) Plant materials and isolation of RNA

As plant materials, there were used both a wild type *Arabidopsis thaliana* plant body which was seeded on an agar medium and grown for 3 weeks (Yamaguchi-Shinozaki and Shinozaki, 1994), and an *Arabidopsis thaliana* (Colombian species) plant body, into which DREB1A cDNA (Kasuga et al., 1999) connected to 35S promoter of a cauliflower mosaic virus was introduced. Drought-and cold-stress treatments were performed by the method of Yamaguchi-Shinozaki and Shinozaki (1994). That is to say, a plant body pulled out of an agar medium was placed on a filter and then dehydration treatment was carried out under conditions of 22°C and 60% relative humidity. Cold treatment was carried out by transferring the plant grown at 22°C into conditions of 4°C. High salt-stress treatment was carried out by water-culturing in a solution containing 250mM NaCl.

After wild type plant bodies were exposed to stress-treatment for 2 or 10 hours, they were subjected to sampling, and then subjected to cryopreservation with liquid nitrogen. Both wild type and DREB1A overexpression type transformants, which were cultured in an agar medium without kanamycin, were used for an experiment for identification of a DREB1A target gene. Stress treatment was not performed for DREB1A overexpression type transformants. The total RNA was isolated from plant bodies using ISOGEN (Nippon gene, Tokyo, Japan), and then mRNA was isolated and purified using an Oligotex-dT30 mRNA purification kit (Takara, Tokyo, Japan).

(8) Fluorescent labeling of probe

In the presence of Cy3 dUTP or Cy5 dUTP (Amersham Pharmacia), each of the mRNA samples was reverse transcribed. The composition of the reverse transcription buffer (30 μl) is as follows.

| | |
|---|---|
| poly $(A)^+$ RNA with 6 μg oligo (dT) 18-mer | 1 μg |
| 10mM DTT | |
| 500 μM dATP, dCTP and dGTP | |
| 200 μM dTTP | |
| 100 μM Cy3 dUTP or Cy5 dUTP | |
| 400 units of SuperScript II reverse transcription enzymes (Life Technologies) | |
| 1 X Superscript first strand buffer (Life Technologies) | |
| | 30 μl in total |

After reaction at 42°C for 1 hour, two samples (one sample labeled with Cy3 and the other sample labeled with Cy5) were mixed, and 15 μl of 0.1M NaOH and 1.5 μl of 20mM EDTA were added thereto and the mixture was treated at 70°C for 10 minutes. Then, 15 μl of 0.1M HCl was further added thereto, and the sample then transferred to a Micro con 30 micro concentrator (Amicon). 400 μl of TE buffer was added, followed by centrifugation so that the amount of the buffer became 10 to 20 μl, and then an effluent was thrown away. After 400 μl of TE buffer and 20 μl of 1mg/ml human Cot-1 DNA (Gibco BRL) were added thereto, the mixture was subjected to centrifugation again. The labeled samples were collected by centrifugation, and several μl of distilled water were added thereto. To the obtained probes, 2 μl of 10 μg/μl yeast tRNA, 2 μl of 1 μg/μl $pd(A)_{12-18}$ (Amersham Pharmacia), 3.4ml of 20 X SSC and 0.6 μl of 10% SDS were added. Furthermore, the samples were denatured at 100°C for 1 minute and placed at room temperature for 30 minutes, and then used for hybridization.

(9) Microarray hybridization and scanning

Using benchtop micro centrifuge, a probe was subjected to high-speed centrifugation for 1 minute. To avoid generation of bubbles, the probe was placed in the center of an array, and a cover slip was placed thereon. Four drops of 5 μl of 3 X SSC were dropped on a slide glass and a chamber was kept at suitable humidity to prevent dehydration of the probe during hybridization. The slide glass was placed into a cassette for hybridization (THC-1, BM machine) and sealed, followed by treatment at 65°C for 12 to 16 hours. The slide glass was taken out of the cassette and placed on a cassette rack, and a cover slip was carefully removed therefrom in solution 1 (2 X SSC, 0.1% SDS). Thereafter, the rack was shaken to wash, and transferred into solution 2 (1 X SSC) to wash for 2 minutes. Then, the rack was further transferred into solution 3 (0.2 X SSC) and left for 2 minutes, and then subjected to centrifugation (800rpm, 1 min) for drying.

Using a scanning laser microscope (ScanArray4000; GSI Lumonics, Watertown, MA), a microarray was scanned in a resolution of 10 μm per pixel. As a program for analyzing the microarray data, Imagene Ver 2.0 (BioDiscovery) and Quant Array (GSI Lumonics) were used.

(10) Northern analysis

Using total RNA, Northern analysis was carried out (Yamaguchi-Shinozaki and Shinozaki, 1994). DNA fragments isolated from *Arabidopsis thaliana* full-length cDNA libraries by PCR were used as probes for Northern hybridization.

(11) Determination of promoter region

Based on the genomic information of *Arabidopsis thaliana* in database (GenBank/EMBL, ABRC), a promoter region was analyzed using the BLAST program for gene analysis.

2. Results

(1) Stress-inducible gene

Fluorescent-labeled cDNA was prepared from mRNA, which were isolated from unstressed *Arabidopsis thaliana* plants, by reverse transcription in the presence of Cy5-dUTP. From cold-treated plants (2 hours), the second probes labeled with Cy3-dUTP were prepared. Both types of probes were simultaneously hybridized to a cDNA microarray comprising about 1,300 *Arabidopsis thaliana* cDNA clones, and then a pseudo color image was created (Figure 1).

Genes induced and inhibited by cold stress are represented by a red signal and a green signal, respectively. Genes which expressed at almost an equivalent level in both treatments are represented by a yellow signal. The strength of each spot corresponds to the absolute value of the expression level of each gene. It is shown that a cold-inducible gene (rd29A) is represented by a red signal whereas an α-tubulin gene (an internal control) is represented by a yellow signal.

By means of cDNA microarray analysis, the total 44 drought-inducible genes were identified (Tables 1 and 2).

Table 1.

| | Number of genes |
|---|---|
| Drought-inducible gene | 44 |
| New drought-inducible gene | 30 |
| Cold-inducible gene | 19 |
| New cold-inducible gene | 10 |
| DREB1A target gene | 12 |
| New DRE 1A target gene | 6 |

Table 2. Drought- and Cold-inducible Genes, and DREB1A Target Genes Identified by cDNA Microarray Analysis

| | | | Drought (2hr) | | Cold (2hr) | | 35S:DREB1A | |
|---|---|---|---|---|---|---|---|---|
| Gane | Accession | Coded Protein/ Other Features | Ratio | New or Reported | Ratio | New or Reported | Ratio | New or Reported |
| rd29A | D13044 | Hydrophilic protein | 6.4 | Reported | 5.1 | Reported | 7.9 | Reported |
| cor15a | U01377 | - | 5.0 | Reported | 3.4 | Reported | 8.1 | Reported |
| kin2 | X55053 | - | 5.8 | Reported | 2.9 | Reported | 4.9 | Reported |
| erd10 | D17714 | Group II LEA protein | 6.0 | Reported | 4.6 | Reported | 3.5 | Reported |
| kin1 | X51474 | - | 2.9 | Reported | 2.0 | Reported | 3.4 | Reported |
| rd17 | AB004872 | Group II LEA protein | 6.4 | Reported | 4.6 | Reported | 4.6 | Reported |
| rd20 | - | Ca-binding EF hand protein | 5.1 | Reported | n.d. | - | n.d. | - |
| erd7 | - | - | 3.8 | Reported | 2.3 | Reported | n.d. | - |
| erd4 | - | Membrane protein | 2.6 | Reported | 2.2 | Reported | 2.5 | New |
| erd3 | - | - | 2.6 | Reported | n.d. | - | n.d. | - |

(continued)

| Gane | Accession | Coded Protein/ Other Features | Drought (2hr) Ratio | Drought (2hr) New or Reported | Cold (2hr) Ratio | Cold (2hr) New or Reported | 35S:DREB1A Ratio | 35S:DREB1A New or Reported |
|---|---|---|---|---|---|---|---|---|
| FL3-519 | D17715 | Group II LEA protein | 3.5 | Reported | 1.5 | - | 1.2 | - |
| FL3-3A1 | D13042 | Thiol protease | 2.8 | Reported | 1.9 | - | 1.5 | - |
| FL5-1F23 | D32138 | Δ'-pyrroline-5-cerboxylate synthetase (AtP5CS) | 2.8 | Reported | 1.5 | - | n.d | - |
| FL2-1F6 | D01113 | Unidentified seed protein | 2.2 | Reported | 1.1 | - | 0.5 | - |
| FL3-5A3 | AC006438 | Putative cold acclimation protein | 6.2 | New | 2.3 | New | 3.4 | New |
| FL6-55 | X91919 | LEA76 type 1 protein | 2.9 | New | n.d. | - | n.d. | - |
| FL5-77 | AF121355 | Peroxiredoxin TRX1 | 2.2 | New | 1.9 | - | 3.0 | New |
| FL3-5J1 | AF057137 | Gamma tonoplast intrinsic protein 2 (TIP2) | 2.0 | New | 1.2 | - | 1.3 | - |
| FL5-1N11 | M80567 | Non-specific lipid transfer protein (LTP1) | 2.7 | New | 1.3 | - | 1.2 | - |
| FL5-95 | - | Rice glyoxalase 1 homolog | 2.3 | New | 2.8 | New | n.d. | - |
| FL5-2H15 | T45998 (EST) | - | 2.1 | New | n.d. | - | 1.4 | - |
| FL5-2O24 | AC005770 | Putative water channel protein | 2.4 | New | n.d | - | 1.4 | - |
| FL5-2G21 | AF034255 | Reversibly glycosylated polypeptide-3 (RGP) | 2.1 | New | n.d. | - | 1.3 | - |
| FL5-1A9 | - | Nodulin-like protein homolog | 2.9 | New | 2.1 | New | 0.8 | - |
| FL5-94 | X58107 | Enolase | 2.0 | New | 1.8 | - | 2.3 | New |
| FL5-3J4 | - | Heat shock protein dnaJ homolog | 2.8 | New | 1.4 | - | n.d | - |

(continued)

| Gane | Accession | Coded Protein/ Other Features | Drought (2hr) | | Cold (2hr) | | 35S:DREB1A | |
|---|---|---|---|---|---|---|---|---|
| | | | Ratio | New or Reported | Ratio | New or Reported | Ratio | New or Reported |
| FL5-3M24 | - | LEA protein SAG21 homolog | 2.3 | New | 2.2 | New | 1.0 | - |
| FL5-1O3 | H37392 (EST) | - | 3.0 | New | n.d. | - | n.d. | - |
| FL5-2I23 | D14442 | Ascorbate peroxidase | 2.1 | New | 1.1 | - | 1.0 | - |
| FL1-159 | AB015098 | HVA22 homolog | 3.7 | New | 3.8 | New | 1.9 | - |
| FL3-27 | - | Cysteine proteinase inhibitor homolog | 2.2 | New | n.d. | - | 2.2 | New |
| FL5-2I22 | X80342 | DC 1.2 homolog | 2.6 | New | 2.9 | New | 2.1 | New |
| FL5-1C20 | - | Major latex protein type 1 homolog | 2.0 | New | 1.4 | - | 1.8 | - |
| FL2-1H6 | - | *Brassica napus* jasmonate-inducible protein homolog | 2.4 | New | 1.3 | - | 0.9 | - |
| FL5-2E17 | - | *Brassica napus* bete-glucosidase homolog | 2.3 | New | 1.1 | - | 1.0 | - |
| FL3-3B1 | AC006403 | Hypothetical protein | 2.7 | New | 1.4 | - | 1.1 | - |
| FL5-3E18 | M80567 | Aquaporin homolog | 2.0 | New | 1.1 | - | 0.9 | - |
| FL5-3A15 | X94248 | Ferritin | 2.8 | New | 2.1 | New | 0.9 | - |
| FL2-56 | AF104330 | Glycine-rich protein 3 short isoform (GRP35) | 2.6 | New | 1.4 | - | 1.6 | - |
| FL5-2D23 | - | T20517 (EST) homolog | 2.6 | New | 1.3 | - | n.d. | - |
| FL3-2C6 | Z35474 | Thioredoxin | 2.3 | New | 1.0 | - | 1.9 | - |
| FL5-1P10 | AC004044 | Putative photosystem I reaction center subunit II precursor | 2.1 | New | 1.4 | - | 1.2 | - |
| FL2-5G7 | U43147 | Catalase 3 (CAT3) | 2.4 | New | 1.2 | - | 1.6 | - |

(continued)

| Gane | Accession | Coded Protein/ Other Features | Drought (2hr) Ratio | Drought (2hr) New or Reported | Cold (2hr) Ratio | Cold (2hr) New or Reported | 35S:DREB1A Ratio | 35S:DREB1A New or Reported |
|---|---|---|---|---|---|---|---|---|
| FL2-1C1 | Z9734 | Cysteine proteinase homolog | 3.0 | New | 1.0 | - | 1.7 | - |
| DREB1A | AB007787 | EREBP/AP2 protein | n.d. | - | 6.3 | Reported | 5.8 | - |
| FL2-5A4 | AB010259 | DEAD box ATPase/RNA helicase protein (DRH1) | n.d. | - | 2.1 | New | n.d. | - |
| FL5-90 | AJ250341 | β-amylase | n.d. | - | 4.4 | New | 1.2 | - |
| FL5-3P12 | D63510 | EXGT-A2 | 1.2 | - | 3.2 | New | 0.8 | - |

In Table 2, genes which are drought- and cold-inducible, and DREB1A target genes (35S:DREB1A) are rd29, cor15A, kin2, erd10, kin1, rd17, erd4, FL3-5A3, FL5-77, FL5-94, FL3-27 and FL5-2I22. Genes which are drought- and cold inducible but are not DREB1A target genes, are FL5-2024, FL5-1A9, FL5-3M24 and FL5-3A15. Specifically drought-inducible genes are rd20, FL6-55, FL5-3J4, FL2-56 and FL5-2D23. Specifically cold-inducible genes are DREB1A and FL5-90. The results of classification of these genes are shown in Figure 4.

Moreover, in Table 2, the "Coded Protein/Other Features" column shows putative functions of a gene product, which are predicted from sequence homology.

The "Ratio" in the "Drought" column is obtained by the following equation (FI: fluorescence intensity):

**Ratio = [(the FI of each cDNA under drought condition) / (the FI of each cDNA under unstressed condition)] ÷ [(the FI of $\alpha$-tubulin under drought condition) / (the FI of $\alpha$-tubulin under unstressed condition)]**

The "Ratio" in the "Cold" column is obtained by the following equation (FI: fluorescence intensity):

**Ratio = [(the FI of each cDNA under cold condition) / (the FI of each cDNA under unstressed condition)] ÷ [(the FI of $\alpha$-tubulin under cold condition) / (the FI of $\alpha$-tubulin under unstressed condition)]**

The "Ratio" in the "35S:DREB1A" column is obtained by the following equation (FI: fluorescence intensity):

**Ratio = [(the FI of each cDNA of 35S:DREB1A plants) / (the FI of each cDNA of wild type plants)] ÷ [(the FI of $\alpha$-tubulin of 35S:DREB1A plants) / (the FI of $\alpha$-tubulin of wild type plants)]**

With regard to the term "New or Reported" in the "Drought" column, where a gene has not been reported as a drought-inducible gene, it is shown as "New", and where it has been reported as a drought-inducible gene, it is shown as "Reported". The same applies for a cold-inducible gene in the "Cold" column and a DREB1A target gene in the "35S: DREB1A" column.

Among the genes described in Table 2, 14 genes (cor15A, kin1, kin2, rd17, rd19A, rd20, rd22, rd29A, erd3, erd4, erd7, erd10, erd14, AtP5CS) have previously been reported as drought-inducible genes (Bohnert, H.J. et al., (1995) Plant Cell 7, 1099-1111.; Ingram, J., and Bartels, D. (1996) Plant Mol. Biol. 47, 377-403.; Bray, E. A. (1997) Trends Plant Sci. 2, 48-54.; Shinozaki. K., and Yamaguchi-Shinozaki, K. (1997) Plant Physiol. 115, 327-334.; Shinozaki, K., and Yamaguchi-Shinozaki, K. (1999). Molecular responses to drought stress. Molecular responses to cold, drought, heat and salt stress in higher plants. Edited by Shinozaki, K. and Yamaguchi-Shinozaki, K., R. G. Landes Company.; Shinozaki, K., and Yamaguchi-Shinozaki, K. (2000) Curr. Opin. Plant Biol. 3, 217-223.; Taji, T. et al., (1999) Plant Cell Physiol. 40, 119-123.; Takahashi, S. et al., (2000) Plant Cell Physiol. 41, 898-903.)

From these results, it is shown that the cDNA microarray system of the present inventors has functioned appropriately to find stress-inducible genes. In the remaining 30 new drought-inducible genes, there were found cDNA molecules (FL3-5A3, FL6-55, FL5-1N11, FL5-2024, FL5-2H15 and FL1-159) which show sequence identity with putative cold acclimation protein (Accession No. AC006438), LEA 76 type 1 protein (Accession No. X91919), non-specific lipid transfer protein (LTP1; Accession No. M80567) putative water channel protein (Accession No. AC005770), T45998 EST, and HVA22 homolog (Accession No. AB015098).

Moreover, the total 19 cold-inducible genes have been identified by cDNA microarray analysis (Tables 1 and 2). Nine of these genes have been reported as cold-inducible genes, rd29A, cor15a, kin1, kin2, rd17, erd10, erd7 and erd4 (Kiyosue et al., 1994; Shinozaki and Yamaguchi-Shinozaki, 1997, 1999, 2000; Taji et al., 1999; Thomashow, 1999) and a DREB1A gene (Lui et al., 1998).

Likewise, in the remaining new cold-inducible genes, there were found cDNA molecules (FL3-5A3, FL5-3A15, FL5-3P12, FL5-90, FL5-2I22 and FL1-159) which show sequence identity with putative cold acclimation protein (Accession No. AC006438), ferritin (Accession No. X94248), EXGT-A2 (Accession No. D63510), β -amylase (Accession No. AJ250341), DC 1.2 homolog (Accession No. X80342), and HVA22 homolog (Accession No. AB015098), and also found cDNA molecules (FL5-1A9, FL5-95 and FL5-3M24) which show sequence similarity with Nodulin-like protein (Accession No. CAA22576), rice glyoxalase I (Accession No. AB017042) and LEA protein homolog (SAG21; Accession No. AF053065).

Furthermore, the present inventors have identified stress-inducible genes controlled by a DREB1A transcription factor, using a full-length cDNA microarray. Figure 2 shows a procedure for identification of DREB1A target genes. The mRNA prepared from a transgenic *Arabidopsis thaliana* plant (a 35S:DREB1A transgenic plant), which overexpresses DREB1A cDNA under the control of a CaMV 35S promoter, and the mRNA prepared from a wild type control plant were used to prepare Cy3-labeled and Cy5-labeled cDNA probes, respectively. These cDNA probes were mixed and then hybridized to a cDNA microarray. A gene having more than a two-fold greater expression level in a 35S:DREB1A transgenic plant than in a wild type control plant was defined as a DREB1A target gene.

The total 12 DREB1A target genes have been identified by cDNA microarray analysis (Tables 1 and 2). Six of these genes have been reported as DREB1A target genes, rd29A/cor78, cor15a, kin1, kin2, rd17/cor47 and erd10 (Kasuga et al., 1999). Likewise, in the remaining 6 new DREB1A target genes, there were found cDNA molecules (FL3-5A3, FL5-2I22, FL5-94 and FL5-77) which show sequence identity with putative cold acclimation protein (Accession No. AC006438), DC 1.2 homolog (Accession No. X80342), enolase (Accession No. X58107) and peroxiredoxin TPX1 (Accession No. AF121355), and also found a cDNA molecule (FL3-27) showing sequence similarity with a cowpea cysteine proteinase inhibitor (Accession No. Z21954) and erd4 cDNA (Kiyosue et al., 1994; Taji et al., 1999).

The identified drought- or cold-inducible genes were classified into the following 3 groups (Figure 4):

1) Drought- and cold-inducible genes
2) Specifically drought-inducible genes
3) Specifically cold-inducible genes

With regard to the following 21 genes, it was difficult to classify them into the above 3 groups and so they did not undergo such a classification: erd3, FL3-519, FL3-3A1, FL5-1F23, FL2-1F6, FL3-5J1, FL5-1N11, FL5-2H15, FL5-2G21, FL5-2I23, FL5-1C20, FL2-1H6, FL5-2E17, FL3-3B1, FL5-3E18, FL3-2C6, FL5-1P10, FL2-5G7, FL2-1C1, FL2-5A4, and FL5-3P12

As a result, the identified genes were classified into 20 drought- and cold-inducible genes, 5 specifically drought-inducible genes and 2 specifically cold-inducible genes. Thereafter, the drought- and cold-inducible genes were classified into two groups:

1) DREB1A target genes
2) Genes other than DREB1A target genes

Thus, 16 drought- and cold-inducible genes were classified into 12 DREB1A target genes and 4 genes other than DREB1A target genes.

(2) RNA gel blot analysis

In cDNA gel blot analysis, it is important to extract appropriate data with minimum effort. The present inventors evaluated the effectiveness of cDNA microarray analysis by the following method.

First, 80 genes having more than double the expression ratio (drought 2 hours/unstressed) of $\alpha$-tubulin were identified. The 80 putative drought-inducible genes were subjected to Northern blot analysis, 44 of which were identified as actual genes. The disparity between the results from microarray analysis and those from Northern blot analysis was caused by (1) low expression of genes, (2) high background, (3) dusts or scratches on a cDNA spot, and (4) a bad cDNA probe with a low specific activity. Accordingly, the above experimental data were marked and a half thereof was excluded from the following analysis. After the data processing, 44 drought-inducible genes, 19 cold-inducible genes and 12 DREB1A target genes were finally identified based on cDNA microarray analysis. Thereafter, RNA gel blot analysis was carried out to confirm the obtained results using a cDNA microarray. The result of expression data obtained by microarray analysis that 44 drought-inducible genes, 19 cold-inducible genes and 12 DREB1A target genes were identified, was consistent with the result obtained by Northern blot analysis.

Figure 3 shows a comparison between the result of microarray analysis and that of Northern blot analysis in respect of 6 new DREB1A target genes (FL3-5A3, FL3-27, FL5-2I22, FL5-94, FL5-77 and erd4). All of the 6 genes were induced by drought- and cold-treatments and overexpressed in 35S:DREB1A plants under unstressed conditions.

Samples derived from drought-treated wild type plants (dehydration for 2 hours or 10 hours (left on a filter paper)), cold-treated wild type plants (cooling at 4°C for 2 hours or 10 hours), or untreated 35S:DREB1A transgenic plants (35S:DREB1A control) were subjected to fluorescent labeling with Cy3-dUTP, whereas samples derived from untreated wild type plants (control) were subjected to fluorescent labeling with Cy5-dUTP. These samples were hybridized to a cDNA microarray followed by scanning to calculate a relative expression ratio and shown in a figure (Figure 3). The figure shows Northern blot analysis images of drought- and cold-treated wild type plants and 35S:DREB1A transgenic plants. The full-length cDNA sequences of two DREB1A target genes (FL3-5A3 and FL3-27) are independently registered with GenBank, EMBL and DDBJ database under Accession Nos. AB044404 and AB044405, respectively.

Likewise, using a full-length cDNA microarray containing about 7,000 Arabidopsis full-length cDNA molecules, 301 drought-inducible genes, 54 cold-inducible genes and 211 high salt stress-inducible genes were isolated.

(3) Identification of promoter region

As a result of identification of promoter regions, there were obtained the promoter regions of 8 types of genes (FL3-5A3, FL5-2H15, FL5-3M24, FL5-90, FL5-2I22, FL6-55, FL1-159 and FL5-2D23) obtained in a full-length cDNA microarray containing about 1,300 Arabidopsis full-length cDNA molecules. The sequences of these promoters are shown in SEQ ID NOS: 1 to 8.

| Gene Name | Promoter Region Sequence |
|---|---|
| FL3-5A3 | SEQ ID NO: 1 |
| FL5-2H15 | SEQ ID NO: 2 |
| FL5-3M24 | SEQ ID NO: 3 |
| FL5-90 | SEQ ID NO: 4 |
| FL5-2I22 | SEQ ID NO: 5 |
| FL6-55 | SEQ ID NO: 6 |
| FL1-159 | SEQ ID NO: 7 |
| FL5-2D23 | SEQ ID NO: 8 |

As a result of the identification of promoter regions, there were obtained the promoter regions of 10 types of genes (FL05-08-P24, FL05-09-G08, FL05-09-P10, FL05-10-N02, FL05-18-I12, FL05-21-F13, FL06-10-C16, FL06-15-P15, FL08-10-E21 and FL09-11-P10) obtained in a full-length cDNA microarray containing about 7,000 Arabidopsis full-length cDNA molecules. The sequences of these promoters are shown in SEQ ID NOS: 9 to 18.

| Gene Name | Promoter Region Sequence |
|---|---|
| FL05-08-P24 | SEQ ID NO: 9 |
| FL05-09-G08 | SEQ ID NO: 10 |

(continued)

| Gene Name | Promoter Region Sequence |
|---|---|
| FL05-09-P10 | SEQ ID NO: 11 |
| FL05-10-N02 | SEQ ID NO: 12 |
| FL05-18-I12 | SEQ ID NO: 13 |
| FL05-21-F13 | SEQ ID NO: 14 |
| FL06-10-C16 | SEQ ID NO: 15 |
| FL06-15-P15 | SEQ ID NO: 16 |
| FL08-10-E21 | SEQ ID NO: 17 |
| FL09-11-P10 | SEQ ID NO: 18 |

It is known that a conserved sequence "PyACGTG (G or T)C" (wherein Py represents a pyrimidine base, that is, C or T) acts as an ABA-responsive element (ABRE) in many ABA-responsive genes (Ingram, J., and Bartels, D. (1996) Plant Mol. Biol. 47, 377-403.; Bray, E. A. (1997) Trends Plant Sci. 2, 48-54.; Shinozaki, K., and Yamaguchi-Shinozaki, K. (1999). Molecular responses to drought stress. Molecular responses to cold, drought, heat and salt stress in higher plants. Edited by Shinozaki, K. and Yamaguchi-Shinozaki, K., R. G. Landes Company.) Furthermore, it is also known that a conserved sequence consisting of 9 nucleotides "TACCGACAT" (a dehydration-responsive element: DRE) is essential to the induction control of rd29A expression under drought-, cold- and high salt-stress conditions, but does not function as an ABA-responsive element (ABRE) (Yamaguchi-Shinozaki, K., and Shinozaki, K. (1994) Plant Cell 6, 251-264). It is also known that CRT or LTRE, which is DRE or DRE-related core motifs (CCCAG), exists in the regions of drought- and cold-inducible genes (e.g. kin1, kin2, rd17/cor47 and cor15a) (see Table 3, Baker, S. S. et al., (1994) Plant Mol. Biol. 24, 701-713.; Wang, H. et al., (1995) Plant Mol. Biol. 28, 605-617.; Iwasaki, T. et al., (1997) Plant Physiol. 115, 1287.)

Table 3. ABRE, DRE, and CCGAC Core Sequences Observed in the Promoter Regions of the DREB1A Target Genes Identified by cDNA Microarray Analysis a)

| Gene | ABRE (PyACGTG(T/G)C) | DRE (TACCGACAT) | CCGAC Core Motif (CCGAC) |
|---|---|---|---|
| rd29A | TACGTGTC(-45 to -38) b) | TACCGACAT(-148 to -140)<br>TACCGACAT(-206 to -198) | AGCCGACAC(-111 to -103)<br>GACCGACTA(-256 to -248) |
| cor15A | CACGTGGC(-132 to -125) | - | GGCCGACCT(-184 to -176)<br>GGCCGACAT(-361 to -353)<br>AACCGACAA(-416 to -424) |
| kin1 | - | TACCGACAT(-120 to -112) | ATCCGACAT(-720 to -712) |
| kin2 | CACGTGGC(-68 to -61) | TACCGACAT(-127 to -119) | CCCCGACGC(-403 to -395) |
| rd17 | TACGTGTC(-920 to -913) | - | TACCGACTT(-162 to -154)<br>AGCCGACCA(-967 to -959)<br>GACCGACAT(-997 to -989) |
| erd10 | CACGTGGC(-838 to -831) | - | GACCGACGT(-198 to -190) c)<br>GACCGACCG(-202 to -194) c)<br>CACCGACCG(-206 to -198) c)<br>GACCGACAT(-999 to -991) |

(continued)

| Gene | ABRE (PyACGTG(T/G)C) | DRE (TACCGACAT) | CCGAC Core Motif (CCGAC) |
|---|---|---|---|
| FL3-5A3 | CACGTGGC(-74 to -67) | TACCGACAT(-415 to -407) | TGCCGACAT(-806 to -798) |
| FL3-27 | - | TACCGACAT(-89 to -81) | - |
| FL5-2122 | - | - | TACCGACTC(-191 to -183)<br>TACCGACTA(-266 to -258)<br>TGCCGACCT(-418 to -410)<br>ACCCGACTA(-695 to -703)<br>GACCGACGT(-786 to -778) |
| FL5-77 | - | - | CCCCGACTA(-315 to -307) |
| FL5-94 | - | - | TACCGACTA(-190 to -198)<br>TTCCGACTA(-260 to -268)<br>ATCCGACGC(-630 to -622) |

In Table 3, a), b) and c) are defined as follows:
a): ABRE, DRE and CCGAC core sequences refer to sequences observed at 1,000bp upstream of the 5'-terminus of the longest cDNA isolated.
b): Figures in parentheses represent nucleotides initiating at the 5'-termini of the isolated cDNA. A minus sign means that a nucleotide exists upstream of the 5'-terminus of a putative transcription initiation site.
c): Each of these sequences consisting of 9 nucleotides comprises a CCGAC core motif, and the sequences overlap with one another.

The present inventors identified 12 DREB1A target genes by cDNA microarray analysis. A DRE sequence of 9bp is observed in the promoter regions of genes corresponding to the cDNA molecules of FL3-5A3 and FL3-27 (Table 3). A core, sequence "CCGAC" is observed in the promoter regions of genes corresponding to the cDNA molecules of FL3-5A3, FL5-2I22, FL5-77 and FL5-94 (Table 3). Almost all of the drought- and cold-inducible genes are DREB1A target genes, each of which contains a DRE/CRT *cis*-acting element in a promoter thereof (Table 3 and Figure 4). An ABRE sequence ("PyACGTG(G or T)C") was observed in 6 promoter regions among the identified 12 DREB1A target genes (Table 3). This shows that many drought- and cold-inducible genes are controlled by both ABA-dependent and ABA-independent routes. However, some drought- and cold-inducible genes (FL5-3M24, FL5-3A15, FL5-1A9 and FL5-2024) did not increase in 35S:DREB1A transgenic plants (Figure 4). This shows that these genes are not DREB1A target genes. A core sequence "CCGAC" was not observed in a region 2,000 bp upstream of the 5'-terminus of cDNA of FL5-3M24. This result shows a possibility that a new *cis*-acting element associated with expression of drought- and cold-inducible genes exists in the promoter region of a FL5-3M24 gene.

(4) Relation between various stress treatment periods of time and expression ratio

With regard to 18 types of stress-inducible genes isolated as above, the results of analysis of the relation between various stress treatment periods of time and expression ratio are shown in the following Figures 5 to 39.

| Gene Name | Stress Result (Figures showing the results) |
|---|---|
| FL3-5A3 | Cold (Fig. 5), Drought (Fig. 6) and High Salt (Fig. 7) |
| FL5-2H15 | Cold (Fig. 8), Drought (Fig. 9) and High Salt (Fig. 10) |
| FL5-3M24 | Drought (Fig. 11) and High Salt (Fig. 12) |
| FL5-90 | Cold (Fig. 13) |
| FL5-2I22 | Cold (Fig. 14), Drought (Fig. 15) and High Salt (Fig. 16) |
| FL6-55 | Drought (Fig. 17) and High Salt (Fig. 18) |
| FL1-159 | Drought (Fig. 19) |
| FL5-2D23 | Drought (Fig. 20) and High Salt (Fig. 21) |
| FL05-08-P24 | Drought (Fig. 22) |

(continued)

| Gene Name | Stress Result (Figures showing the results) |
|---|---|
| FL05-09-G08 | Drought (Fig. 23) |
| FL05-09-P10 | Drought (Fig. 24) and ABA (Fig. 25) |
| FL05-10-N02 | High Salt (Fig. 26) |
| FL05-18-I12 | Drought (Fig. 27), High Salt (Fig. 28) and ABA (Fig. 29) |
| FL05-21-F13 | Drought (Fig. 30) and Cold (Fig. 31) |
| FL06-10-C16 | Drought (Fig. 32), High Salt (Fig. 33) and ABA (Fig. 34) |
| FL06-15-P15 | Drought (Fig. 35), High Salt (Fig. 36) and ABA (Fig. 37) |
| FL08-10-E21 | Drought (Fig. 38) |
| FL-9-11-P10 | High Salt (Fig. 39) |

As shown in Figures 5 to 39, the stress-inducible genes isolated by the method of the present invention are different profiles, but are expressions induced by the addition of various types of stresses.

Effect of the Invention

According to the present invention, a stress responsive promoter as defined in the claims 1 and 2 is provided. The promoter of the present invention is useful in that it can be used for molecular breeding of environmental stress-resistant plants.

## Claims

1. An environmental stress responsive promoter comprising DNA of the following (a) or (b):

(a) DNA consisting of a nucleotide sequence identified as SEQ ID NO: 1; and
(b) DNA consisting of a nucleotide sequence comprising a deletion, substitution or addition of one to ten nucleotides relative to the nucleotide sequence identified as SEQ ID NO: 1, and functioning as an environmental stress responsive promoter.

**2.** The promoter according to claim 1, wherein the environmental stress is at least one selected from the group consisting of cold stress, drought stress, and salt stress.

**3.** An expression vector comprising the promoter according to claim 1 or 2.

**4.** The expression vector according to claim 3, into which a desired gene is further incorporated.

**5.** A transformant comprising the expression vector according to claim 3 or 4.

**6.** A transgenic plant comprising the expression vector according to claim 3 or the recombinant vector according to claim 4.

**7.** The transgenic plant according to claim 6, wherein the plant is a plant body, plant organ, plant tissue or plant culture cell.

## Patentansprüche

**1.** Auf Umweltstress reagierender Promotor, der DNA der folgenden (a) oder (b) enthält:

(a) DNA, die aus einer als SEQ ID NO: 1 bestimmten Nukleotidsequenz besteht; und
(b) DNA, die aus einer Nukleotidsequenz besteht, die eine Deletion, Substitution oder Addition von eins bis zehn Nukleotiden relativ zu der als SEQ ID NO: 1 bestimmten Nukleotidsequenz aufweist, und die als ein auf Umweltstress reagierender Promotor wirkt.

**2.** Promotor nach Anspruch 1, wobei der Umweltstress zumindest einer ausgewählt aus der aus Kältestress, Trokkenheitsstress, und Salzstress bestehenden Gruppe ist.

**3.** Expressionsvektor, der den Promotor nach Anspruch 1 oder 2 enthält.

**4.** Expressionsvektor nach Anspruch 3, in welchen ferner ein gewünschtes Gen eingebracht ist.

**5.** Transformant, der den Expressionsvektor nach Anspruch 3 oder 4 enthält.

**6.** Transgenes Gewächs, das den Expressionsvektor nach Anspruch 3 oder den rekombinanten Vektor nach Anspruch 4 enthält.

**7.** Transgenes Gewächs nach Anspruch 6, wobei das Gewächs ein Pflanzenkörper, Pflanzenorgan, Pflanzengewebe oder eine Pflanzenkulturzelle ist.

## Revendications

**1.** Promoteur sensible à un stress environnemental comprenant un ADN du (a) ou du (b) suivant :

(a) ADN consistant en une séquence nucléotidique identifiée comme SEQ ID N°1 ; et
(b) ADN consistant en une séquence nucléotidique comprenant une délétion, une substitution ou une addition de un à dix nucléotides par rapport à la séquence nucléotidique identifiée comme SEQ ID N°1, et fonctionnant comme un promoteur sensible à un stress environnemental.

**2.** Promoteur selon la revendication 1, dans lequel le stress environnemental est au moins un choisi dans le groupe constitué d'un stress froid, un stress sec et un stress salin.

**3.** Vecteur d'expression comprenant le promoteur selon la revendication 1 ou 2.

4. Vecteur d'expression selon la revendication 3, dans lequel, en outre, un gène désiré est incorporé.

5. Transformant comprenant le vecteur d'expression selon la revendication 3 ou 4.

6. Plante transgénique comprenant le vecteur d'expression selon la revendication 3 ou le vecteur recombinant selon la revendication 4.

7. Plante transgénique selon la revendication 6, dans laquelle la plante est un organisme végétal, un organe végétal, un tissu végétal ou une culture cellulaire végétale.

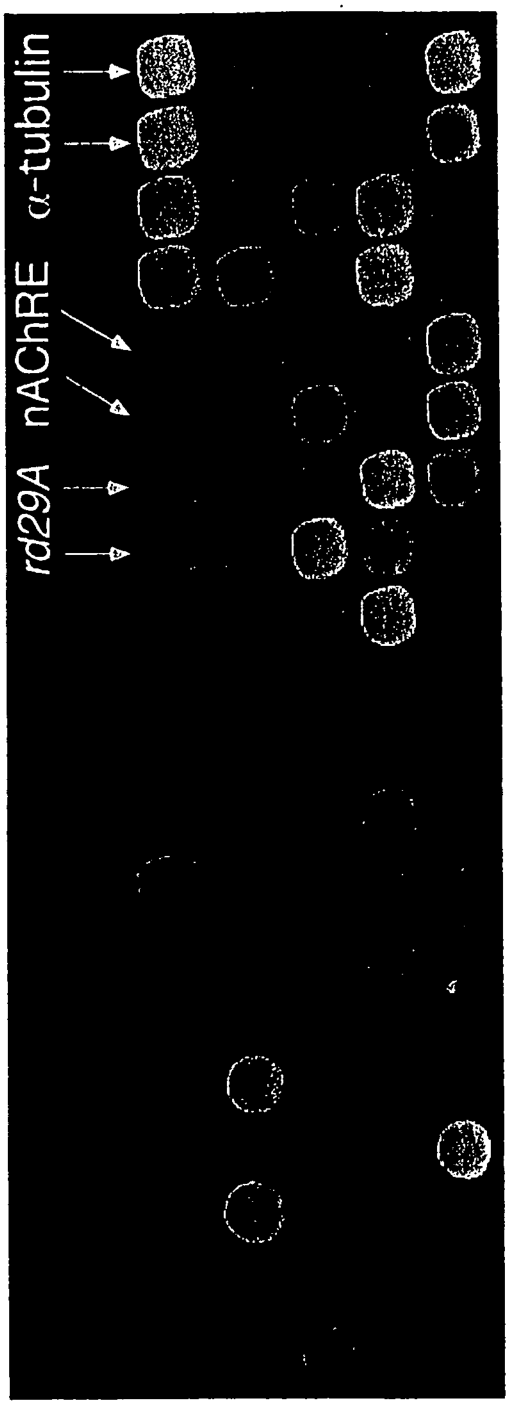


FIG.1

FIG.2

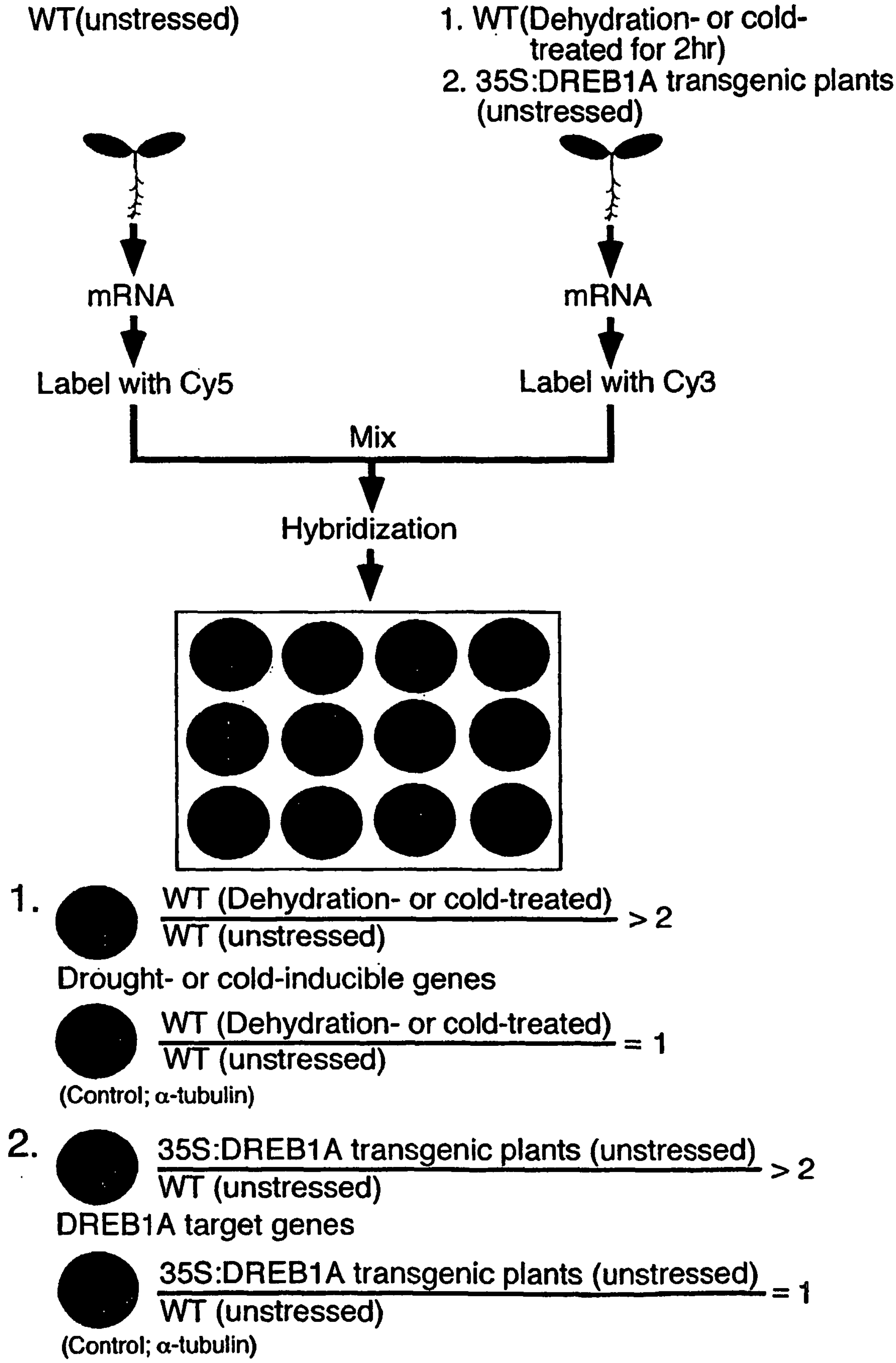
WT(unstressed)
1. WT(Dehydration- or cold-treated for 2hr)
2. 35S:DREB1A transgenic plants (unstressed)
mRNA
mRNA
Label with Cy5
Label with Cy3
Mix
Hybridization
1.
WT (Dehydration- or cold-treated) / WT (unstressed) > 2
Drought- or cold-inducible genes
WT (Dehydration- or cold-treated) / WT (unstressed) = 1
(Control; α-tubulin)
2.
35S:DREB1A transgenic plants (unstressed) / WT (unstressed) > 2
DREB1A target genes
35S:DREB1A transgenic plants (unstressed) / WT (unstressed) = 1
(Control; α-tubulin)

FIG.3

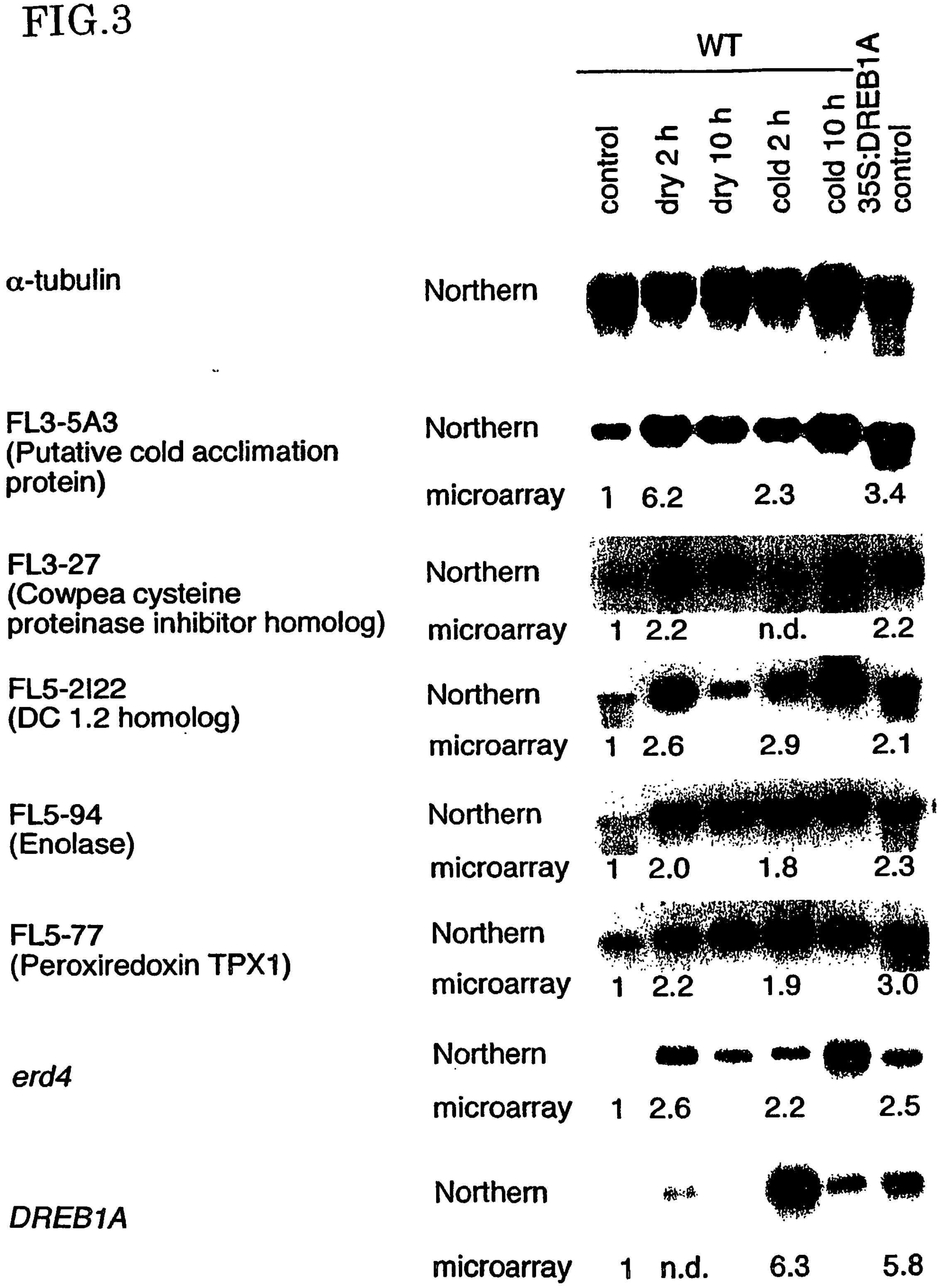
WT
control
dry 2 h
dry 10 h
cold 2 h
cold 10 h
35S:DREB1A
control
α-tubulin
Northern
FL3-5A3
(Putative cold acclimation protein)
Northern
microarray 1 6.2 2.3 3.4
FL3-27
(Cowpea cysteine proteinase inhibitor homolog)
Northern
microarray 1 2.2 n.d. 2.2
FL5-2I22
(DC 1.2 homolog)
Northern
microarray 1 2.6 2.9 2.1
FL5-94
(Enolase)
Northern
microarray 1 2.0 1.8 2.3
FL5-77
(Peroxiredoxin TPX1)
Northern
microarray 1 2.2 1.9 3.0
erd4
Northern
microarray 1 2.6 2.2 2.5
DREB1A
Northern
microarray 1 n.d. 6.3 5.8

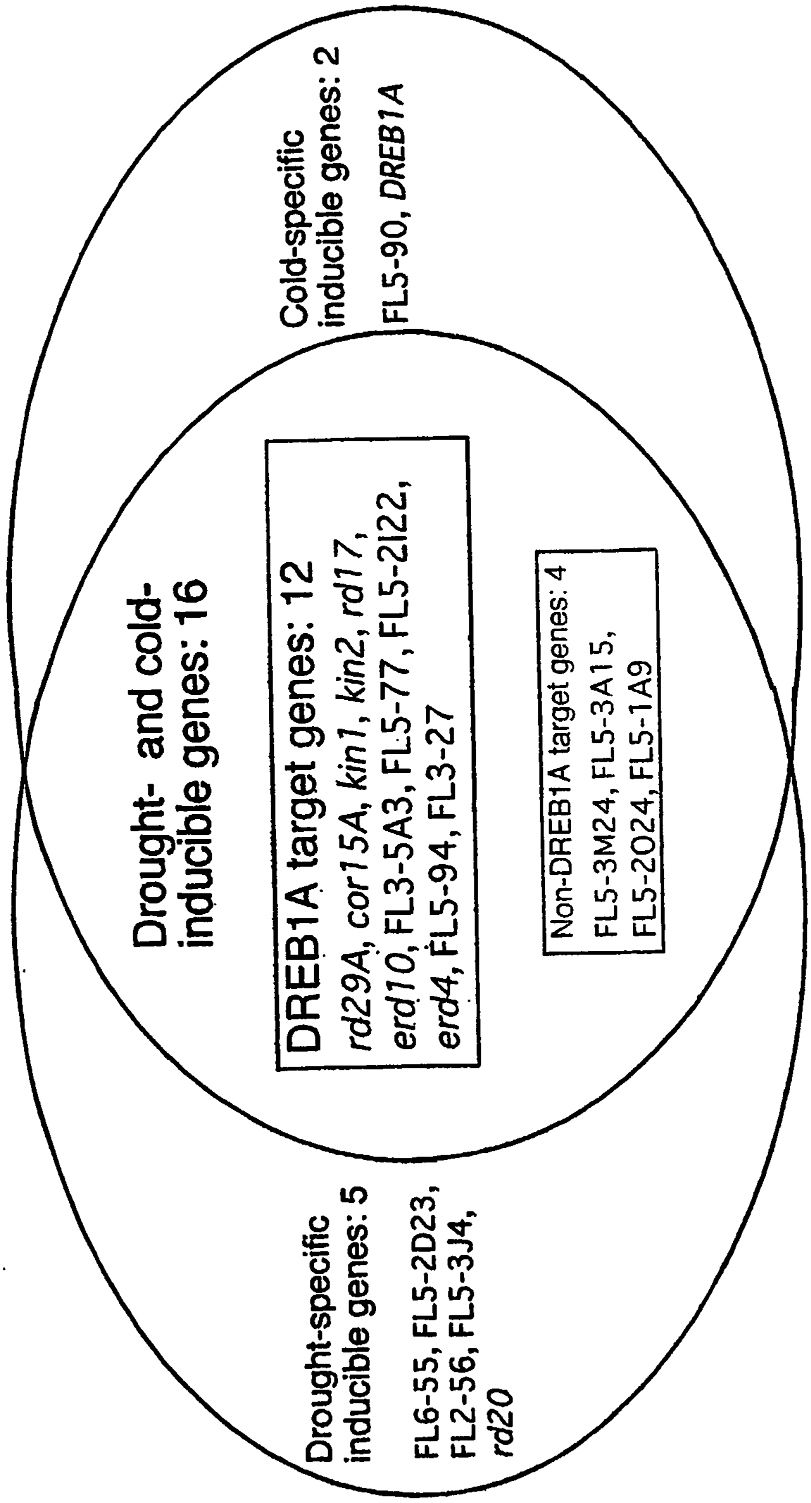
Cold-specific inducible genes: 2
FL5-90, DREB1A
Drought- and cold-inducible genes: 16
DREB1A target genes: 12
rd29A, cor15A, kin1, kin2, rd17, erd10, FL3-5A3, FL5-77, FL5-2I22, erd4, FL5-94, FL3-27
Non-DREB1A target genes: 4
FL5-3M24, FL5-3A15, FL5-2O24, FL5-1A9
Drought-specific inducible genes: 5
FL6-55, FL5-2D23, FL2-56, FL5-3J4, rd20

FIG.4

FIG.5

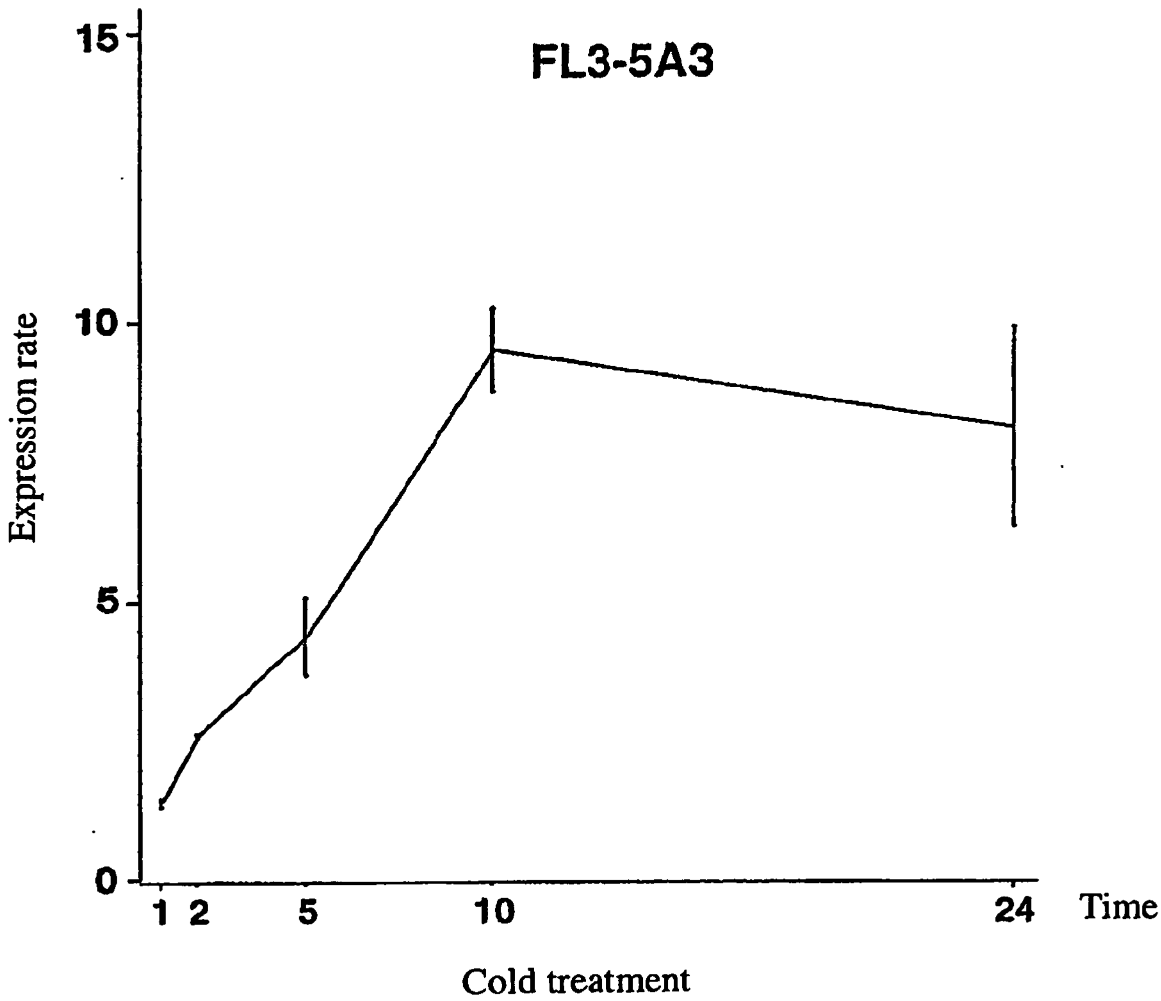
FL3-5A3
Expression rate
15
10
5
0
1
2
5
10
24
Time
Cold treatment

FIG.6

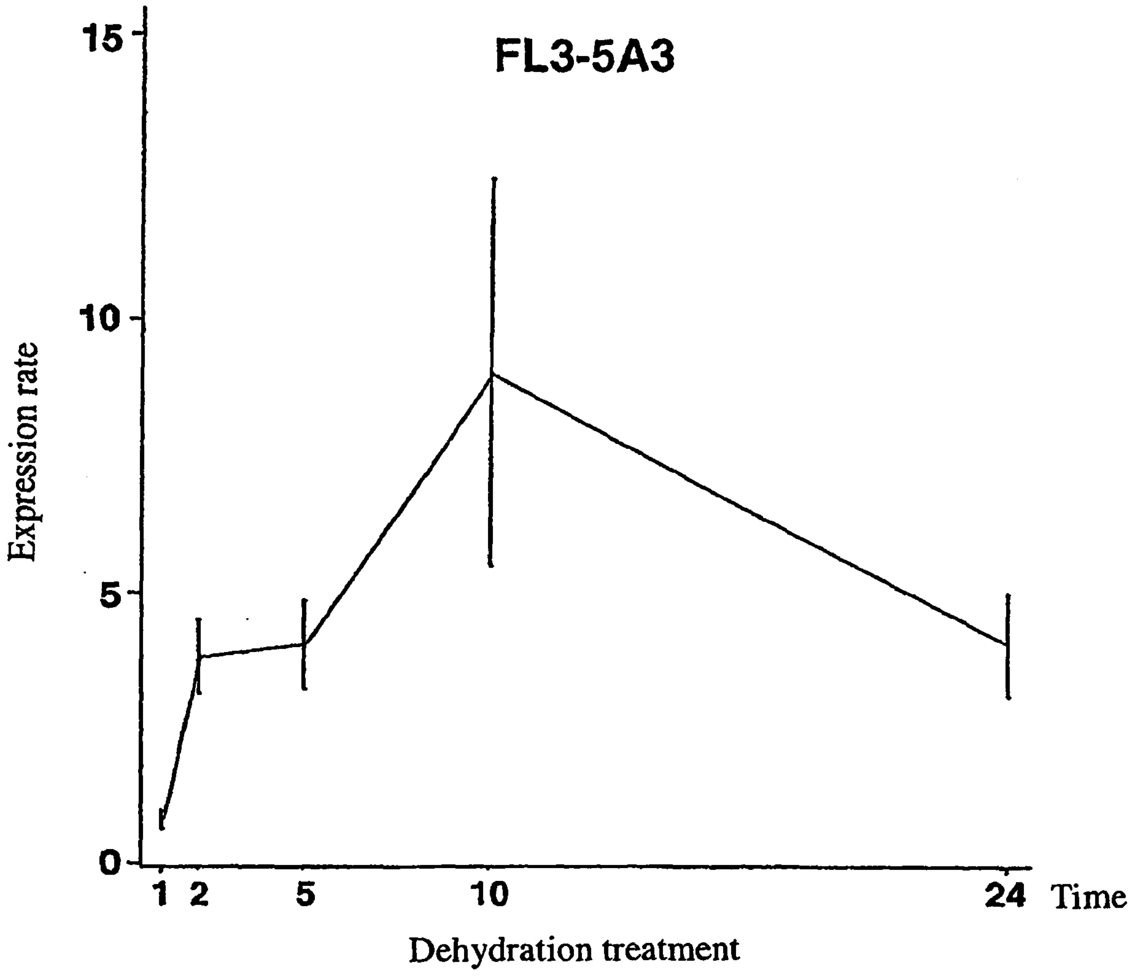
FL3-5A3
Expression rate
15
10
5
0
1
2
5
10
24
Time
Dehydration treatment

FIG.7

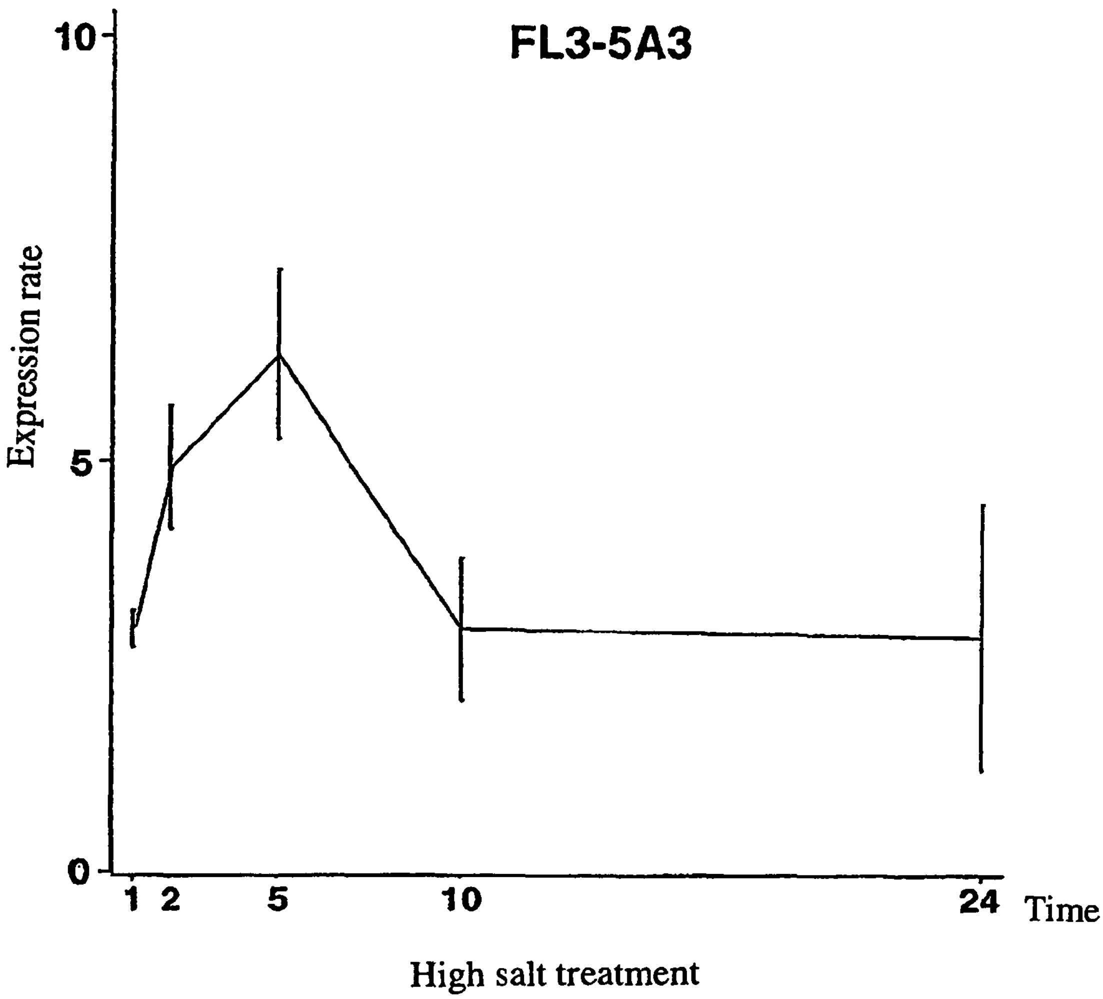
FL3-5A3
10
5
0
Expression rate
1 2 5 10 24 Time
High salt treatment

FIG.8

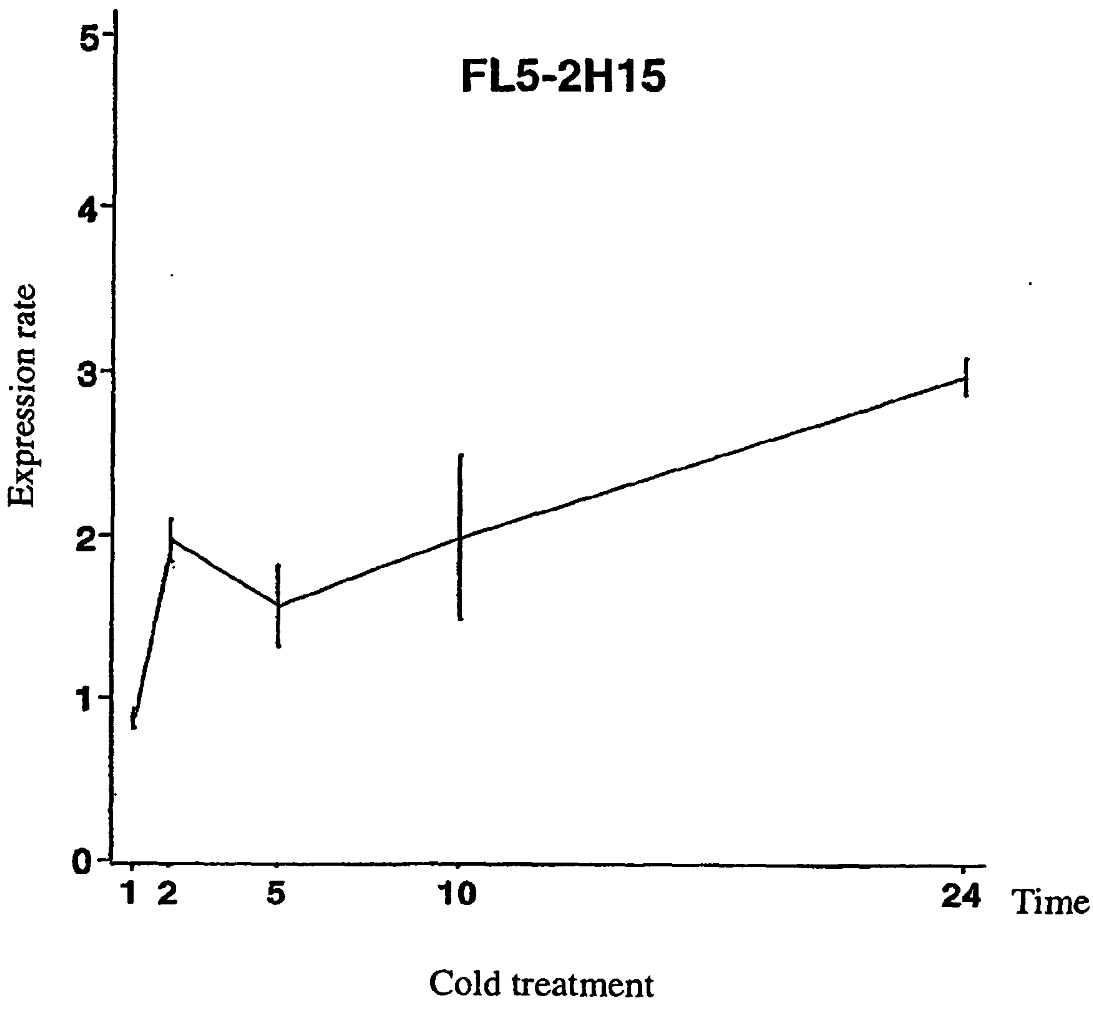
FL5-2H15
Expression rate
5
4
3
2
1
0
1 2 5 10 24 Time
Cold treatment

FIG.9

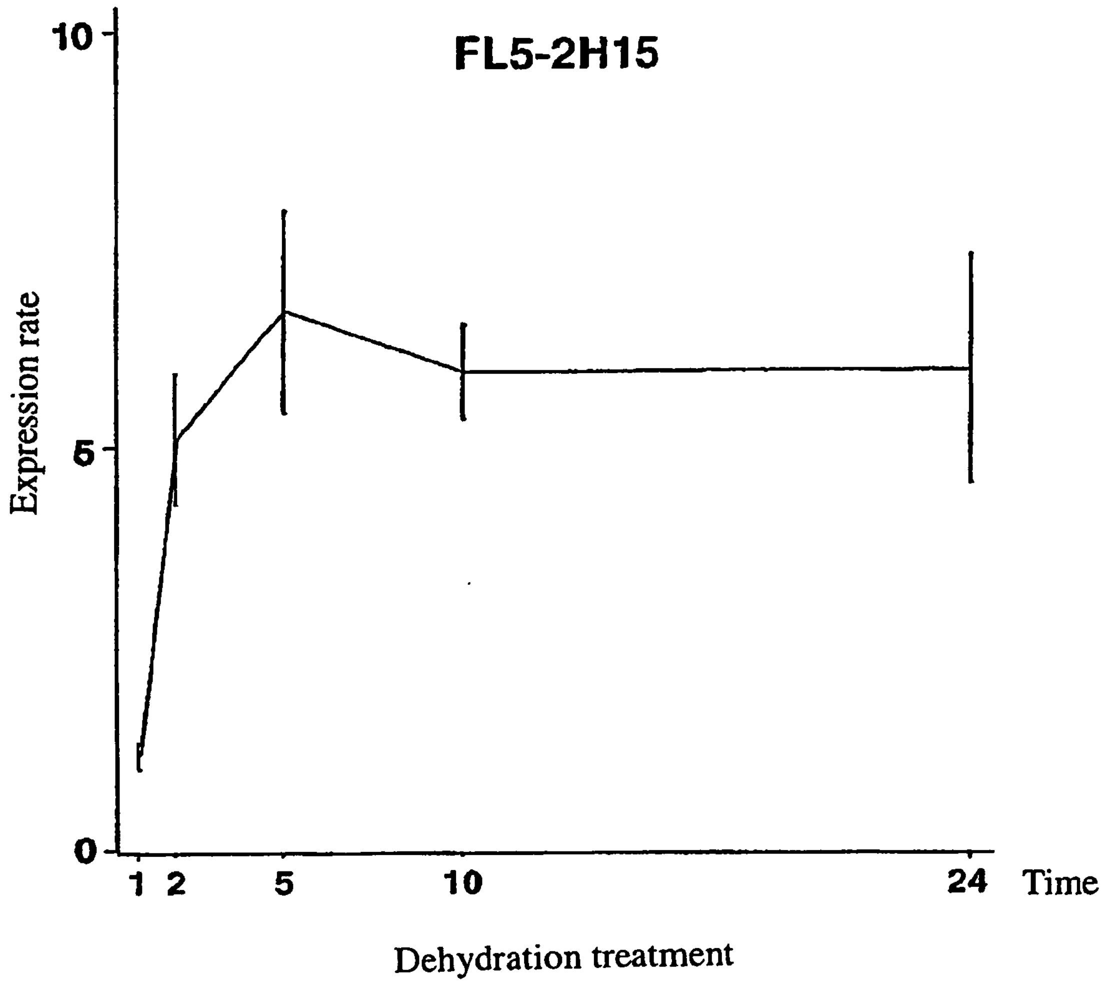
FL5-2H15
10
5
0
Expression rate
1 2 5 10 24 Time
Dehydration treatment

FIG.10

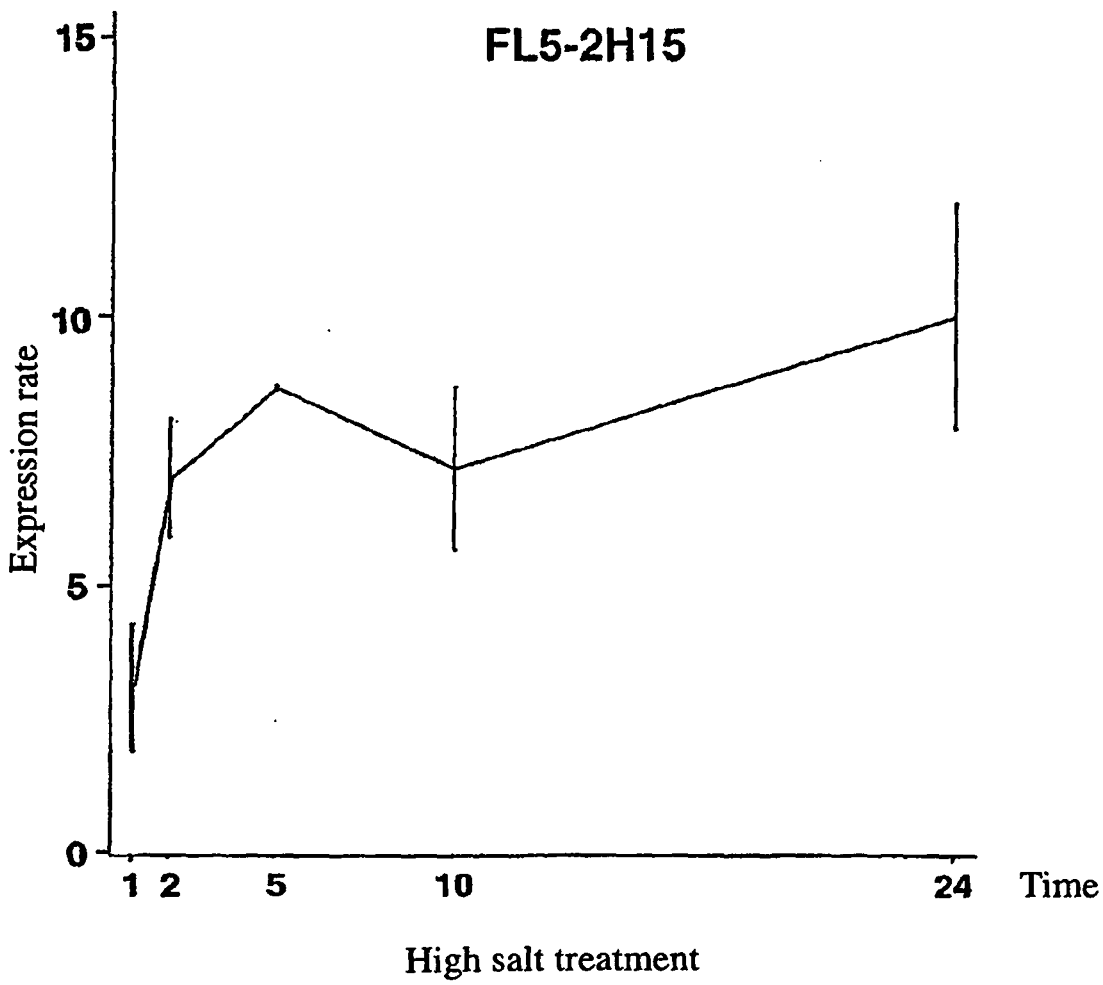
FL5-2H15
15
10
5
0
Expression rate
1 2 5 10 24 Time
High salt treatment

FIG.11

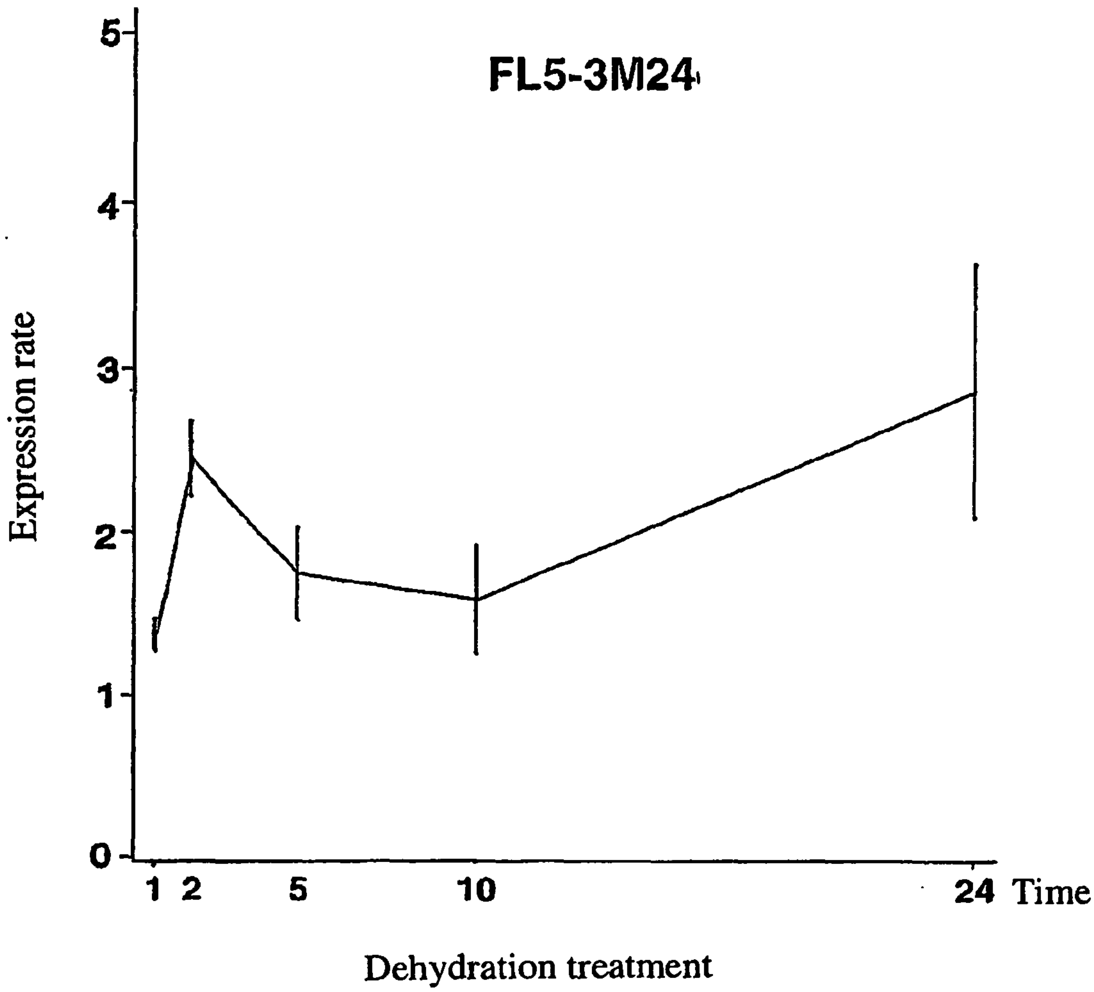
FL5-3M24
Expression rate
0
1
2
3
4
5
1 2 5 10 24 Time
Dehydration treatment

FIG.12

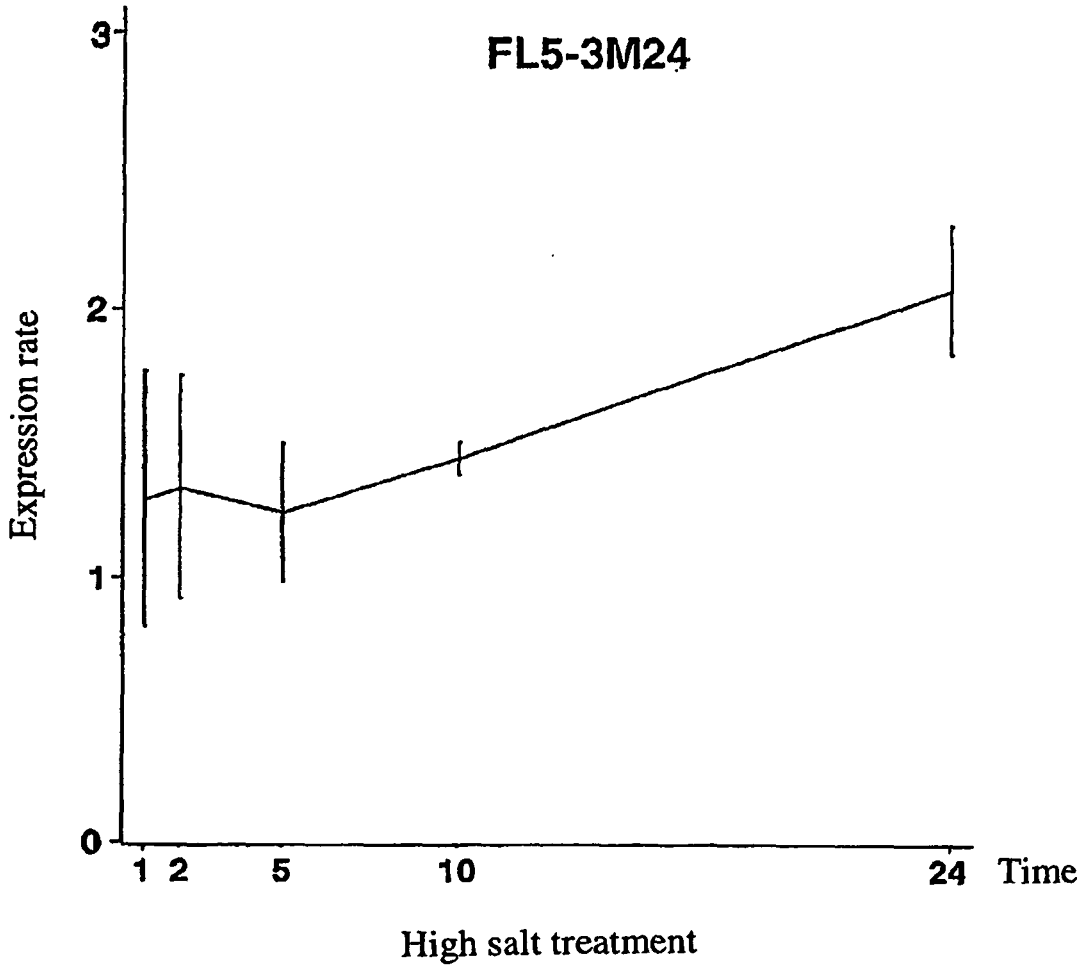
FL5-3M24
Expression rate
0
1
2
3
1
2
5
10
24
Time
High salt treatment

FIG.13

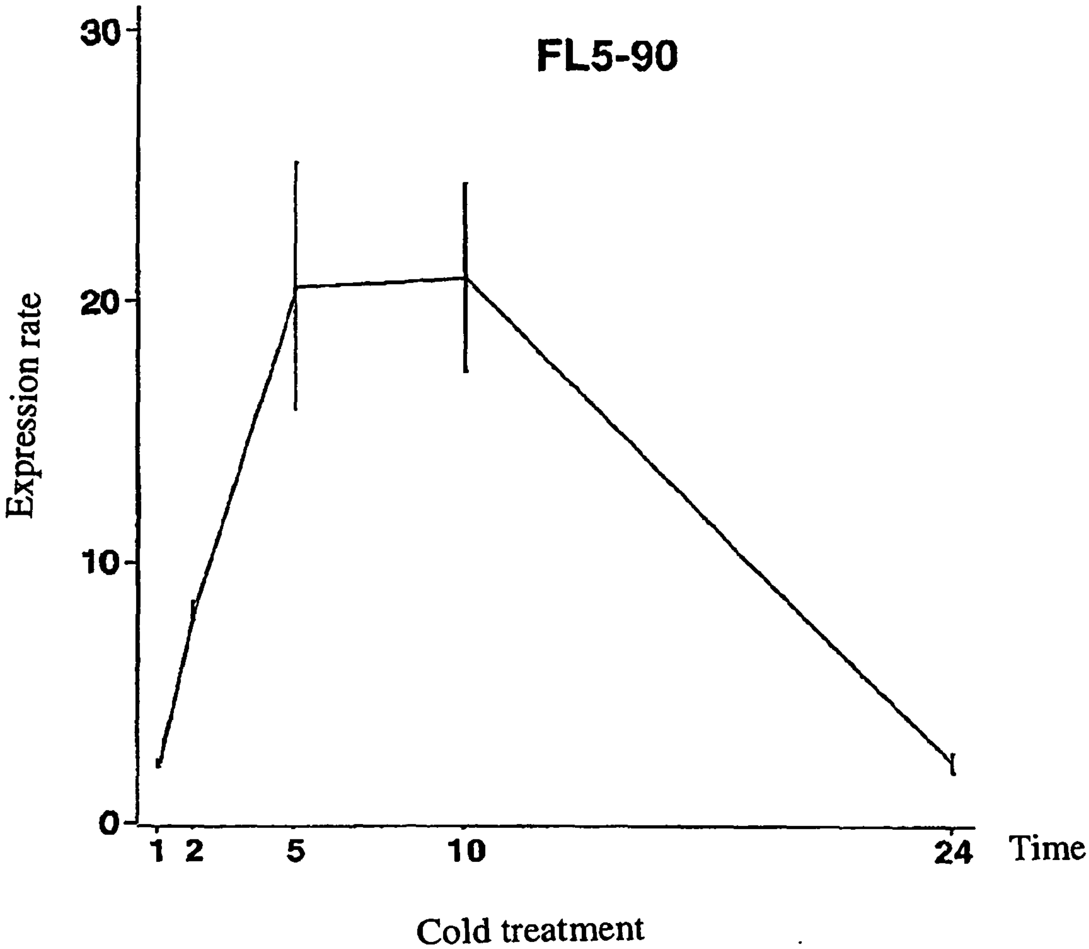
FL5-90
30
20
10
0
Expression rate
1
2
5
10
24
Time
Cold treatment

FIG.14

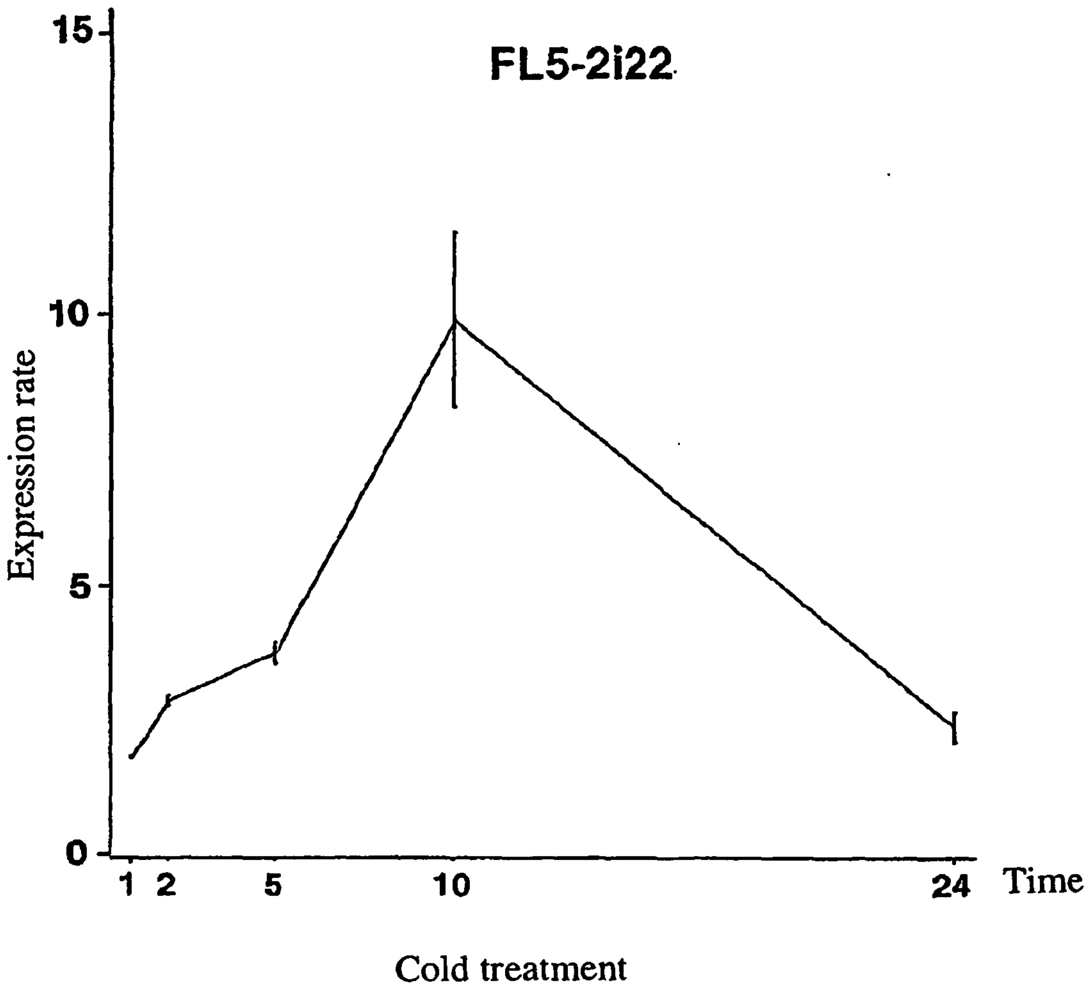
FL5-2i22
15
10
5
0
Expression rate
1
2
5
10
24
Time
Cold treatment

FIG.15

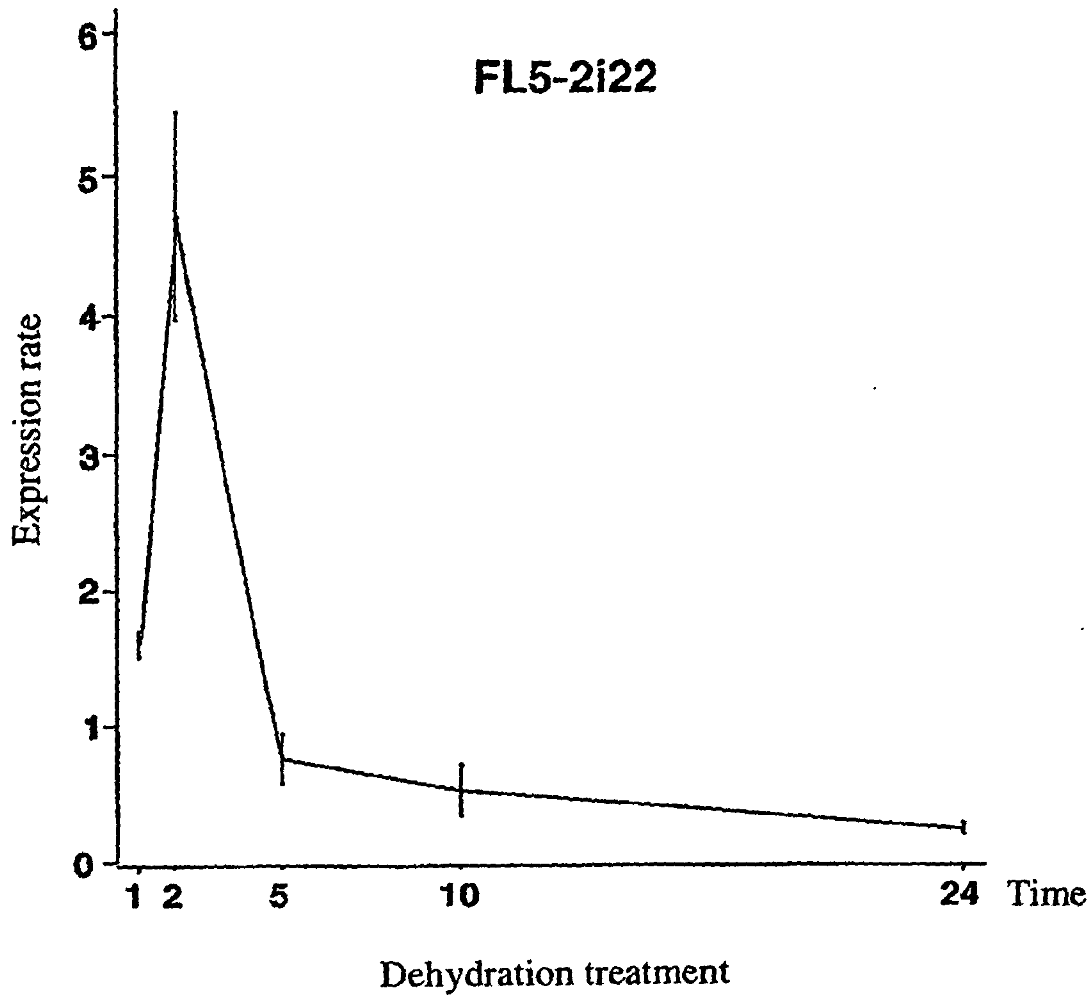
FL5-2i22
Expression rate
0
1
2
3
4
5
6
1 2 5 10 24 Time
Dehydration treatment

FIG.16

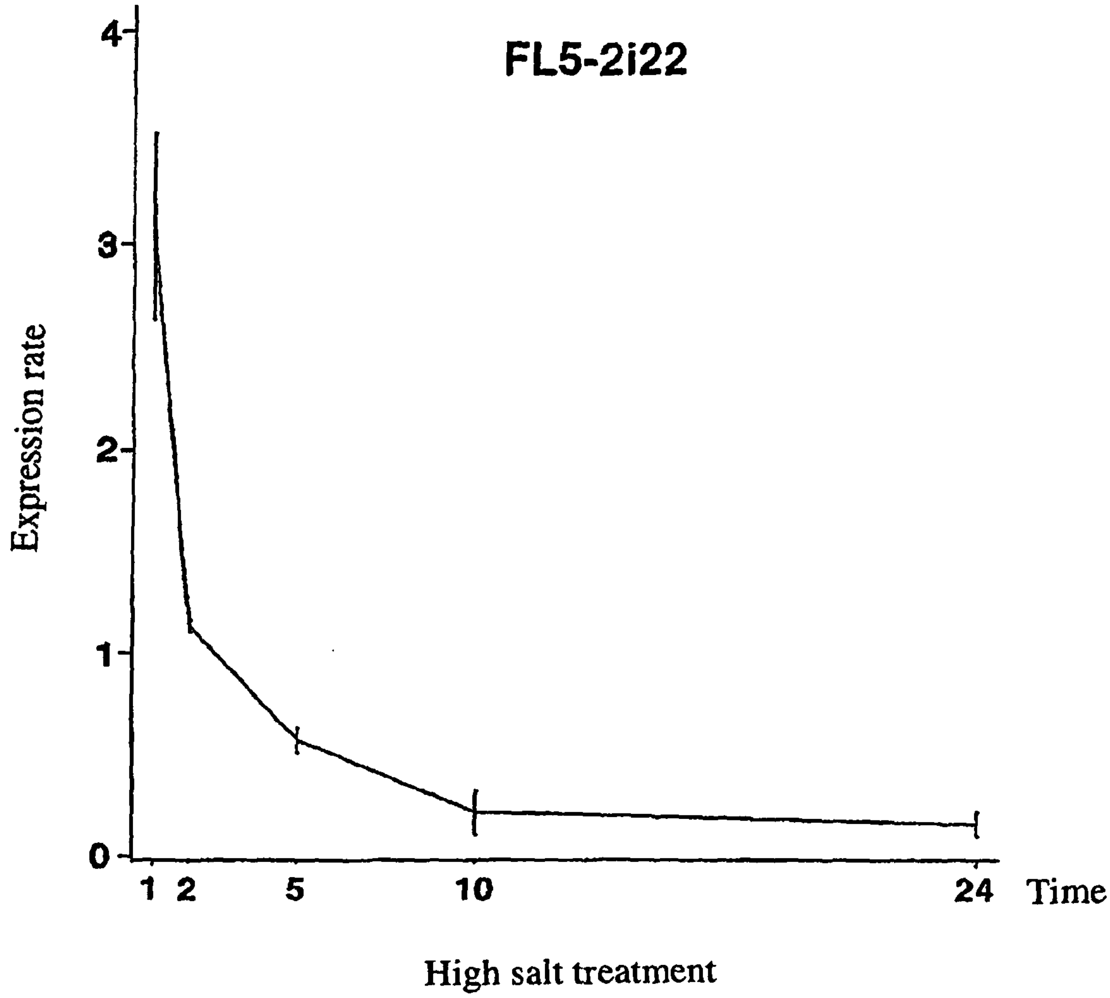
FL5-2i22
Expression rate
0
1
2
3
4
1 2 5 10 24 Time
High salt treatment

FIG.17

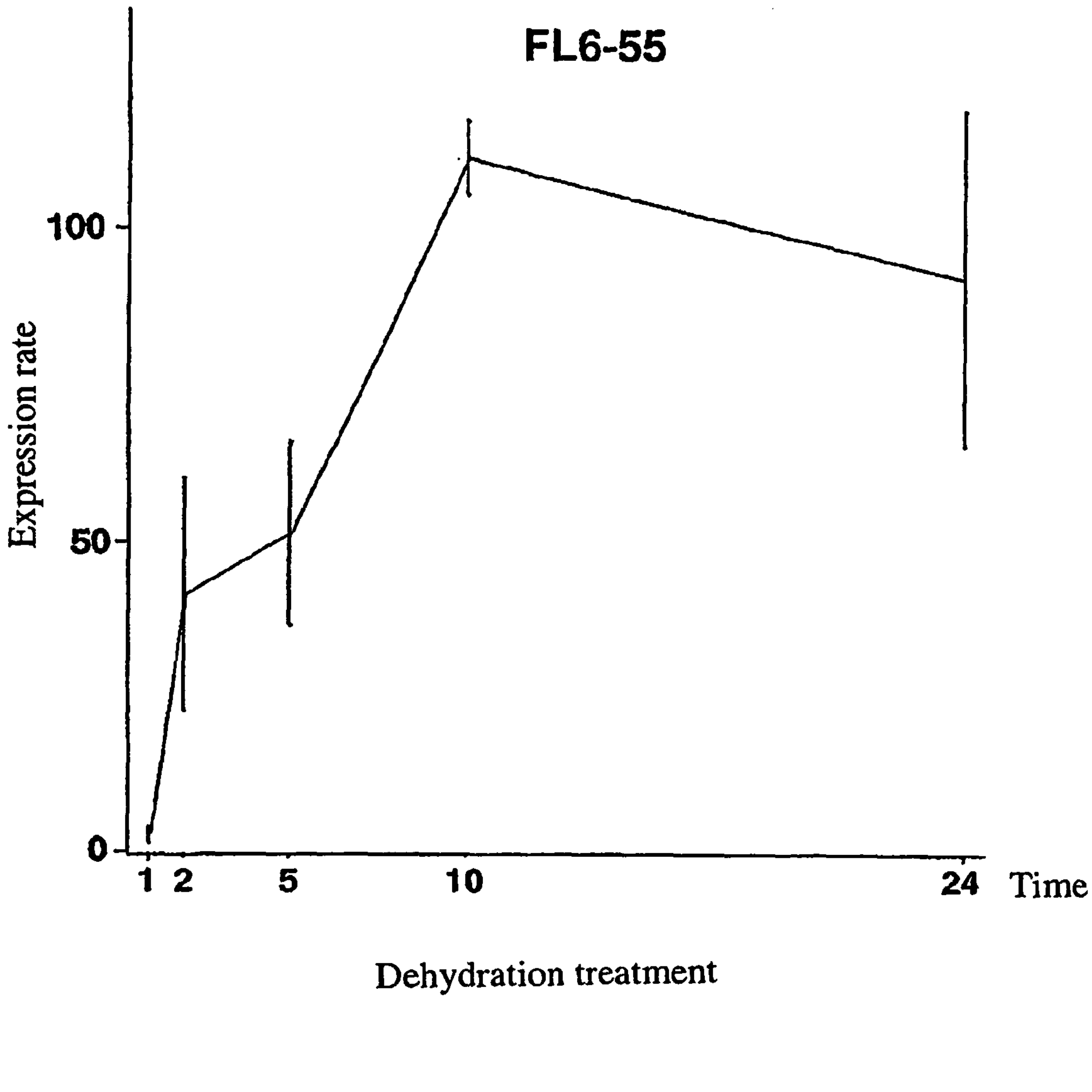
FL6-55
100
50
0
Expression rate
1 2 5 10 24 Time
Dehydration treatment

FIG.18

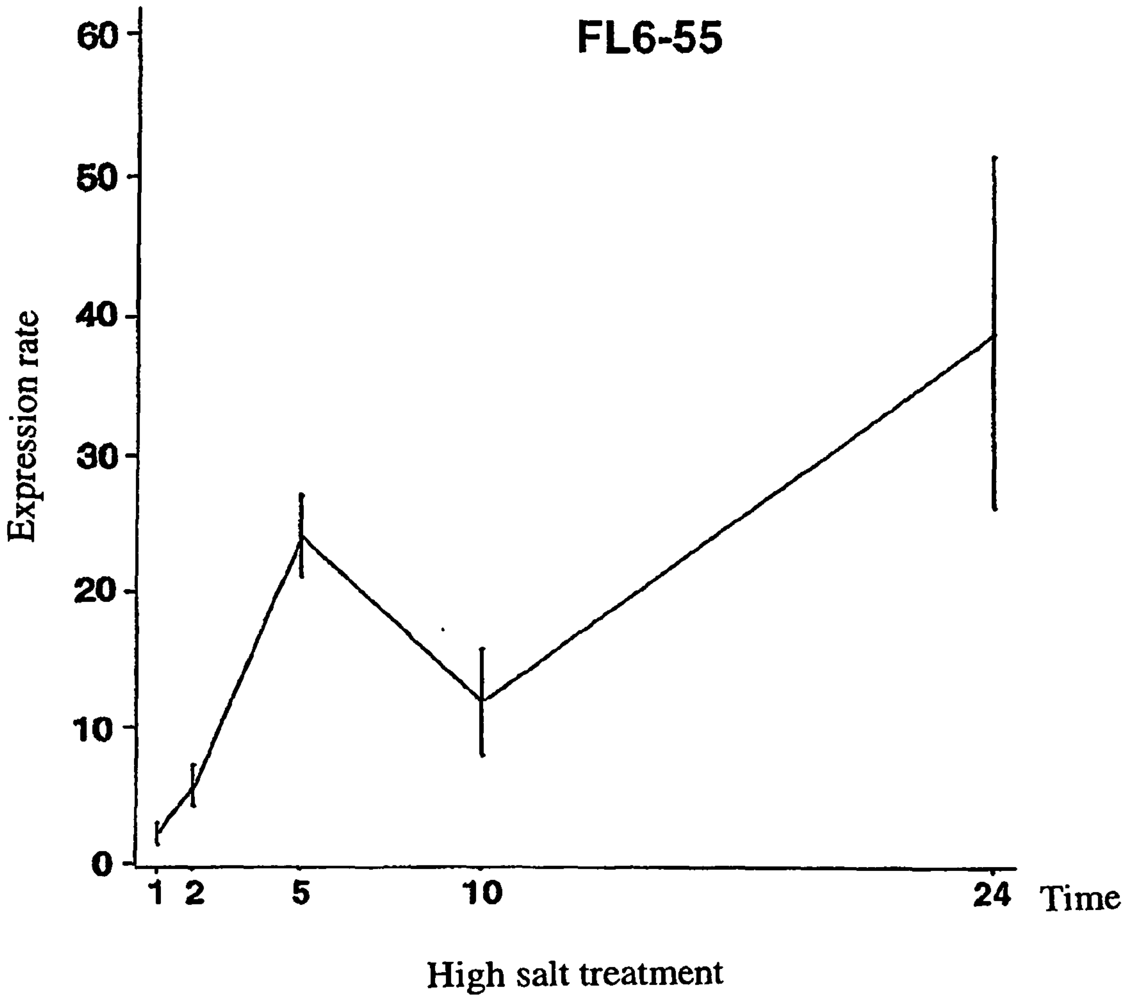
FL6-55
60
50
40
30
20
10
0
Expression rate
1
2
5
10
24
Time
High salt treatment

FIG.19

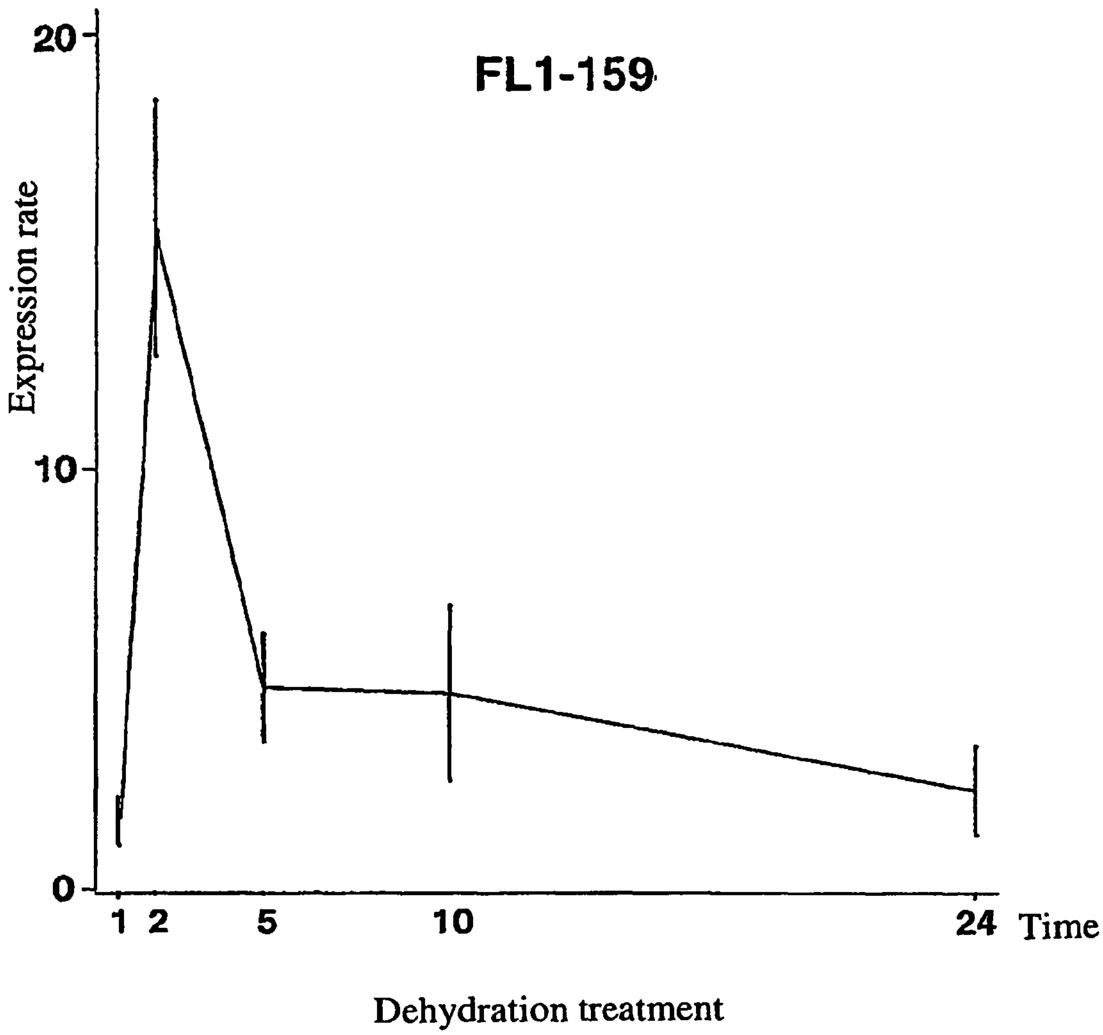
20
10
0
FL1-159
Expression rate
1 2 5 10 24 Time
Dehydration treatment

FIG.20

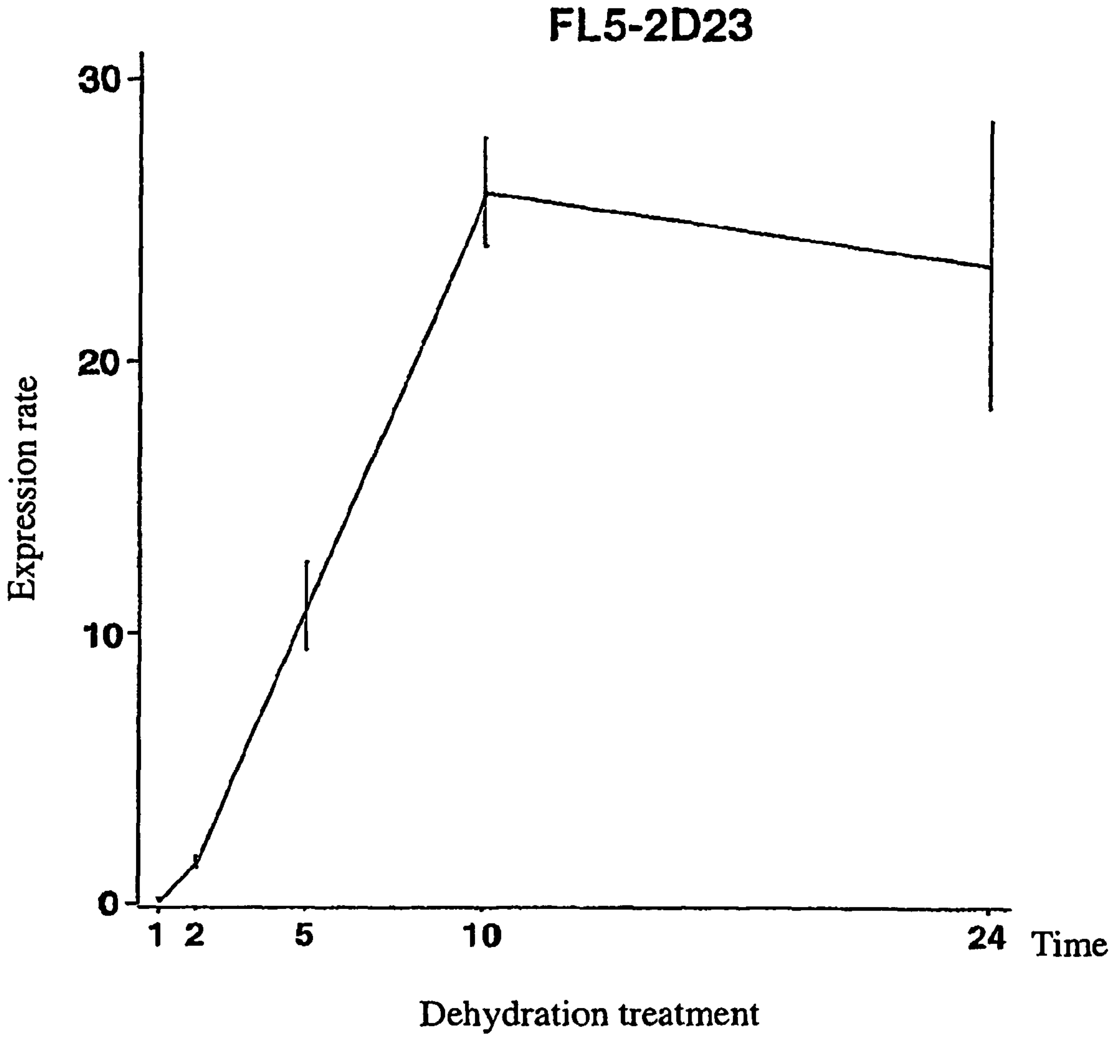
FL5-2D23
30
20
10
0
Expression rate
1
2
5
10
24
Time
Dehydration treatment

FIG.21

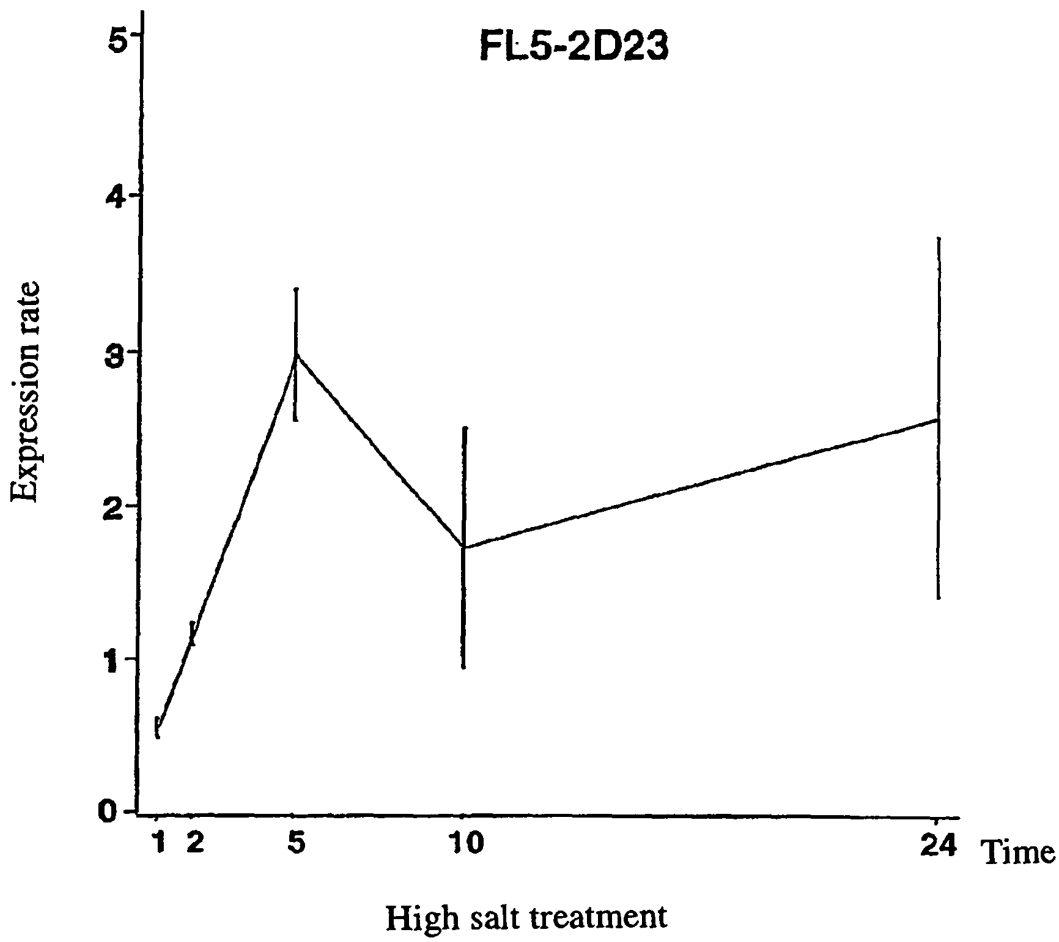
FL5-2D23
Expression rate
5
4
3
2
1
0
1
2
5
10
24
Time
High salt treatment

FIG.22

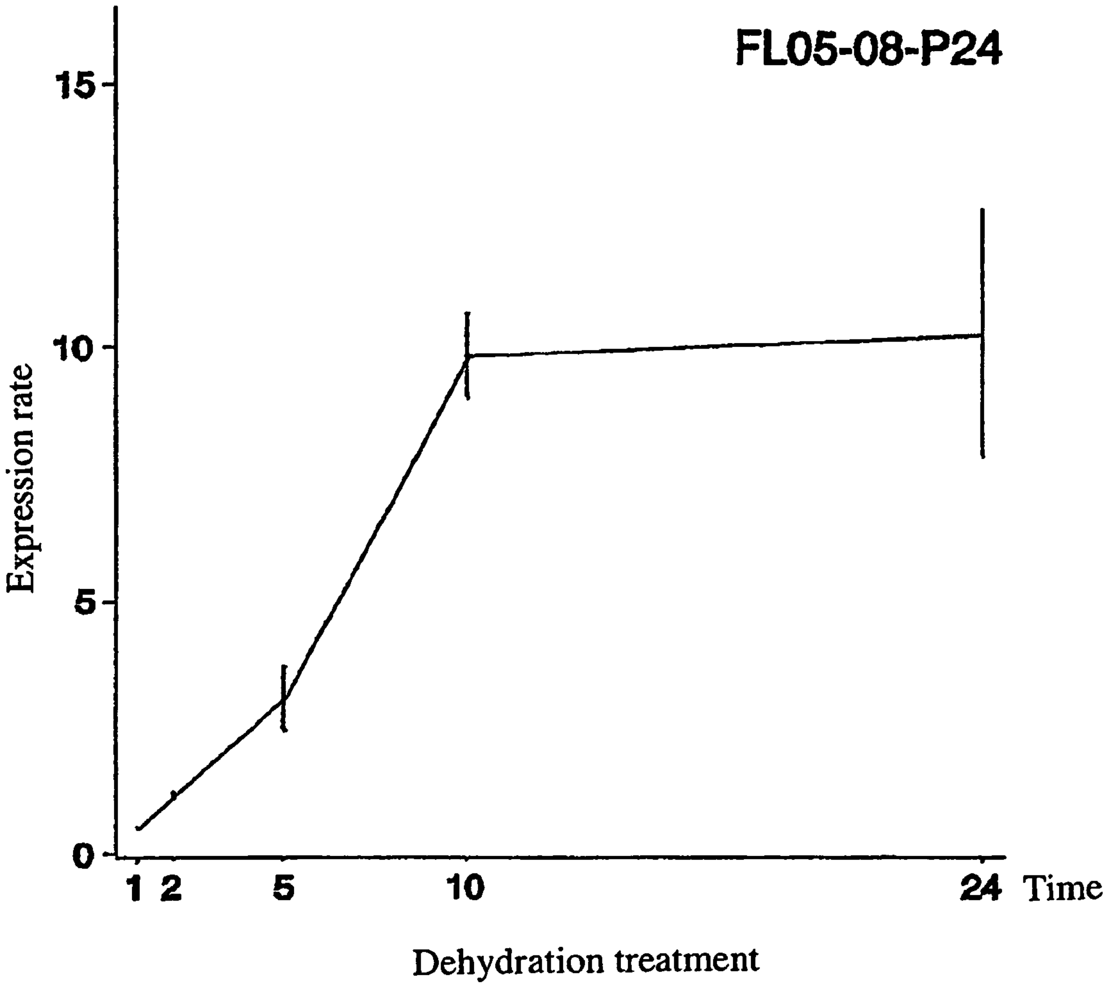
FL05-08-P24
15
10
5
0
Expression rate
1 2
5
10
24
Time
Dehydration treatment

FIG.23

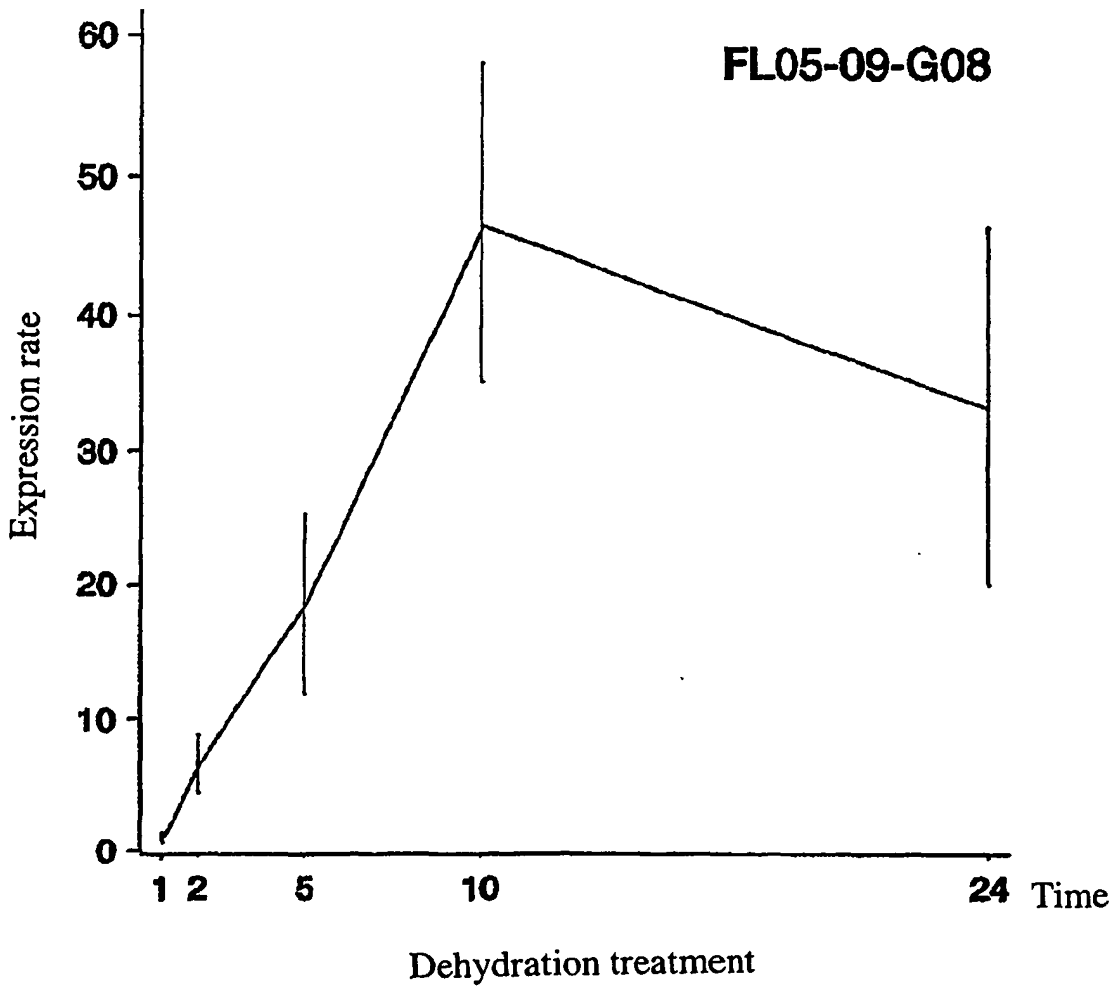
FL05-09-G08
Expression rate
60
50
40
30
20
10
0
1 2 5 10 24 Time
Dehydration treatment

FIG.24

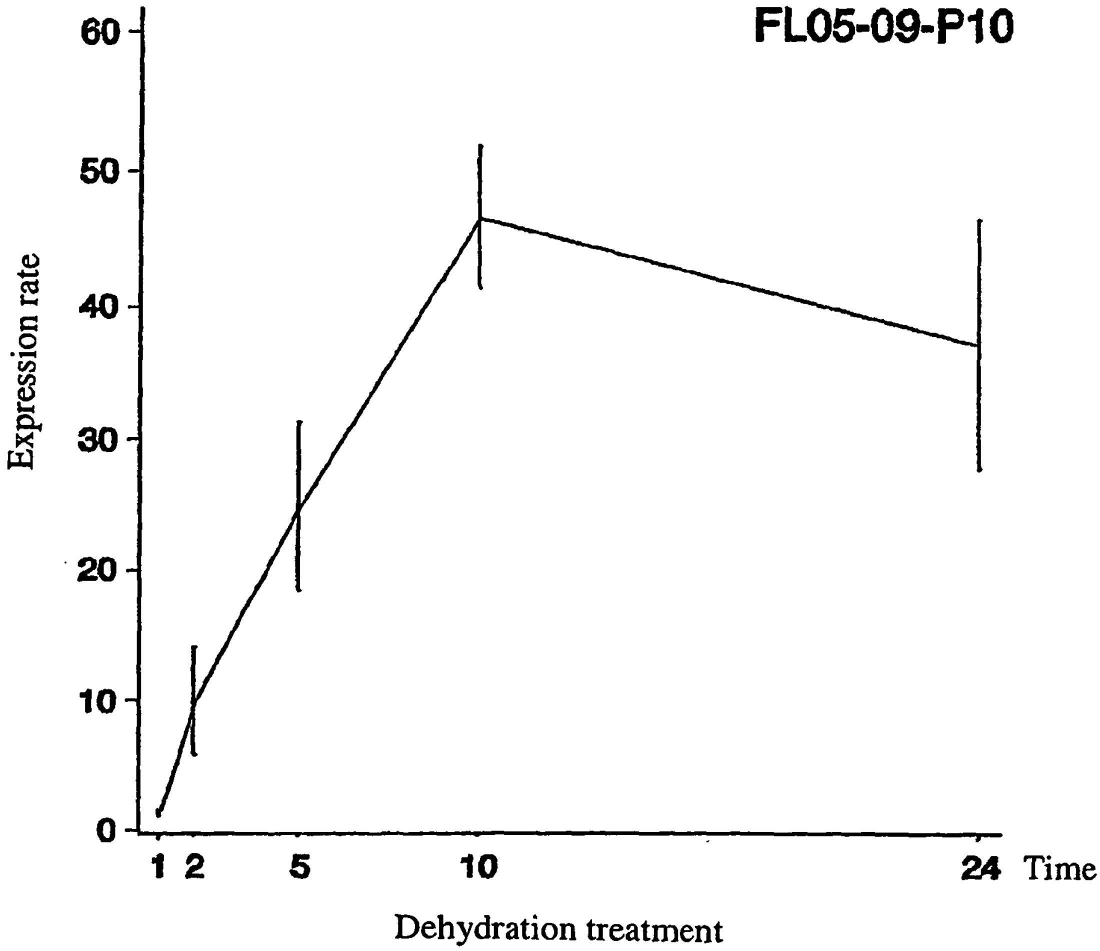
FL05-09-P10
Expression rate
60
50
40
30
20
10
0
1
2
5
10
24
Time
Dehydration treatment

FIG.25

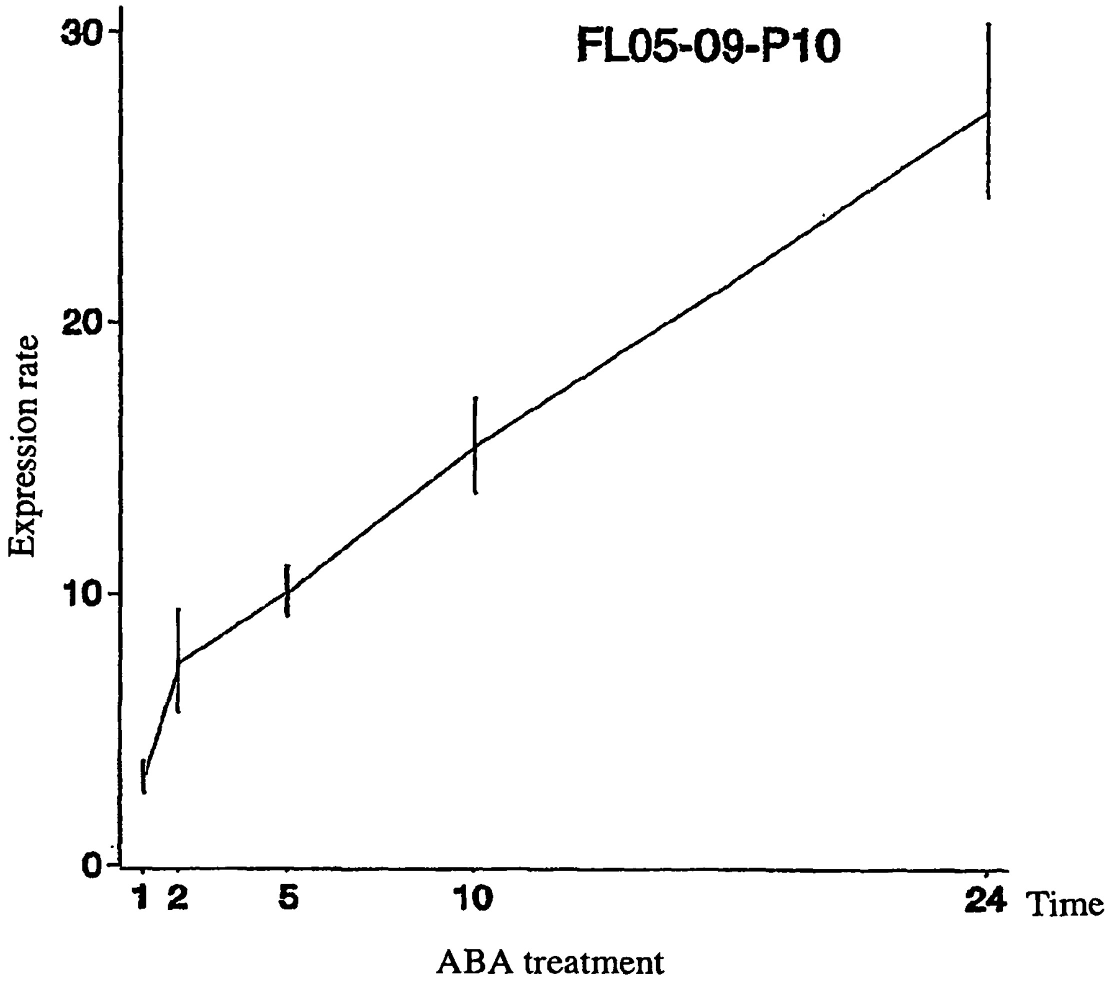
FL05-09-P10
30
20
10
0
Expression rate
1 2
5
10
24
Time
ABA treatment

FIG.26

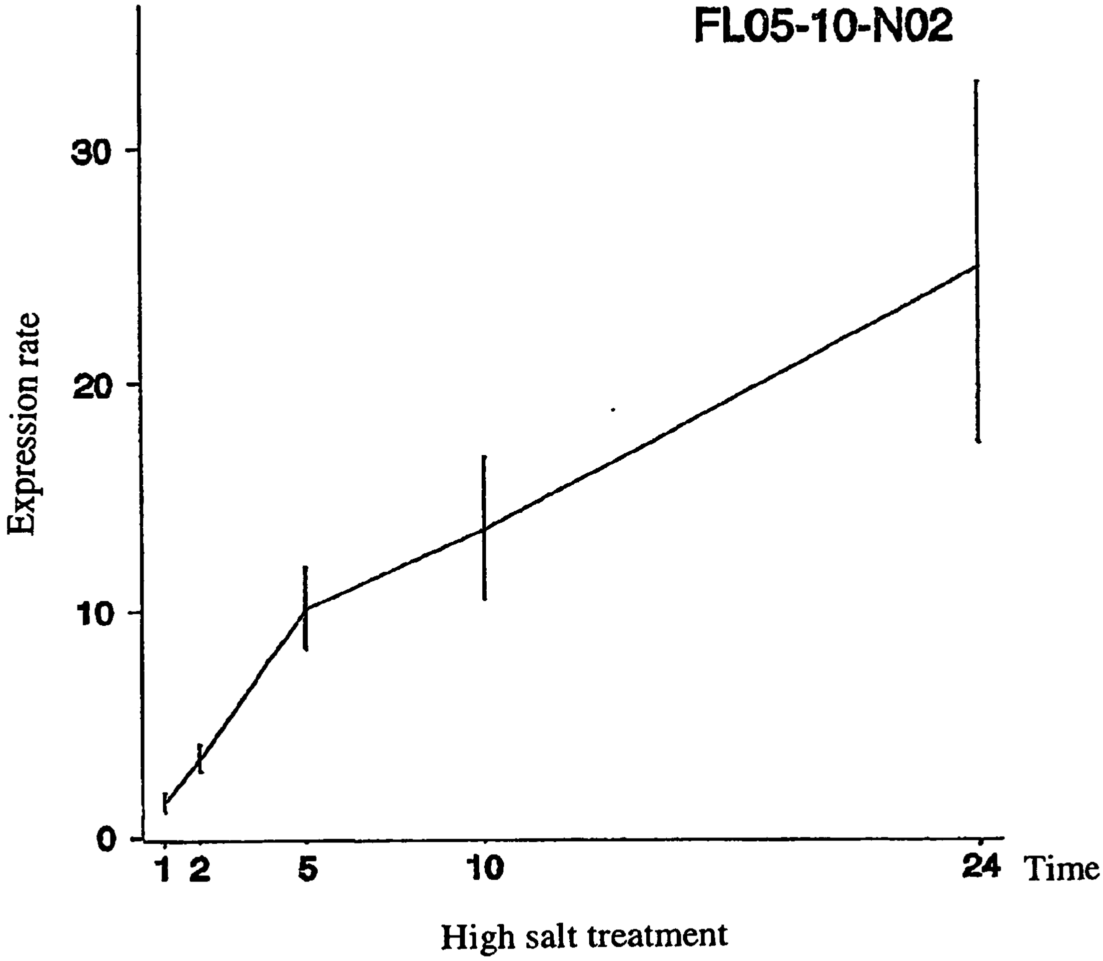
FL05-10-N02
Expression rate
0
10
20
30
1 2
5
10
24
Time
High salt treatment

FIG.27

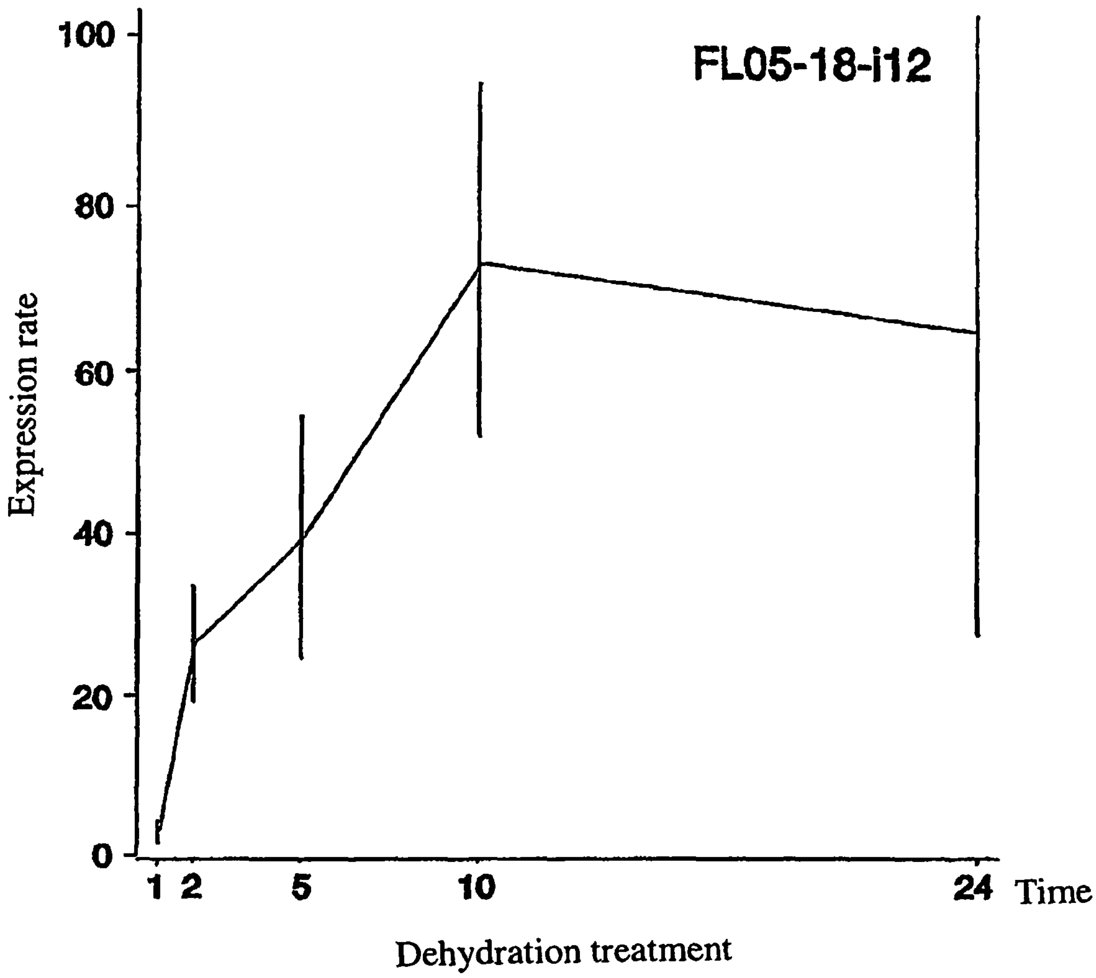
FL05-18-i12
100
80
60
40
20
0
Expression rate
1 2
5
10
24
Time
Dehydration treatment

FIG.28

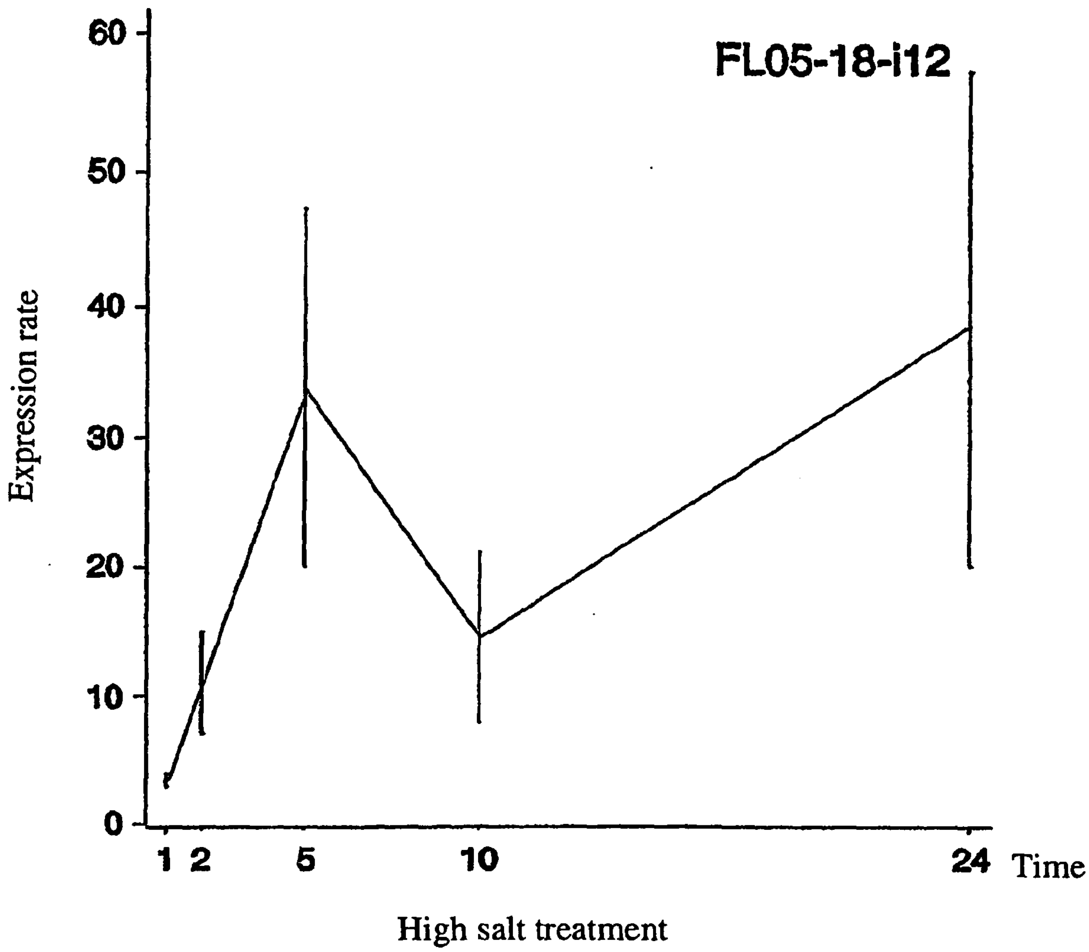
FL05-18-i12
60
50
40
30
20
10
0
Expression rate
1 2
5
10
24
Time
High salt treatment

FIG.29

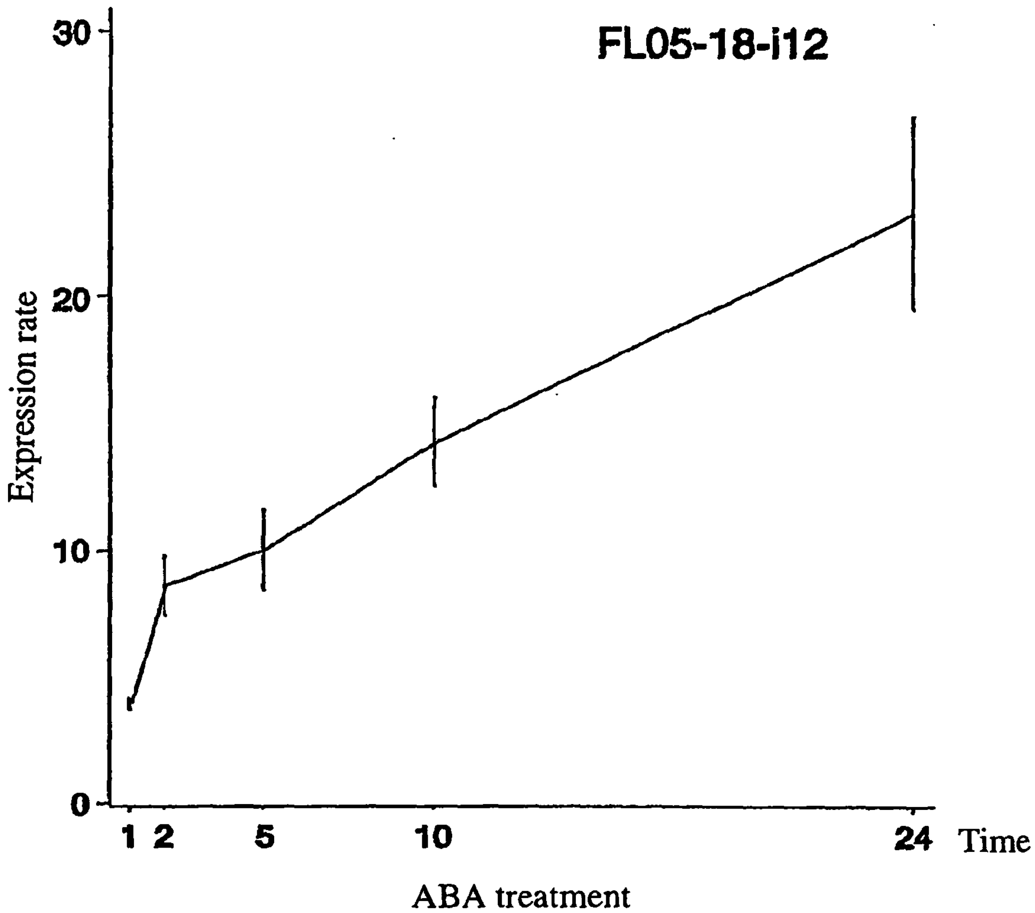
FL05-18-i12
30
20
10
0
Expression rate
1 2
5
10
24
Time
ABA treatment

FIG.30

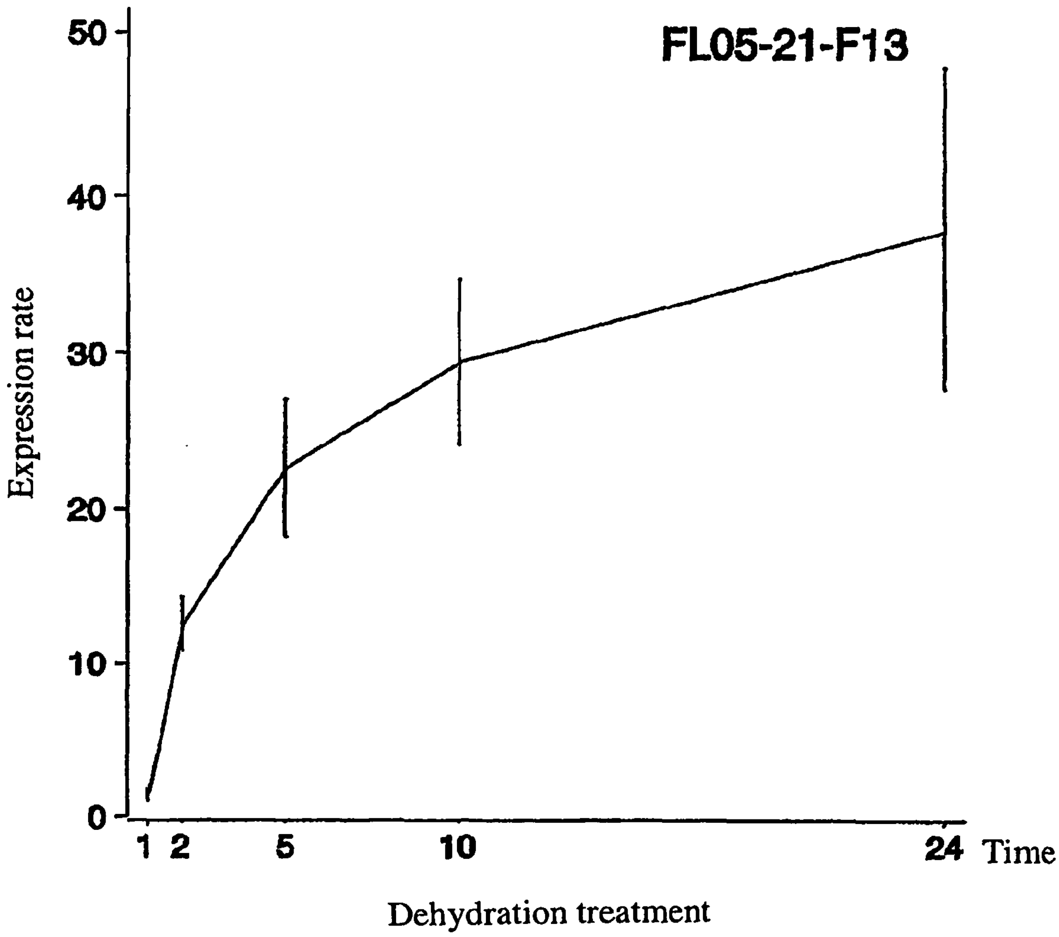
FL05-21-F13
Expression rate
50
40
30
20
10
0
1
2
5
10
24
Time
Dehydration treatment

FIG.31

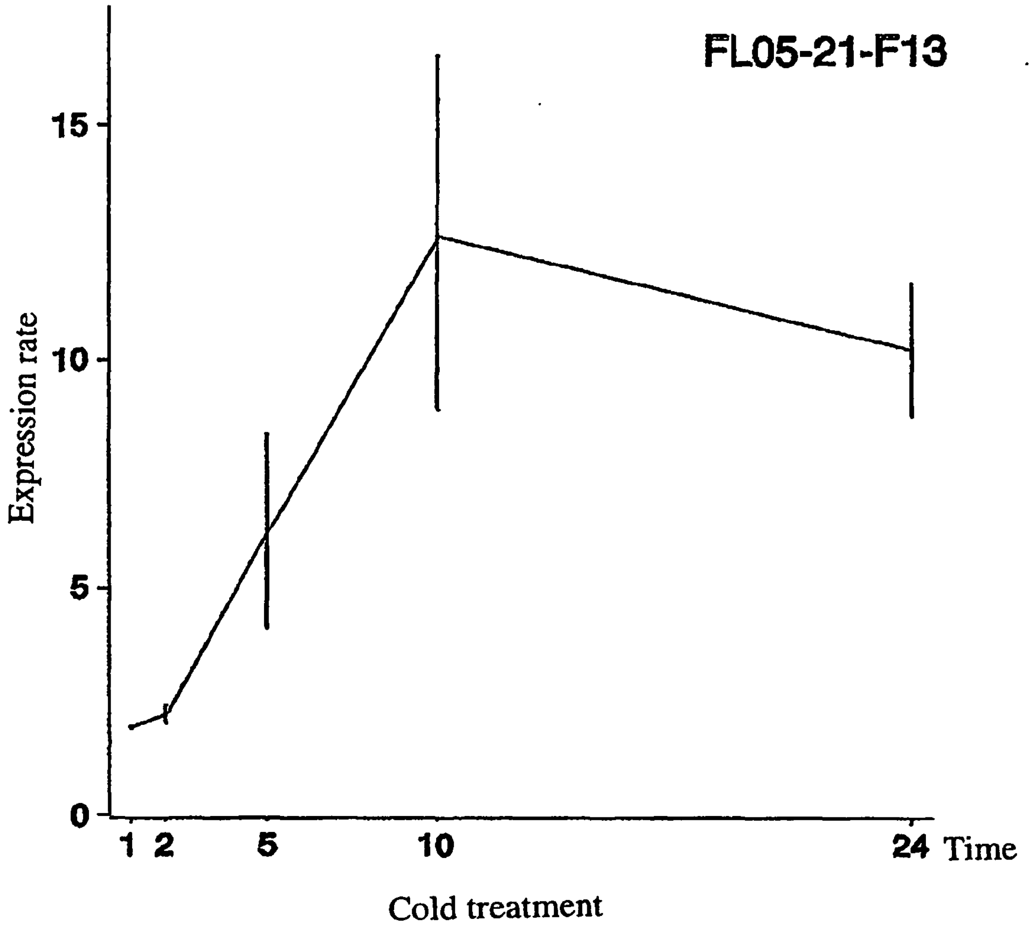
FL05-21-F13
15
10
5
0
Expression rate
1 2
5
10
24
Time
Cold treatment

FIG.32

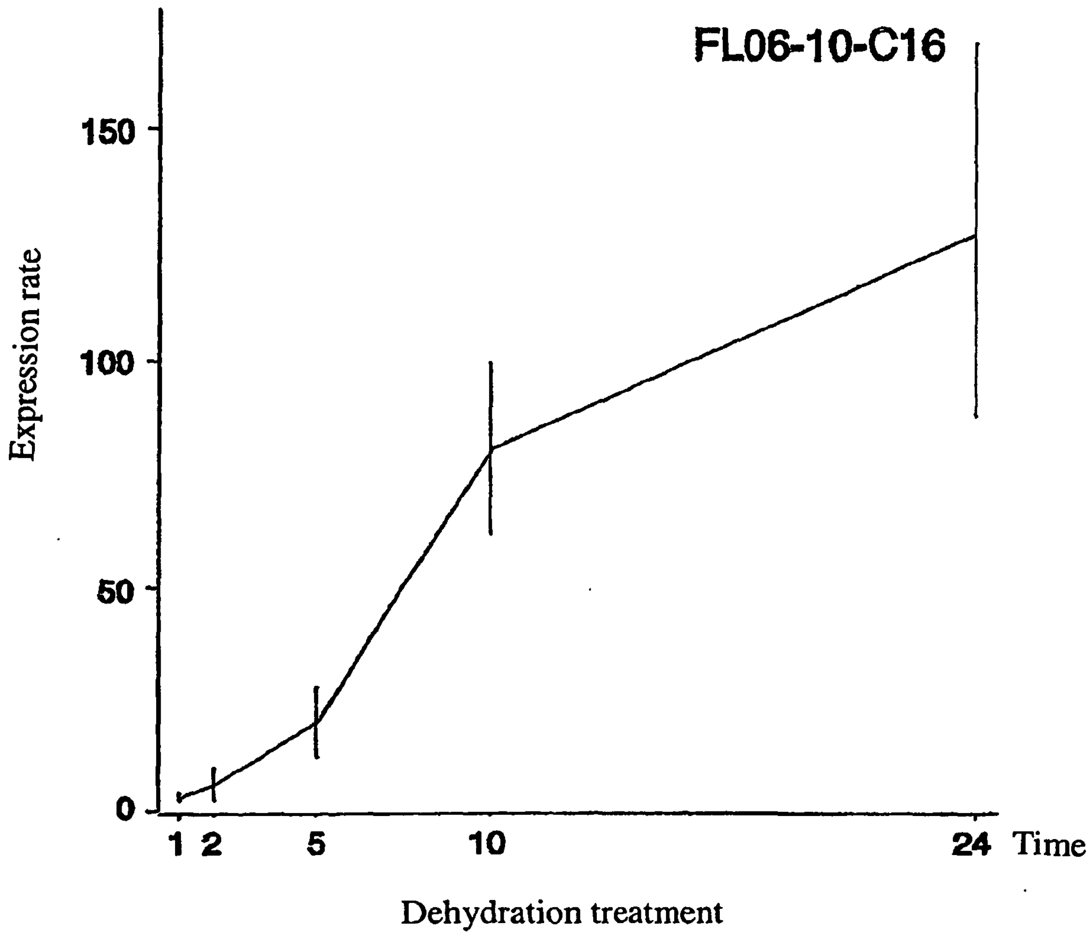
FL06-10-C16
150
100
50
0
Expression rate
1 2
5
10
24
Time
Dehydration treatment

FIG.33

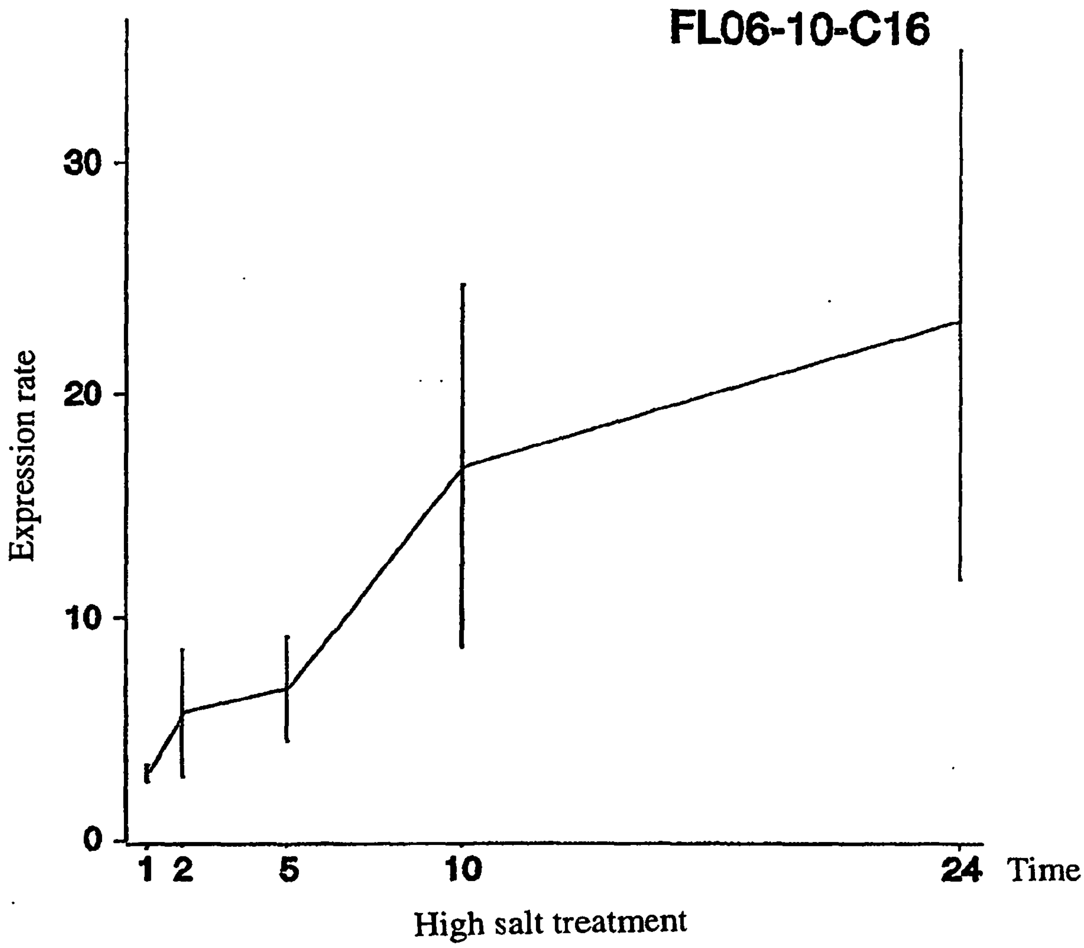
FL06-10-C16
Expression rate
30
20
10
0
1 2 5 10 24 Time
High salt treatment

FIG.34

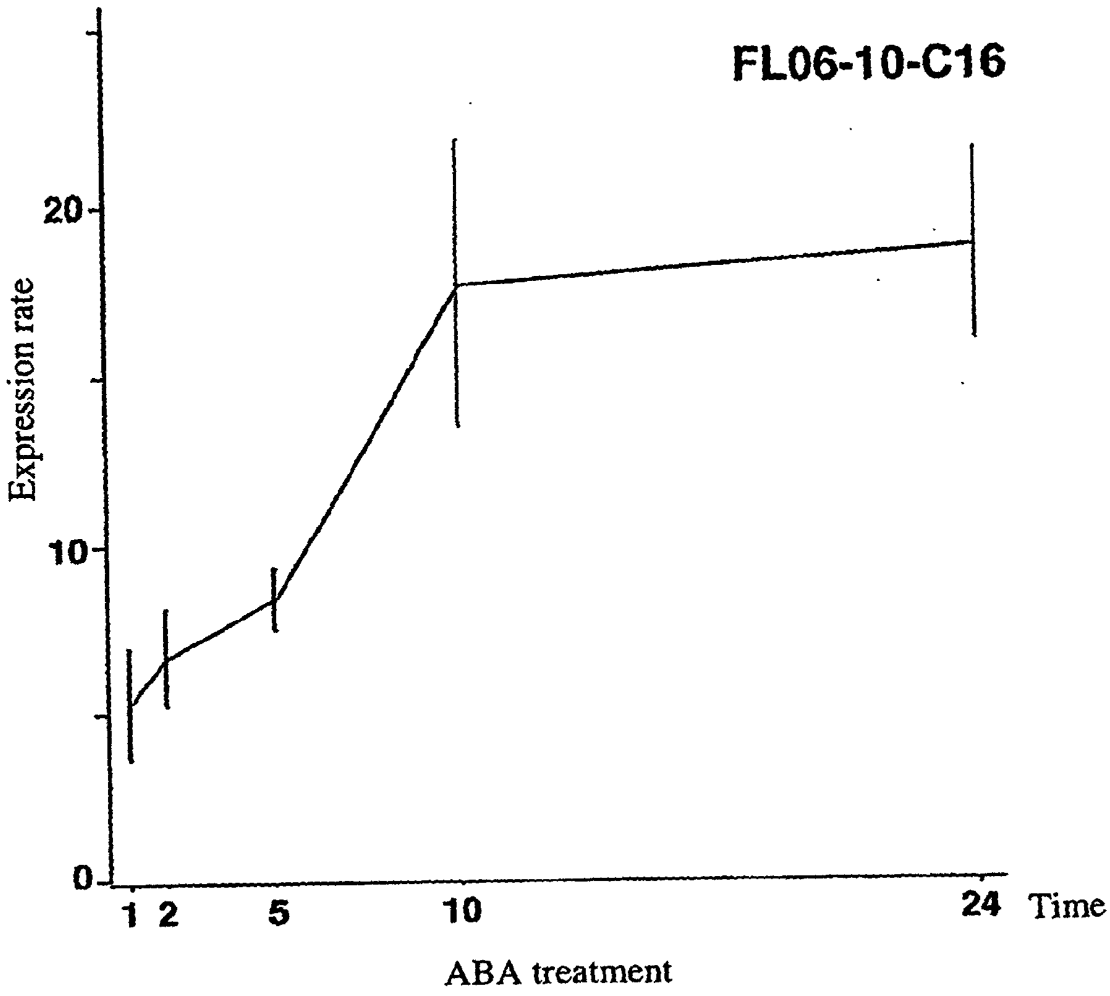
FL06-10-C16
Expression rate
20
10
0
1 2 5 10 24 Time
ABA treatment

FIG.35

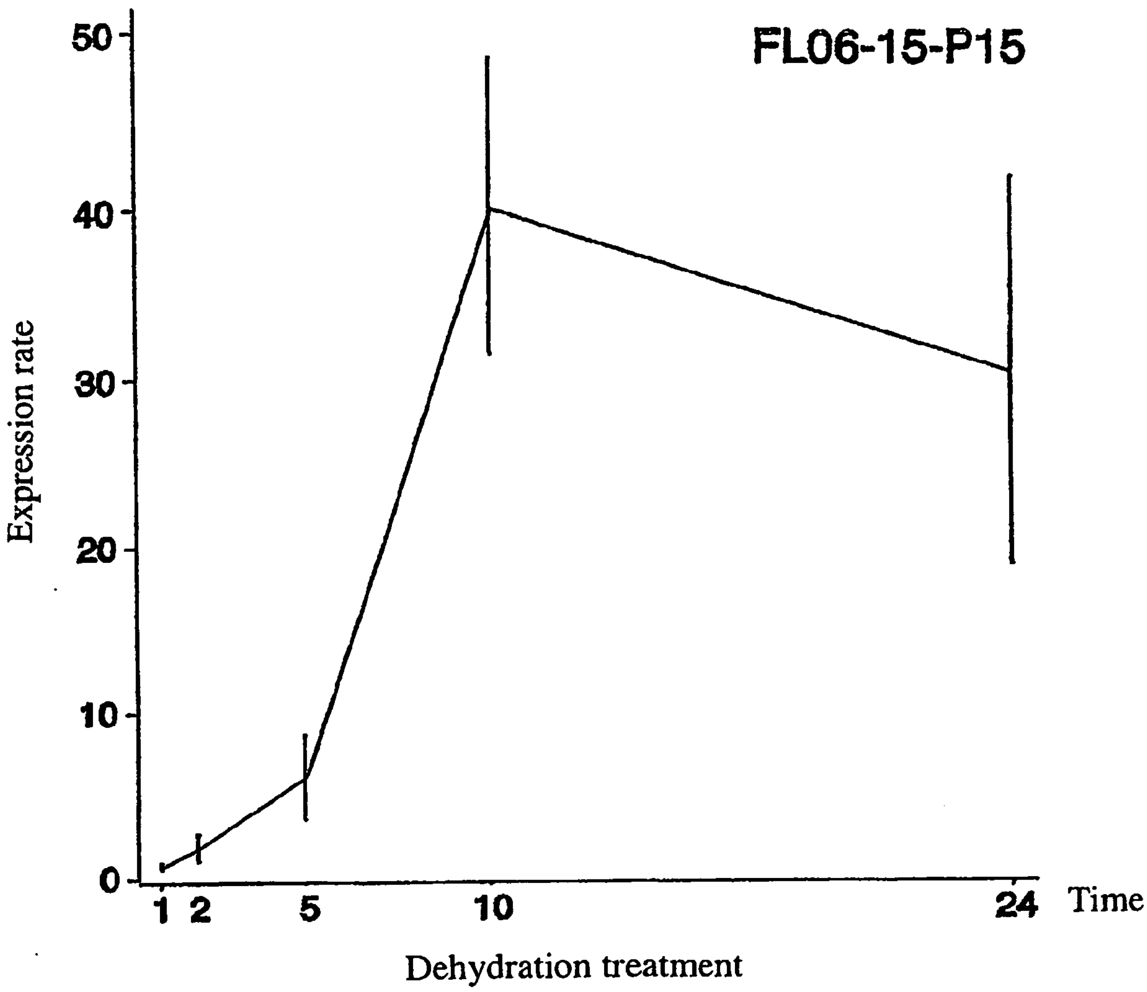
FL06-15-P15
50
40
30
20
10
0
Expression rate
1 2 5 10 24 Time
Dehydration treatment

FIG.36

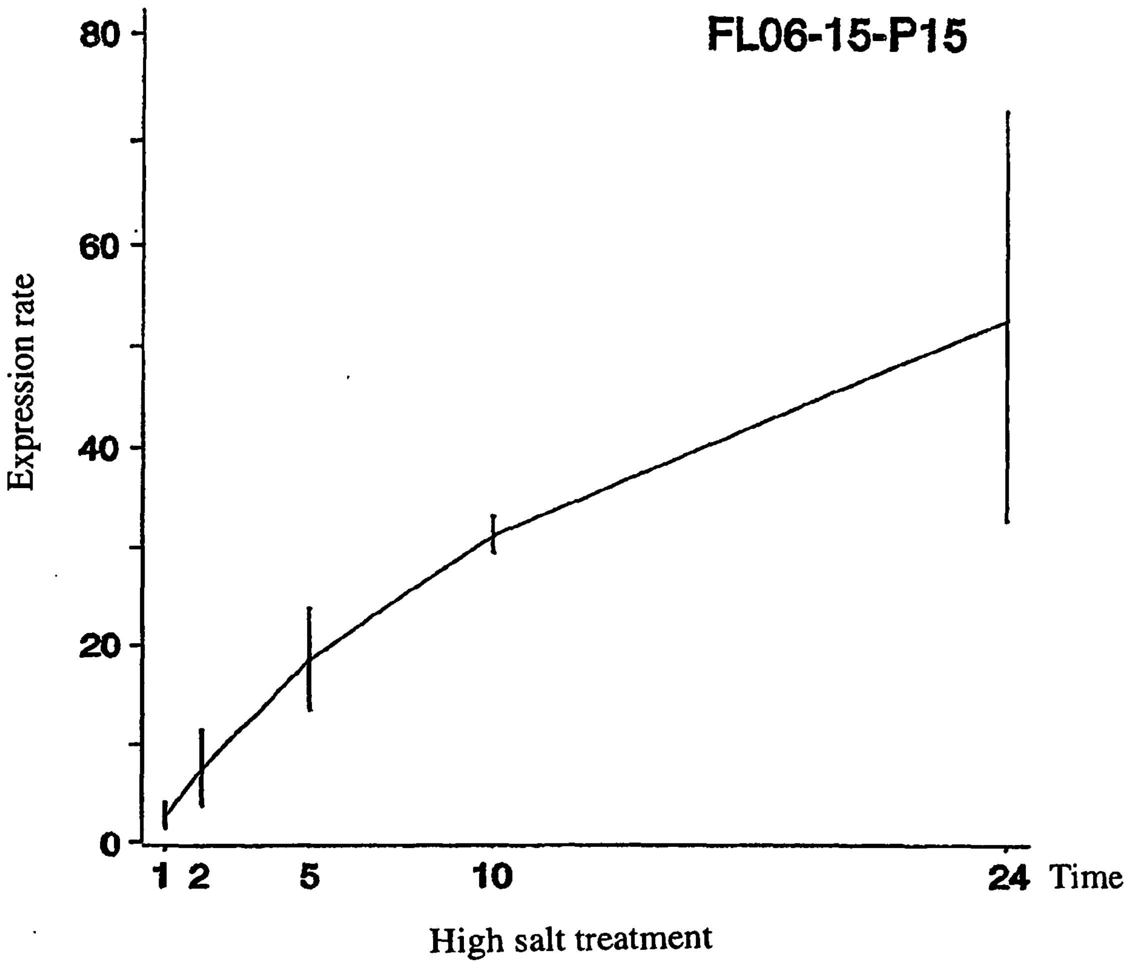
FL06-15-P15
80
60
40
20
0
Expression rate
1 2 5 10 24 Time
High salt treatment

FIG.37

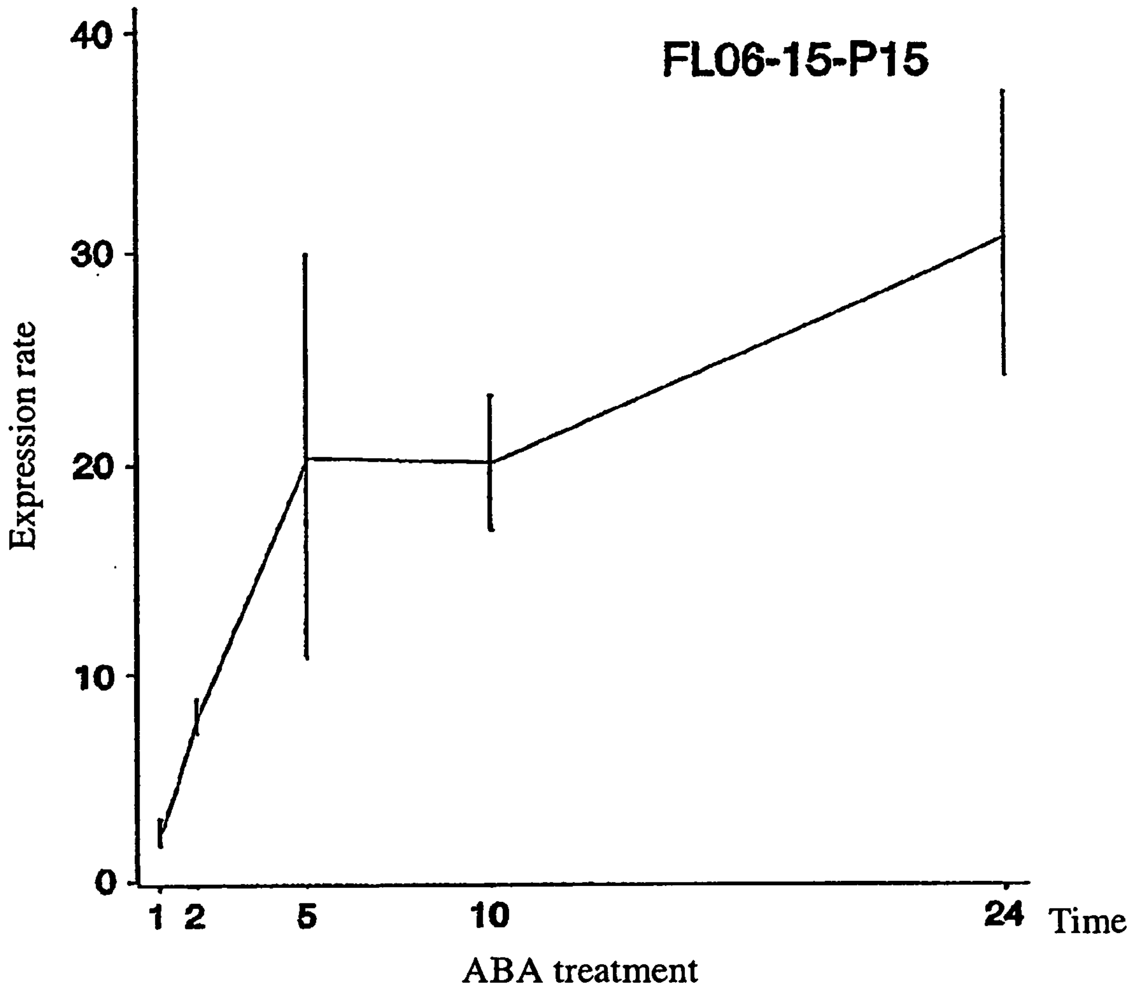
FL06-15-P15
40
30
20
10
0
Expression rate
1 2
5
10
24
Time
ABA treatment

FIG.38

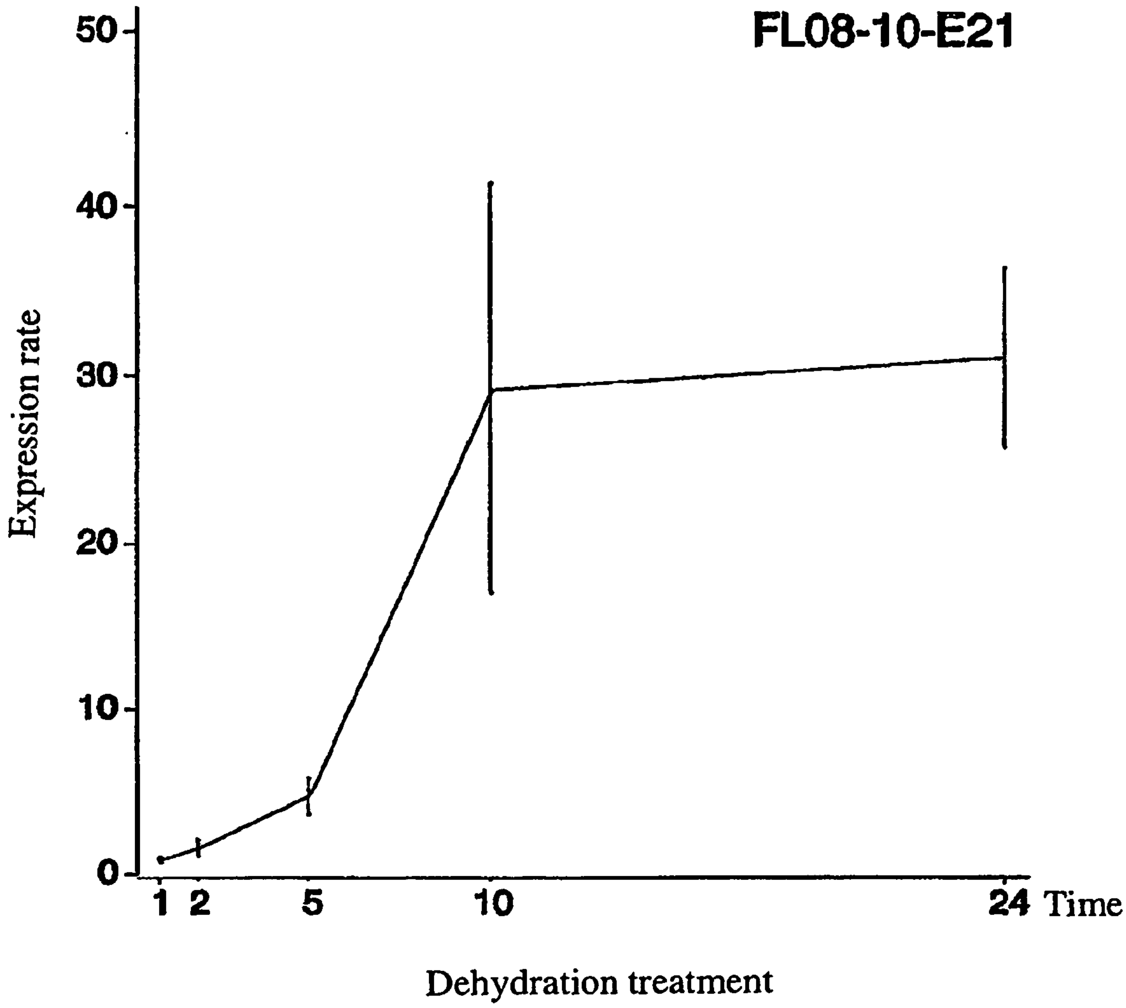
FL08-10-E21
Expression rate
0
10
20
30
40
50
1
2
5
10
24
Time
Dehydration treatment

FIG.39

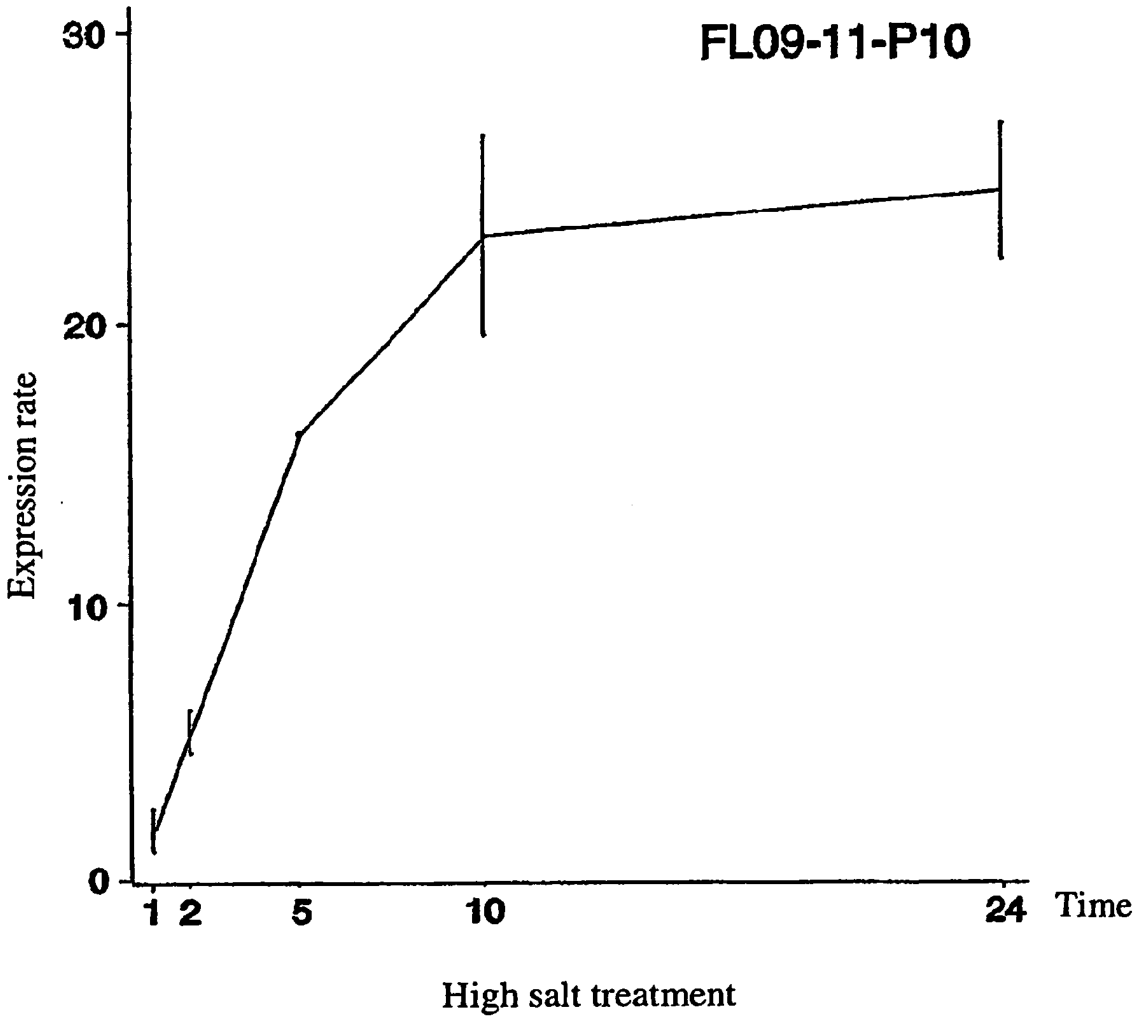
FL09-11-P10
30
20
10
0
Expression rate
1
2
5
10
24
Time
High salt treatment

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001309984 A
- JP 2000356652 A

### Non-patent literature cited in the description


- **LIN, X. et al.** *Nature,* 1999, vol. 402, 761-768
- **MAYER, K. et al.** *Nature,* 1999, vol. 402, 769-777
- **HOFTE, H. et al.** *Plant J.,* 1993, vol. 4, 1051-1061
- **NEWMAN, T. et al.** *Plant Physiol.,* 1994, vol. 106, 1241-1255
- **COOKE, R. et al.** *Plant J.,* 1996, vol. 9, 101-124
- **ASAMIZU, E. et al.** *DNA Res.,* 2000, vol. 7, 175-180
- **SCHENA, M. et al.** *Science,* 1995, vol. 270, 467-470
- **EISEN, M. B. ; BROWN, P. O.** *Methods Enzymol.,* 1999, vol. 303, 179-205
- **SCHENA M. et al.** *Proc. Natl. Acad. Sci. USA,* 1996, vol. 93, 10614-10619
- **HELLER, R. A. et al.** *Proc. Natl. Acad. Sci. USA,* 1997, vol. 94, 2150-2155
- **DERISI, J. L. et al.** *Science,* 1997, vol. 278, 680-686
- **WODICKA, L. et al.** *Nature Biotechnol.,* 1997, vol. 15, 1359-1367
- **RUAN, Y. et al.** *Plant J.,* 1998, vol. 15, 821-833
- **AHARONI. A. et al.** *Plant Cell,* 2000, vol. 12, 647-661
- **REYMOND, P. et al.** *Plant Cell,* 2000, vol. 12, 707-719
- **BOHNERT, H.J. et al.** *Plant Cell,* 1995, vol. 7, 1099-1111
- **INGRAM, J. ; BARTELS, D.** *Plant Mol. Biol.,* 1996, vol. 47, 377-403
- **BRAY, E. A.** *Trends Plant Sci.,* 1997, vol. 2, 48-54
- **SHINOZAKI. K. ; YAMAGUCHI-SHINOZAKI, K.** *Plant Physiol.,* 1997, vol. 115, 327-334
- Molecular responses to drought stress. **SHINOZAKI, K. ; YAMAGUCHI-SHINOZAKI, K.** Molecular responses to cold, drought, heat and salt stress in higher plants. Landes Company, 1999
- **SHINOZAKI, K. ; YAMAGUCHI-SHINOZAKI, K.** *Curr. Opin. Plant Biol.,* 2000, vol. 3, 217-223
- **HOLMBERG, N. ; BULOW, L.** *Trends Plant Sci.,* 1998, vol. 3, 61-66
- **BAJAJ. S. et al.** *Mol. Breed.,* 1999, vol. 5, 493-503
- **YAMAGUCHI-SHINOZAKI, K. ; SHINOZAKI, K.** *Plant Cell,* 1994, vol. 6, 251-264
- **THOMASHOW, M.F. et al.** *Plant Mol. Biol.,* 1999, vol. 50, 571-599
- **STOCKINGER. E.J. et al.** *Proc. Natl. Acad. Sci. USA,* 1997, vol. 94, 1035-1040
- **LIU, Q. et al.** *Plant Cell,* 1998, vol. 10, 1391-1406
- **SHINWARI, Z.K. et al.** *Biochem. Biophys. Res. Commun.,* 1998, vol. 250, 161-170
- **GILMOUR, S.J. et al.** *Plant J.,* 1998, vol. 16, 433-443
- **JAGLO-OTTOSEN, K.R. et al.** *Science,* 1998, vol. 280, 104-106
- **KASUGA, M. et al.** *Nature Biotechnol.,* 1999, vol. 17, 287-291
- **CARNINCI. P. et al.** *Genomics,* 1996, vol. 37, 327-336
- **SEKI. M. et al.** *Plant J.,* 1998, vol. 15, 707-720
- Molecular responses to drought stress. **SHINOZAKI, K. ; YAMAGUCHI-SHINOZAKI, K.** Molecular responses to cold, drought, heat and salt stress in higher plants. Landes Company, 1999
- **TAJI, T. et al.** *Plant Cell Physiol.,* 1999, vol. 40, 119-123
- **TAKAHASHI, S. et al.** *Plant Cell Physiol.,* 2000, vol. 41, 898-903
- **BAKER, S. S. et al.** *Plant Mol. Biol.,* vol. 24, 701-713
- **WANG, H. et al.** *Plant Mol. Biol.,* vol. 28, 605-617
- **IWASAKI, T. et al.** *Plant Physiol.,* 1997, vol. 115, 1287